# EUROPEAN PATENT APPLICATION

(11) **EP 4 671 863 A2**
(43) Date of publication of application: **31.12.2025**
(21) Application number: 25215313.5
(22) Date of filing: 21.11.2022
(51) Int. Cl.: G03B 42/08

(54) **RADIOGRAPHY IMAGE READING APPARATUS**

(30) Priority: 22.11.2021 JP 2021189740; 22.11.2021 JP 2021189734; 22.11.2021 JP 2021189736; 22.11.2021 JP 2021189735
(62) Divisional of application: 22208546.6
(71) Applicant: J. Morita Manufacturing Corporation, Kyoto-shi Kyoto 612-8533 (JP)
(72) Inventor: YOSHIOKA, Tadashi, Kyoto, 612-8533 (JP); SUGIHARA, Yoshito, Kyoto, 612-8533 (JP); FUJII, Yusuke, Kyoto, 612-8533 (JP)
(74) Representative: Müller Hoffmann & Partner

(57) **Abstract**

A reading apparatus that reads a radiograph from an imaging plate includes: a first light source that irradiates the imaging plate with excitation light; a first detector that detects photostimulated light from the imaging plate emitted by the excitation light; a second light source that irradiates an object with light; and a second detector that detects reflected light of the light from the object.

## Description

### BACKGROUND OF THE INVENTION

### Field of the Invention

The present disclosure relates to reading apparatuses.

### Description of the Background Art

Japanese Patent Application Laid-Open No. 61-267451 discloses technology of reading image information from a storage phosphor sheet.

It would be desirable to improve usability of a reading apparatus that reads a radiograph from an imaging plate.

### SUMMARY

It is thus an object of the present disclosure to provide technology enabling improvement in usability of a reading apparatus that reads a radiograph from an imaging plate.

One aspect of a reading apparatus is a reading apparatus that reads a radiograph from an imaging plate, and includes: a first light source that irradiates the imaging plate with excitation light; a first detector that detects photostimulated light from the imaging plate emitted by the excitation light; a second light source that irradiates an object with light; and a second detector that detects reflected light of the light from the object.

A radiograph based on detection of the photostimulated light from the imaging plate emitted by the excitation light and a reflected light image based on detection of the reflected light of the light from the object can be acquired to improve usability of the reading apparatus.

These and other objects, features, aspects and advantages of the present invention will become more apparent from the following detailed description of the present invention when taken in conjunction with the accompanying drawings.

### BRIEF DESCRIPTION OF THE DRAWINGS

FIG. 1 is a schematic diagram illustrating one example of appearance of a reading apparatus;
FIG. 2 is a schematic diagram illustrating one example of a configuration of the reading apparatus;
FIG. 3 is a schematic diagram illustrating one example of a configuration of the reading apparatus;
FIG. 4 is a schematic diagram illustrating one example of a configuration of the reading apparatus;
FIG. 5 is a schematic diagram illustrating one example of a configuration of the reading apparatus;
FIG. 6 is a block diagram showing one example of a configuration of the reading apparatus;
FIG. 7 is a schematic diagram illustrating one example of a configuration of the reading apparatus;
FIG. 8 is a schematic diagram illustrating one example of a configuration of the reading apparatus;
FIG. 9 is a schematic diagram illustrating one example of a configuration of the reading apparatus;
FIG. 10 is a schematic diagram illustrating one example of an IP image region, an excitation light irradiation range, and a detection range;
FIG. 11 is a schematic diagram illustrating one example of a configuration of the reading apparatus;
FIG. 12 is a schematic diagram illustrating one example of the IP image region, the excitation light irradiation range, and the detection range;
FIG. 13 is a schematic diagram illustrating one example of the IP image region, the excitation light irradiation range, and the detection range;
FIG. 14 is a schematic diagram showing one example of an acquired whole image;
FIG. 15 is a schematic diagram showing one example of the acquired whole image;
FIG. 16 is a schematic diagram showing one example of the acquired whole image;
FIG. 17 is a schematic diagram showing one example of the acquired whole image;
FIG. 18 is a flowchart showing one example of operation of the reading apparatus;
FIG. 19 is a schematic diagram illustrating one example of a tilt of an imaging plate;
FIG. 20 is a schematic diagram showing one example of the acquired whole image;
FIG. 21 is a schematic diagram for explaining one example of tilt angle identification processing;
FIG. 22 is a schematic diagram for explaining one example of the tilt angle identification processing;
FIG. 23 is a schematic diagram for explaining one example of size identification processing;
FIG. 24 is a diagram showing one example of types of sizes of the imaging plate;
FIG. 25 is a schematic diagram showing examples of the imaging plate;
FIG. 26 is a flowchart showing one example of operation of the reading apparatus;
FIG. 27 is a flowchart showing one example of operation of the reading apparatus;
FIG. 28 is a schematic diagram showing one example of the acquired whole image;
FIG. 29 is a schematic diagram showing an example of display of the acquired whole image;
FIG. 30 is a schematic diagram showing an example of display of the acquired whole image;
FIG. 31 is a schematic diagram for explaining one example of tilt correction processing;
FIG. 32 is a schematic diagram showing one example of the acquired whole image;
FIG. 33 is a schematic diagram for explaining one example of cutting-out processing;
FIG. 34 is a schematic diagram showing one example of a cutout image;
FIG. 35 is a schematic diagram for explaining one example of the cutting-out processing;
FIG. 36 is a schematic diagram showing one example of the cutout image;
FIG. 37 is a schematic diagram showing an example of display of the cutout image;
FIG. 38 is a schematic diagram for explaining one example of the cutting-out processing;
FIG. 39 is a schematic diagram showing one example of the cutout image;
FIG. 40 is a schematic diagram showing one example of the cutout image;
FIG. 41 is a schematic diagram illustrating one example of a configuration of a holder;
FIG. 42 is a schematic diagram showing one example of the acquired whole image;
FIG. 43 is a schematic diagram for explaining one example of the cutting-out processing;
FIG. 44 is a schematic diagram for explaining one example of the cutting-out processing;
FIG. 45 is a schematic diagram showing one example of the cutout image;
FIG. 46 is a schematic diagram showing one example of the acquired whole image;
FIG. 47 is a schematic diagram for explaining one example of the cutting-out processing;
FIG. 48 is a schematic diagram showing one example of the cutout image;
FIG. 49 is a schematic diagram showing one example of the cutout image;
FIG. 50 is a schematic diagram showing an example of display of unexposure notification information;
FIG. 51 is a schematic diagram showing an example of display of the unexposure notification information;
FIG. 52 is a schematic diagram showing an example of display of the unexposure notification information;
FIG. 53 is a schematic diagram showing an example of display of the unexposure notification information;
FIG. 54 is a schematic diagram showing an example of display of the unexposure notification information;
FIG. 55 is a schematic diagram showing an example of display of the unexposure notification information;
FIG. 56 is a schematic diagram showing one example of a radiograph included in the acquired whole image;
FIG. 57 is a schematic diagram showing one example of an IP whole reflected light image included in the acquired whole image;
FIG. 58 is a schematic diagram showing an example of display of the cutout image;
FIG. 59 is a schematic diagram showing an example of display of the cutout image;
FIG. 60 is a flowchart showing one example of operation of the reading apparatus;
FIG. 61 is a schematic diagram showing one example of dividing an IP reflected light image into a plurality of subregions;
FIG. 62 is a schematic diagram showing an example of display of the reading apparatus;
FIG. 63 is a schematic diagram showing an example of display of the reading apparatus;
FIG. 64 is a schematic diagram showing one example of a back surface of the imaging plate;
FIG. 65 is a schematic diagram showing one example of the IP reflected light image included in the acquired whole image;
FIG. 66 is a schematic diagram showing one example of a front surface of the imaging plate;
FIG. 67 is a schematic diagram showing one example of the front surface of the imaging plate;
FIG. 68 is a schematic diagram showing one example of the imaging plate;
FIG. 69 is a schematic diagram showing one example of the imaging plate;
FIG. 70 is a schematic diagram showing one example of the imaging plate;
FIG. 71 is a schematic diagram showing one example of the imaging plate;
FIG. 72 is a schematic diagram showing one example of the imaging plate;
FIG. 73 is a schematic diagram showing one example of the imaging plate;
FIG. 74 is a schematic diagram showing one example of the imaging plate;
FIG. 75 is a schematic diagram showing one example of the imaging plate;
FIG. 76 is a flowchart showing one example of operation of the reading apparatus;
FIG. 77 is a schematic diagram showing an example of display of the reading apparatus;
FIG. 78 is a flowchart showing one example of operation of the reading apparatus;
FIG. 79 is a schematic diagram illustrating one example of setting the imaging plate;
FIG. 80 is a schematic diagram illustrating one example of setting the imaging plate;
FIG. 81 is a schematic diagram illustrating one example of setting the imaging plate;
FIG. 82 is a schematic diagram illustrating one example of setting the imaging plate;
FIG. 83 is a schematic diagram illustrating one example of setting the imaging plate;
FIG. 84 is a schematic diagram illustrating one example of setting the imaging plate;
FIG. 85 is a flowchart showing one example of operation of the reading apparatus;
FIG. 86 is a flowchart showing one example of operation of the reading apparatus;
FIG. 87 is a flowchart showing one example of operation of the reading apparatus;
FIG. 88 is a schematic diagram showing one example of an evaluation member;
FIG. 89 is a schematic diagram showing one example of the evaluation member;
FIG. 90 is a schematic diagram showing one example of the evaluation member;
FIG. 91 is a schematic diagram showing one example of the evaluation member;
FIG. 92 is a flowchart showing one example of operation of the reading apparatus;
FIG. 93 is a schematic diagram showing one example of an evaluation whole image;
FIG. 94 is a schematic diagram showing one example of the evaluation whole image;
FIG. 95 is a schematic diagram showing one example of the evaluation whole image;
FIG. 96 is a schematic diagram showing one example of the evaluation whole image;
FIG. 97 is a schematic diagram illustrating one example of a configuration of the reading apparatus;
FIG. 98 is a schematic diagram illustrating one example of a configuration of the reading apparatus;
FIG. 99 is a schematic diagram illustrating one example of a configuration of the reading apparatus;
FIG. 100 is a schematic diagram illustrating one example of a configuration of the reading apparatus;
FIG. 101 is a schematic diagram illustrating one example of a configuration of the reading apparatus; and
FIG. 102 is a schematic diagram showing one example of a configuration of the reading apparatus.

### DESCRIPTION OF THE PREFERRED EMBODIMENTS

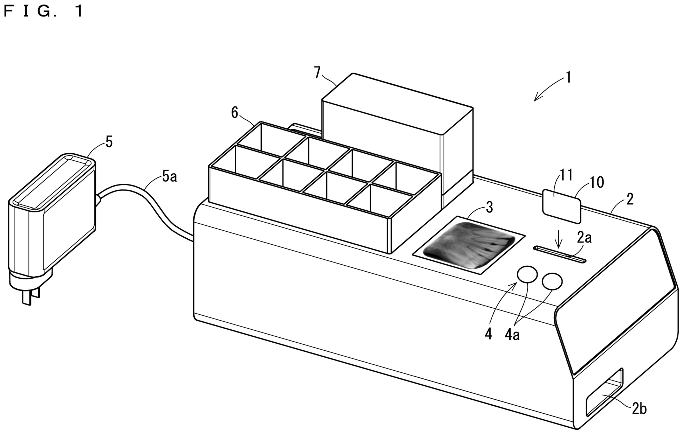

FIG. 1 is a schematic diagram illustrating one example of appearance of a reading apparatus 1. The reading apparatus 1 is an apparatus that reads, from an imaging plate 10 on which a radiograph is recorded, the radiograph. It can be said that the reading apparatus 1 is an apparatus that detects the radiograph recorded on the imaging plate 10.

The imaging plate 10 is a recording medium which is flat to include a radiograph formation layer 11 and on which the radiograph is recorded. The imaging plate 10 has a substantially rectangular flat shape with four rounded corners, for example. The radiograph formation layer 11 is a layer in which energy of emitted radiation is stored and which emits photostimulated light responsive to the stored energy. For example, the radiograph formation layer 11 is formed by applying a photostimulable phosphor to one main surface of a film formed of resin. As radiation with which the radiograph formation layer 11 is irradiated, an X-ray is used, for example. When the imaging plate 10 is irradiated with an X-ray from an X-ray generator having passed through an imaging object, energy responsive to intensity of the X-ray is stored in the radiograph formation layer 11. The intensity of the X-ray is based on distribution of an X-ray absorption region of the imaging object, so that distribution of the energy stored in the radiograph formation layer 11 is a radiograph of the imaging object by the X-ray. As described above, the radiograph by the X-ray is recorded on the imaging plate 10 as a latent image, for example. The reading apparatus 1 reads the radiograph from the radiograph formation layer 11, and generates an image signal (also referred to as image data) representing the read radiograph.

In this example, the imaging plate 10 is irradiated with radiation in a state of being inserted into a person's mouth, for example. The imaging plate 10 is thus sized to be insertable into the person's mouth. A radiograph of teeth is recorded on the radiograph formation layer 11 of the imaging plate 10, for example. Application of the imaging plate 10 is not limited to this application.

A main surface of the imaging plate 10 on a side of the radiograph formation layer 11 is hereinafter also referred to as a front surface. A main surface of the imaging plate 10 opposite the front surface is also referred to as a back surface.

As illustrated in FIG. 1, the reading apparatus 1 includes a housing 2, for example. Components to read the radiograph from the imaging plate 10 are contained in the housing 2. The components will be described below.

The housing 2 has an inlet 2a and an outlet 2b. The inlet 2a is formed in an upper surface of the housing 2, for example. A user of the reading apparatus 1 can insert the imaging plate 10 into the housing 2 through the inlet 2a. The radiograph is read from the imaging plate 10 in the housing 2. The outlet 2b is formed in a lower portion of one side surface of the housing 2, for example. The imaging plate 10 (also referred to as the read imaging plate 10) from which the radiograph has been read is discharged to the outlet 2b. The user of the reading apparatus 1 can retrieve the read imaging plate 10 through the outlet 2b.

In this example, the reading apparatus 1 can erase the radiograph from the imaging plate 10 after reading the radiograph from the imaging plate 10. The imaging plate 10 from which the radiograph has been erased is discharged to the outlet 2b, for example.

An operation unit 4 that receives an operation from the user is provided to the housing 2, for example. The operation unit 4 includes a plurality of operation buttons 4a, for example. The operation buttons 4a are hardware buttons, for example. The operation buttons 4a include a power supply button and a start button to provide instructions to start reading, for example. The operation unit 4 may include a touch sensor that detects a touch operation of the user.

A display 3 is provided to the housing 2, for example. The display 3 is configured by a liquid crystal display panel or an organic electro-luminescence (EL) display panel, for example. The display 3 can display various pieces of information, such as characters, symbols, graphics, and images. The display 3 may display the radiograph (i.e., a detected radiograph) read from the imaging plate 10.

When the operation unit 4 includes the touch sensor, the touch sensor and the display 3 may constitute a touch panel display having a display function and a touch detection function. In this case, at least one of the plurality of operation buttons 4a may be replaced with a software button displayed on the touch panel display, or the operation unit 4 may not include the plurality of operation buttons 4a. The reading apparatus 1 may not include the display 3.

Plate containment cases 6 and 7 that can contain the imaging plate 10 are provided to the housing 2, for example. The plate containment cases 6 and 7 are provided to the upper surface of the housing 2, for example. The plate containment case 6 is a compartmentalized case, and the plate containment case 7 is a case with a lid. The reading apparatus 1 may not include at least one of the plate containment cases 6 and 7.

A cable 5a of an AC adapter 5 extends outward from the housing 2. Power is supplied from the AC adapter 5 to each component of the reading apparatus 1. The reading apparatus 1 may include not only the AC adapter 5 but also a battery that supplies power to each component of the reading apparatus 1. Alternatively, the reading apparatus 1 may include the battery in place of the AC adapter 5.

### <One Example of Mechanism in Housing>

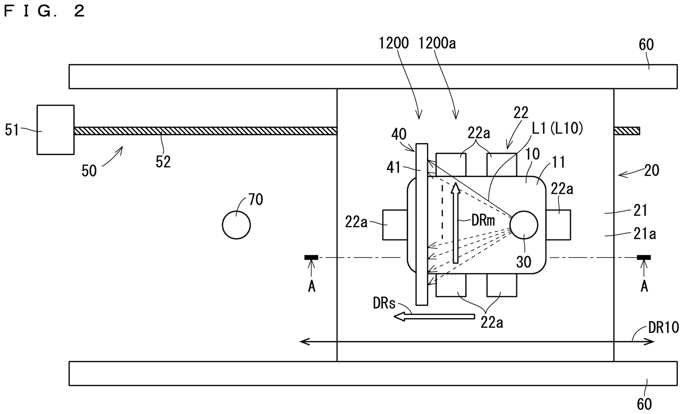

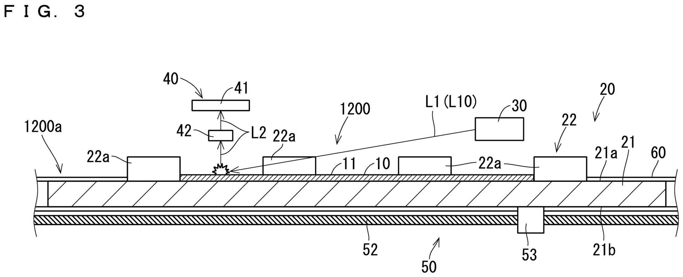

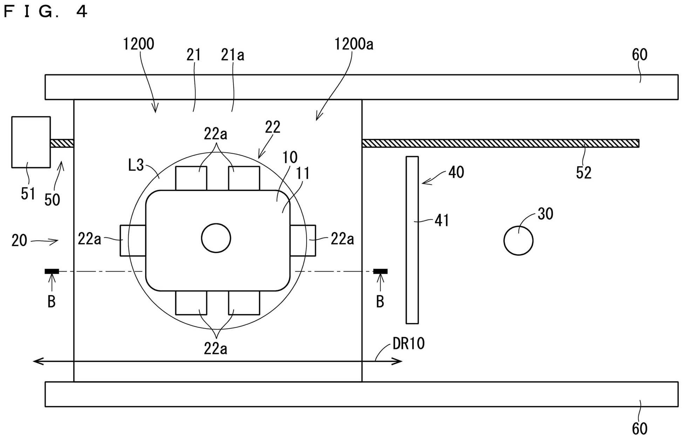

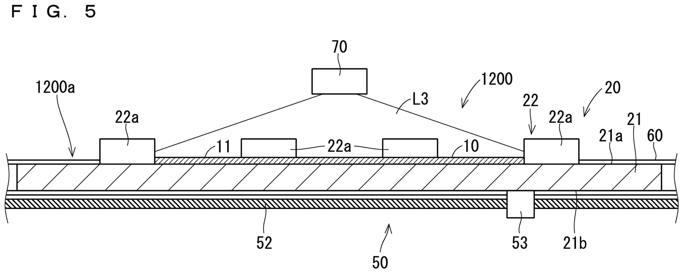

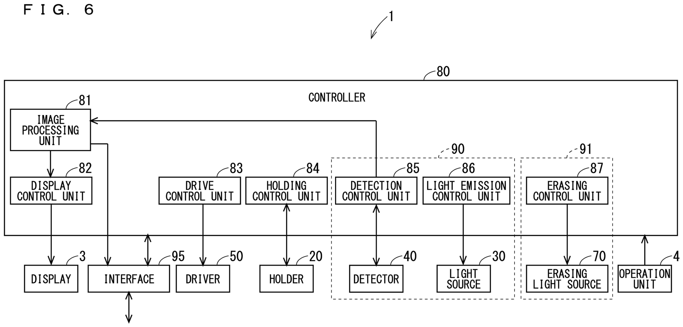

FIGs. 2 to 5 are schematic diagrams each illustrating one example of a configuration in the housing 2. FIG. 3 is a schematic diagram illustrating one example of a cross-sectional configuration along the line A-A of FIG. 2. FIG. 5 is a schematic diagram illustrating one example of a cross-sectional configuration along the line B-B of FIG. 4. FIG. 6 is a block diagram mainly showing one example of a configuration of a controller 80 of the reading apparatus 1. As will be described below, a holder 20 that holds the imaging plate 10 can be moved along a predetermined direction DR10 in the housing 2. FIG. 4 illustrates the imaging plate 10 and the holder 20 having been moved from a state illustrated in FIG. 2.

As illustrated in FIGs. 2 to 6, the reading apparatus 1 includes the holder 20, a light source 30, a detector 40, a driver 50, a pair of guides 60, an erasing light source 70, the controller 80, and an interface 95, for example. These components are contained in the housing 2.

### <Controller>

The controller 80 can manage operation of the reading apparatus 1 in an integrated manner, and can be said to be a control circuit. The controller 80 can control the display 3, the holder 20, the light source 30, the detector 40, the driver 50, the erasing light source 70, and the interface 95, for example. The controller 80 can also perform processing in response to a user operation received by the operation unit 4.

The controller 80 is configured by a computer device including at least one processor and a storage, for example. The at least one processor of the controller 80 may include a central processing unit (CPU) or may include a processor other than the CPU. The at least one processor of the controller 80 executes a program in the storage (also referred to as a storage circuit) to perform various functions described below.

The at least one processor of the controller 80 executes the program in the storage to form, as functional blocks, an image processing unit 81, a display control unit 82, a drive control unit 83, a holding control unit 84, a detection control unit 85, a light emission control unit 86, and an erasing control unit 87, for example.

The image processing unit 81 can perform image processing on an image signal, which will be described below, output from the detector 40, for example. The display control unit 82 can control display of the display 3. The drive control unit 83 can control the driver 50. The holding control unit 84 can control the holder 20. The detection control unit 85 can control the detector 40. The light emission control unit 86 can control the light source 30. The erasing control unit 87 can control the erasing light source 70.

Some or all of the functions of the controller 80 may be performed by a hardware circuit without the need for software (i.e., a program) to perform the functions. For example, some or all of the functions of the image processing unit 81 may be performed by a hardware circuit without the need for software to perform the functions. The image processing unit 81 may be an image processing circuit independent of the other components. Some or all of the functions of the display control unit 82 may be performed by a hardware circuit without the need for software to perform the functions. The display control unit 82 may be a display control circuit independent of the other components. Some or all of the functions of the drive control unit 83 may be performed by a hardware circuit without the need for software to perform the functions. The drive control unit 83 may be a drive control circuit independent of the other components. Some or all of the functions of the holding control unit 84 may be performed by a hardware circuit without the need for software to perform the functions. The holding control unit 84 may be a holding control circuit independent of the other components. Some or all of the functions of the detection control unit 85 may be performed by a hardware circuit without the need for software to perform the functions. The detection control unit 85 may be a detection control circuit independent of the other components. Some or all of the functions of the light emission control unit 86 may be performed by a hardware circuit without the need for software to perform the functions. The light emission control unit 86 may be a light emission control circuit independent of the other components. Some or all of the functions of the erasing control unit 87 may be performed by a hardware circuit without the need for software to perform the functions. The erasing control unit 88 may be an erasing control circuit independent of the other components.

### <Interface>

The interface 95 can communicate with a device external to the housing 2 (hereinafter also referred to as an external device), and can be said to be an interface circuit, a communication circuit, or a communication unit. The external device may include a personal computer, a mobile phone, such as a smartphone, and other computer devices. The external device may include a data recording medium (e.g., flash memory) removable from the reading apparatus 1. The interface 95 can receive a signal from the external device, and input the received signal to the controller 80. The interface 95 can also transmit a signal from the controller 80 to the external device. For example, the interface 95 can transmit the image signal on which the image processing has been performed by the image processing unit 81 of the controller 80 to the external device. The interface 95 may communicate with the external device by wire or wirelessly. Communication between the interface 95 and the external device may conform to Ethernet, Universal Serial Bus (USB), WiFi, or other standards.

### <Holder>

The holder 20 holds the imaging plate 10 inserted through the inlet 2a of the housing 2. The holder 20 includes a support plate 21 that supports the imaging plate 10 and a fixture 22 that fixes a position of the imaging plate 10 supported by the support plate 21, for example.

The support plate 21 has a main surface 21a (also referred to as a support surface 21a) that supports the back surface of the imaging plate 10 and a main surface 21b (also referred to as a back surface 21b) opposite the main surface 21a. The fixture 22 includes a plurality of fixing portions 22a to be close to a peripheral edge portion of the imaging plate 10, for example. It can be said that the fixture 22 is a fixing member. The plurality of fixing portions 22a are to be close to the peripheral edge portion of the imaging plate 10 to surround the peripheral edge portion. The position (i.e., a relative position) and an orientation (i.e., a relative orientation) of the imaging plate 10 relative to the support plate 21 are thereby fixed. In this example, two fixing portions 22a are to be close to each of long sides of the imaging plate 10, and one fixing portion 22a is to be close to each of short sides of the imaging plate 10, for example.

Each of the fixing portions 22a can be moved between a close position where the fixing portion 22a is close to the imaging plate 10 supported by the support plate 21 and a spaced position where the fixing portion 22a is spaced apart from the imaging plate 10 supported by the support plate 21 through control performed by the holding control unit 84. With each of the fixing portions 22a being in the spaced position, the imaging plate 10 is inserted into the housing 2 through the inlet 2a, and is supported by the support plate 21. Each of the fixing portions 22a is then moved from the spaced position to the close position, so that the position and the orientation of the imaging plate 10 are fixed by the fixture 22. Each of the fixing portions 22a is in contact with the peripheral edge portion of the imaging plate 10 when being in the close position, for example.

At least one of the fixing portions 22a may not be in contact with the peripheral edge portion of the imaging plate 10 when being in the close position. A configuration of the fixture 22 is not limited to the above-mentioned configuration. A configuration of the holder 20 is also not limited to the above-mentioned configuration.

### <Driver and Pair of Guides>

The driver 50 can move the holder 20 along the predetermined direction DR10 through control performed by the drive control unit 83. The imaging plate 10 held by the holder 20 can thereby be moved along the predetermined direction DR10. It can be said that the driver 50 can move the imaging plate 10 along the predetermined direction DR10 via the holder 20.

The pair of guides 60 extends along the predetermined direction DR10 with the holder 20 being sandwiched therebetween. Each of the guides 60 has, in the inside thereof, a groove extending along the predetermined direction DR10. Side edge portions of the support plate 21 opposing each other fit into respective grooves in the insides of the guides 60. The pair of guides 60 can thus guide the holder 20 so that the holder 20 is moved along the predetermined direction DR10. A configuration of the guides 60 is not limited to this configuration.

The driver 50 is configured by a ball screw mechanism including a motor 51, a threaded shaft 52, and a nut 53, for example. The motor 51 is controlled by the drive control unit 83. The threaded shaft 52 is a rod-like member having threads in the periphery thereof. The threaded shaft 52 extends along the predetermined direction DR10, and is rotated by the motor 51. The nut 53 is fixed to the holder 20. The nut 53 is fixed to the back surface 21b of the support plate 21 of the holder 20, for example. The threaded shaft 52 is screwed into the nut 53. The threaded shaft 52 is rotated in a forward direction or in a reverse direction in response to rotation of the motor 51 in the forward direction or in the reverse direction. The holder 20 is moved to one side along the predetermined direction DR10 in response to rotation of the threaded shaft 52 in the forward direction. In this case, the pair of guides 60 guides the holder 20 so that the holder 20 is moved to the one side. On the other hand, the holder 20 is moved to the other side along the predetermined direction DR10 in response to rotation of the threaded shaft 52 in the reverse direction. In this case, the pair of guides 60 guides the holder 20 so that the holder 20 is moved to the other side. A configuration of the driver 50 is not limited to this configuration.

The driver 50 can move the holder 20 holding the imaging plate 10 to a reading start position where reading of the radiograph from the imaging plate 10 starts. When reading of the radiograph from the imaging plate 10 ends, the driver 50 can move the holder 20 holding the imaging plate 10 to an erasing position where the radiograph is erased from the imaging plate 10. FIGs. 2 and 3 illustrate reading of the radiograph from the imaging plate 10. FIGs. 4 and 5 illustrate erasing of the radiograph from the imaging plate 10.

### <Light Source and Detector>

In this example, the light source 30, the light emission control unit 86 that controls the light source 30, the detector 40, and the detection control unit 85 that controls the detector 40 constitute a light measuring instrument 90 that reads the radiograph from the front surface of the imaging plate 10 as shown in FIG. 6. The light source 30, the detector 40, the detection control unit 85, and the light emission control unit 86 constituting the light measuring instrument 90 may be contained in a single case to be unitized, or may not be contained in the single case.

The light source 30 can irradiate the imaging plate 10 held by the holder 20 with excitation light L10 to excite the radiograph formation layer 11. An object of irradiation with the excitation light L10 is the imaging plate 10 as a light receiver in biological radiography, for example. The light source 30 emits the excitation light L10 toward the support surface 21a of the holder 20. The light source 30 can scan the imaging plate 10 with the excitation light L10 in a single direction (also referred to as a main scanning direction DRm). The main scanning direction DRm is a direction perpendicular to the predetermined direction DR10. That is to say, the main scanning direction DRm is a direction perpendicular to a direction of movement of the holder 20. The light source 30 can irradiate not only the imaging plate 10 but also a region around the imaging plate 10 with the excitation light L10.

In the present disclosure, light acting on an object is referred to as acting light L1. Light generated by the acting light L1 acting on the object is referred to as acted light L2. The excitation light L10 is one example of the acting light L1. Light not having an excitation force to generate the photostimulated light but generating reflected light from the object is another example of the acting light L1. It can be said that the acting light L1 is light including at least one of the excitation light and the acting light that is not the excitation light. The acted light L2 is light emitted from the object by being acted on by the acting light L1.

The excitation light L10 is visible laser light, for example. The excitation light L10 may be red laser light, or may be laser light of another color, for example. The detector 40 detects the acted light L2 from the imaging plate 10 generated by irradiation with the excitation light L10 as the acting light L1, and outputs an electric signal responsive to the intensity of the detected acted light L2, for example. The detector 40 also detects the acted light L2 from outside the imaging plate 10 generated by irradiation with the excitation light L10, and outputs an electric signal responsive to the intensity of the detected acted light L2.

The light source 30 includes a laser generator that generates and outputs the excitation light L10 and a scanner that scans the imaging plate 10 with the excitation light L10 in the main scanning direction DRm, for example. The laser generator includes a semiconductor laser oscillator, for example, and is controlled by the light emission control unit 86. The laser generator may include a laser diode, or may include another semiconductor laser. The scanner includes a micro electro mechanical systems (MEMS) mirror that reflects the excitation light L10 from the laser generator toward the radiograph formation layer 11 of the imaging plate 10, for example. The MEMS mirror changes a reflection angle of the excitation light L10 so that a point of irradiation with the excitation light L10 on the radiograph formation layer 11 is moved in the main scanning direction DRm through control performed by the light emission control unit 86. The scanner may include another mirror, such as a galvanometer mirror, in place of the MEMS mirror.

The detector 40 detects the acted light L2 from a position as a target of irradiation with the excitation light L10. The detector 40 includes an optical filter 42 that the acted light L2 (see FIG. 3) from the position as the target of irradiation with the excitation light L10 enters and a sensor 41 that detects the acted light L2 emitted from the optical filter 42, for example. The sensor 41 is controlled by the detection control unit 85. The optical filter 42 is disposed to oppose a detection surface of the sensor 41 where the acted light L2 is detected and to be located between the main surface 21a of the support plate 21 and the detection surface. That is to say, the optical filter 42 is disposed between the position as the target of irradiation with the excitation light L10 and the detection surface. The acted light L2 from the position as the target of irradiation with the excitation light L10 first enters the optical filter 42, and the acted light L2 having undergone filtration is emitted from the optical filter 42, and enters the detection surface of the sensor 41.

A region of the radiograph formation layer 11 in which energy of radiation is stored due to irradiation with radiation is excited by the excitation light L10. It can thus be said that a region of the radiograph formation layer 11 in which energy of radiation is stored and which is in a range of the target of irradiation with the excitation light L10 is an excited region excited by the excitation light L10. It can be said that the excited region is a radiograph region, a latent image region, or an image recording region as the radiograph is recorded in the excited region as the latent image.

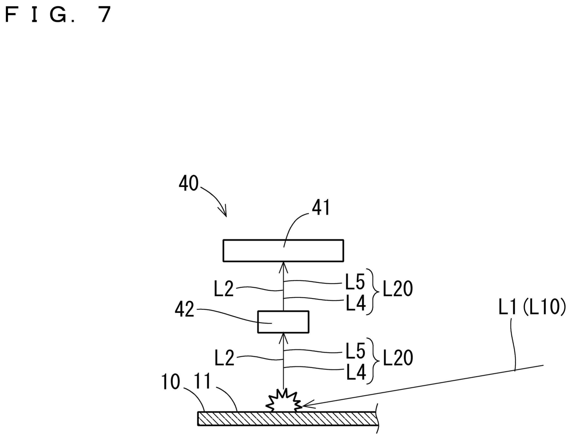

FIG. 7 is a schematic diagram illustrating an enlarged view around the light detector 40 of FIG. 3. When the excited region of the radiograph formation layer 11 is irradiated with the excitation light L10, the excited region emits light in response to distribution of energy stored in the excited region, and photostimulated light L5 is emitted from the excited region. The photostimulated light L5 is visible blue light, for example. When the excited region of the radiograph formation layer 11 is irradiated with the excitation light L10, reflected light (hereinafter also referred to as reflected light L4) of the excitation light L10 in the excited region is also generated. The reflected light L4 may be referred to as excited region reflected light L4. The emitted light L2 can be thought to be light of which luminescence start position is at the imaging plate 10 without reflection. The photostimulated light L5 is an example of the emitted light L2.

Light from the excited region (hereinafter also referred to as excited region light L20) includes the photostimulated light L5 emitted from the excited region and the reflected light L4 from the excited region. It can be said that the excited region light L20 is radiograph region light, latent image region light, or image recording region light. The excited region light L20 is light including at least the photostimulated light L5. When the excited region refers to the latent image region in the range of the target of irradiation with the excitation light L10, a region excited by irradiation with the excitation light L10, and, specifically, a region excited to emit the photostimulated light L5, the excited region light L20 is light including at least the photostimulated light L5 from the excited region. While the excited region light L20 is light including the photostimulated light L5 and the reflected light L4 in the above-mentioned example, the excited region light L20 can include only the photostimulated light L5 as will be described below.

The acted light L2 entering the optical filter 42 includes the excited region light L20. The excited region light L20 enters the sensor 41 after undergoing filtering processing performed by the optical filter 42. Transmittance of the photostimulated light L5 (also referred to as photostimulated light transmittance) of the optical filter 42 is extremely high in this example. The optical filter 42 thus sufficiently transmits the photostimulated light L5 from the excited region of the imaging plate 10, and emits the transmitted photostimulated light L5 to the sensor 41. On the other hand, transmittance of the excitation light L10 (also referred to as excitation light transmittance) of the optical filter 42 is lower than the photostimulated light transmittance. For example, the optical filter 42 having an excitation light transmittance of approximately 10% of the photostimulated light transmittance may be used. The optical filter 42 attenuates the reflected light L4 of the excitation light L10 from the excited region of the imaging plate 10 toward the sensor 41. The optical filter 42 attenuates the excitation light L10, but transmits the excitation light L10 to some extent. The sensor 41 thus not only detects the photostimulated light L5 but also detects the reflected light L4 of the excitation light L10 from the excited region to some extent in this example. The excited region light L20 emitted from the optical filter 42 thus includes the photostimulated light L5 and the reflected light L4.

The sensor 41 can detect the excited region light L20 having been transmitted by the optical filter 42, and output an electric signal responsive to the intensity of the detected excited region light L20. The sensor 41 may be configured by a plurality of photodiodes, or may be configured by a photomultiplier, for example. In this example, the acted light L2 detected by the sensor 41 (i.e., the acted light L2 detected by the detector 40) includes the photostimulated light L5 and the reflected light L4, for example.

When the reading apparatus 1 performs processing (also referred to as reading processing) of reading the radiograph from the imaging plate 10, the holder 20 holding the imaging plate 10 is transferred to the reading start position by the driver 50. The light measuring instrument 90 then starts the reading processing. In the reading processing, the light source 30 repeatedly performs processing (also referred to as main scanning direction scanning) of scanning the imaging plate 10 with the excitation light L10 in the main scanning direction DRm through control performed by the light emission control unit 86. On the other hand, in the reading processing, the driver 50 moves the holder 20 holding the imaging plate 10 in one direction DRs (also referred to as a subscannig direction DRs) along the predetermined direction DR10. The subscannig direction DRs is a direction perpendicular to the main scanning direction DRm. The main scanning direction scanning is repeatedly performed during movement of the holder 20 in the subscannig direction DRs to two-dimensionally irradiate the radiograph formation layer 11 of the imaging plate 10 with the excitation light L10 for raster scanning of the radiograph formation layer 11. All regions of the radiograph formation layer 11 are thus sequentially irradiated with the excitation light L10 to scan all the regions of the radiograph formation layer 11 with the excitation light L10 in the reading processing. During raster scanning of the radiograph formation layer 11 with the excitation light L10, the sensor 41 of the detector 40 detects the excited region light L20 (i.e., light including the photostimulated light L5) sequentially coming from the radiograph formation layer 11 in response to raster scanning to read the radiograph from the radiograph formation layer 11. The sensor 41 outputs an image signal representing the read radiograph (i.e., the detected radiograph) to the detection control unit 85 as a result of detection of the excited region light L20 during raster scanning with the excitation light L10. The image signal includes luminance values (i.e., pixel values) of a plurality of pixels representing the read radiograph. The sensor 41 outputs a gray-scale image signal, for example. The radiograph read by the detector 40 is hereinafter also referred to as a detected radiograph.

Scanning with the excitation light L10 is only required to be performed two-dimensionally over the whole region of a detection target range of the imaging plate 10 by determining coordinates thereof, and the main scanning direction DRm and the subscannig direction DRs may not be perpendicular to each other. For example, the main scanning direction DRm and the subscannig direction DRs may cross each other at an angle other than a right angle. One or both of the main scanning direction DRm and the subscannig direction DRs may be set to a curvilinear direction.

In this example, in a state of the imaging plate 10 being properly held by the holder 20, a transverse direction of the imaging plate 10 is parallel to the main scanning direction DRm, and a longitudinal direction of the imaging plate 10 is parallel to the subscannig direction DRs as illustrated in FIGs. 2 and 4. An orientation of the imaging plate in the state of the imaging plate 10 being properly held by the holder 20 is herein referred to as a reference orientation, and, in this example, the reference orientation is an orientation of the imaging plate 10 in which the transverse direction of the imaging plate 10 is parallel to the main scanning direction DRm, and the longitudinal direction of the imaging plate 10 is parallel to the subscannig direction DRs. The reference orientation is not limited to this orientation.

The imaging plate 10 is basically held by the holder 20 in the reference orientation. When the holder 20 does not properly hold the imaging plate 10 due to a malfunction of the fixture 22 and the like, the imaging plate 10 is held by the holder 20 in an orientation tilting relative to the reference orientation.

In this example, the sensor 41 outputs a greater luminance value when detected light has a higher intensity, for example. The photostimulated light L5 having a higher intensity is emitted from a portion of the radiograph formation layer 11 of the imaging plate 10 which is irradiated with radiation having a higher intensity and in which more energy is stored. On the other hand, the intensity of the reflected light L4 from the excited region is substantially constant regardless of the stored energy when the excitation light L10 has a constant intensity. The sensor 41 thus outputs a greater luminance value for an image based on detection of the excited region light L20 from the portion in which more energy is stored in the excited region of the radiograph formation layer 11.

Consider a case where a radiograph of teeth is recorded on the radiograph formation layer 11, for example. In this case, the excited region light L20 from a portion in which the radiograph of the teeth is recorded, that is, a portion irradiated with radiation having transmitted the teeth in the excited region of the radiograph formation layer 11 has a relatively low intensity. The sensor 41 thus outputs a relatively small luminance value for a portion of the detected radiograph in which the teeth appear. On the other hand, the excited region light L20 from a portion (also referred to as a direct irradiation portion) directly irradiated with radiation in the excited region of the radiograph formation layer 11 has a relatively high intensity. The sensor 41 thus outputs a relatively great luminance value for an image corresponding to the direct irradiation portion of the radiograph formation layer 11 of the detected radiograph.

As described above, in this example, the sensor 41 detects not only the photostimulated light L5 emitted from the excited region of the imaging plate 10 but also the reflected light L4 from the excited region of the imaging plate 10 to some extent. A luminance value for the detected radiograph output from the sensor 41 thus includes a luminance value (also referred to as a photostimulated light corresponding luminance value) responsive to the intensity of the detected photostimulated light L5 and a luminance value (also referred to as a reflected light corresponding luminance value) responsive to the detected reflected light L4.

The photostimulated light corresponding luminance value is a value equal to or greater than ten times the reflected light corresponding luminance value under a standard dose condition and source image distance (SID) condition, for example. The detected radiograph based on the image signal output from the detector 40 is less affected by the reflected light L4, and the reflected light L4 is less likely to interfere with reading of the radiograph recorded on the imaging plate 10 and processing after the reading. The photostimulated light L5 has a higher luminance intensity ratio than the reflected light L4 in the excited region light L20 detected by the sensor 41, so that the influence of the reflected light L4 is small. Thus, by outputting an electric signal responsive to the intensity of the detected excited region light L20, the detector 40 outputs an electric signal responsive to the intensity of the photostimulated light L5.

In this example, the radiograph formation layer 11 of the imaging plate 10 can partially include an unexposed portion in which energy responsive to irradiation with radiation is not stored. For example, when the imaging plate 10 is irradiated with radiation through the imaging object, the radiograph formation layer 11 can include the unexposed portion because a portion originally to be irradiated with radiation is sometimes not irradiated with radiation due to displacement of a light source that emits radiation and the like. The unexposed portion is also referred to as a cone cut. In this example, the imaging plate 10 is inserted into a mouth, and is irradiated with radiation. All or part of the imaging plate 10 is thus hidden in the mouth, so that the all or part of the imaging plate 10 can fall outside a range of irradiation with radiation even when an operator might believe that the light source that emits radiation is positioned properly. The cone cut is caused in such a case. The radiograph formation layer 11 can partially include the unexposed portion as the radiograph is unintentionally erased from the portion irradiated with radiation. For example, the imaging plate 10 on which the radiograph is recorded is typically stored after being covered not to be irradiated with ambient light. When the imaging plate 10 is not properly covered, however, a portion of the radiograph formation layer 11 can be irradiated with ambient light during storage of the imaging plate 10 to unintentionally erase the radiograph recorded in the portion. Also in this case, the radiograph formation layer 11 partially includes the unexposed portion (a portion once exposed but returning to an unexposed state due to erasing herein).

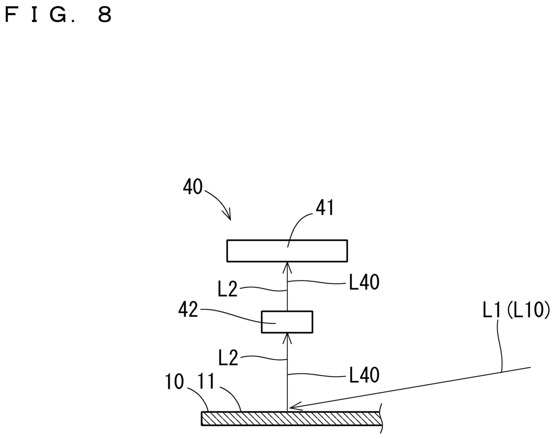

Energy of radiation is not stored in the unexposed portion of the imaging plate 10, so that the unexposed portion is not excited even when being irradiated with the excitation light L10. It can thus be said that the unexposed portion is a region that is not the excited region, that is, an unexcited region. Even when the unexposed portion (i.e., the unexcited region) of the imaging plate 10 is irradiated with the excitation light L10, the photostimulated light L5 is not emitted from the unexposed portion. Thus, when the unexposed portion is irradiated with the excitation light L10, the detector 40 detects the reflected light of the excitation light L10 from the unexposed portion without detecting the photostimulated light L5 as illustrated in FIG. 8. The image signal output from the detector 40 can thus include luminance values of a plurality of pixels representing an image in the unexposed portion, that is, an image based on detection of the reflected light of the excitation light L10 from the unexposed portion. The reflected light of the excitation light L10 from a region, such as an unexposed region, of the imaging plate 10 in which energy of radiation is not stored is hereinafter referred to as reflected light L40.

The reflected light L40 from the unexposed portion enters the sensor 41 through the optical filter 42. A whole image based on the image signal output from the detector 40 as a result of detection of the excited region light L20 and the reflected light L40 sometimes includes not only the detected radiograph based on detection of the excited region light L20 but also the image in the unexposed portion (also referred to as an unexposed region image). A luminance value for the detected radiograph output from the detector 40 is equal to or greater than ten times a luminance value for the unexposed region image output from the detector 40 under the standard dose condition and SID condition, for example. It can be said that the reflected light L40 from the unexposed portion, that is, the unexcited region is unexcited region light. The reflected light L40 may be referred to as unexcited region reflected light L40. The acted light L2 detected by the detector 40 also includes the reflected light L40.

When no radiograph is recorded on the imaging plate 10, the sensor 41 detects the reflected light L40 from the position as the target of irradiation with the excitation light L10 without detecting the excited region light L20. As with the reflected light L40 from the unexcited region and the reflected light L4 from the excited region, the reflected light of the excitation light L10 from the imaging plate 10 is sometimes expressed as non-photostimulable reflected light in contrast with the photostimulated light L5. The reflected light of the excitation light L10 is also simply referred to as reflected light regardless of whether the excitation light L10 is reflected from the imaging plate 10 or reflected from outside the imaging plate 10.

In the present disclosure, a symbol IP representing the imaging plate 10 may be attached to a name of light from the imaging plate 10 or a name of light with which the imaging plate 10 is irradiated. For example, the excited region light L20 may be referred to as IP excited region light L20. The unexcited region light may be referred to as IP unexcited region light. The photostimulated light L5 may be referred to as IP photostimulated light L5. The reflected light L4 may be referred to as IP reflected light L4 (also referred to as IP excited region reflected light L4), and the reflected light L40 may be referred to as IP reflected light L40 (also referred to as IP unexcited region reflected light L40). The non-photostimulable reflected light may be referred to as IP non-photostimulable reflected light. The acting light L1 may be referred to as IP acting light L1, and the acted light L2 may be referred to as IP acted light L2. The IP acted light L2 may also simply be referred to as IP light L2. The excitation light L10 may be referred to as IP excitation light L10. When there is no particular need to distinguish between the reflected light L4 and the reflected light L40, each of them or a combination of them is hereinafter referred to as IP reflected light.

The excitation light L10 includes a component to generate the photostimulated light L5. The excitation light L10 also includes a component to generate the IP reflected light. The light source 30 is a photostimulated light generation light source as a source of light including the component to generate the photostimulated light L5. The light source 30 is also a reflected light generation light source as a source of light including the component to generate the IP reflected light. For example, the light source 30 as the photostimulated light generation light source is a first light source, and the light source 30 as the reflected light generation light source is a second light source. The photostimulated light generation light source and the reflected light generation light source are not required to be integral with each other, and may separately be provided. The reflected light generation light source is only required to emit light, which is not limited to the excitation light L10, to generate reflected light from an object.

The sensor 41 is a photostimulated light detector that detects the photostimulated light L5, and is also a reflected light detector that detects the IP reflected light. For example, the sensor 41 as the photostimulated light detector that detects the photostimulated light is a first detector, and the sensor 41 as the reflected light detector that detects the reflected light is a second detector. The sensor 41 is a detector that detects the excited region light L20. Since the excited region light L20 is light including at least the photostimulated light L5, at least the photostimulated light L5 is detected by detecting the excited region light L20. The sensor 41 is the photostimulated light detector in that sense. The sensor 41 as the first detector may be considered as an excited region light detector. The excited region light is the IP acted light, so that the first detector may be considered as an IP excited region light detector that detects the IP excited region light L20. The second detector may be considered as an unexcited region light detector that detects the unexcited region light. When the unexcited region light is the IP acted light, the second detector may be considered as an IP unexcited region light detector that detects the IP unexcited region light. The photostimulated light detector and the reflected light detector are not required to be integral with each other, and may separately be provided.

In the present disclosure, an image formed by certain light, that is, an image based on detection of the certain light may be referred to by a name of an image to which a name of the certain light has been attached. For example, an image formed by the excited region light L20, that is, an image based on detection of the excited region light L20 may be referred to as an excited region light image. The above-mentioned detected radiograph acquired by the detector 40 is the excited region light image. An image formed by the reflected light, that is, an image based on detection of the reflected light may be referred to as a reflected light image. The reflected light image formed by the reflected light L40 from the unexposed portion is the above-mentioned unexposed region image acquired by the detector 40. An image formed by the non-photostimulable reflected light may be referred to as a non-photostimulable reflected light image, and an image formed by the acted light L2 may be referred to as an acted light image. There can thus be a photostimulated light image, a reflected light image, an excited region reflected light image, an unexcited region light image, an unexcited region reflected light image, and the like.

In the present disclosure, the symbol IP representing the imaging plate 10 may be attached to a name of an image relating to the imaging plate 10. For example, the excited region light image may be referred to as an IP excited region light image, an image formed by the IP reflected light (i.e., an image based on detection of the IP reflected light or an image acquired by detection of the IP reflected light) may be referred to as an IP reflected light image, the non-photostimulable reflected light image may be referred to as an IP non-photostimulable reflected light image, and the acted light image may be referred to as an IP acted light image. Similarly, there can be an IP photostimulated light image, an IP excited region reflected light image, an IP unexcited region light image, an IP unexcited region reflected light image, and the like. The IP acted light image may also simply be referred to as an IP image. The IP acted light image having been processed may be referred to as the IP acted light image (i.e., the IP image).

In the present disclosure, an image signal acquired as a result of detection of certain light may be referred to by a name of an image signal to which a name of the certain light has been attached. For example, an image signal acquired as a result of detection of the excited region light L20 may be referred to as an excited region light image signal. An image signal acquired as a result of detection of the reflected light may be referred to as a reflected light image signal. An image signal acquired as a result of detection of the non-photostimulable reflected light image may be referred to as a non-photostimulable reflected light image signal. An image signal acquired as a result of detection of the acted light L2 may be referred to as an acted light image signal. Similarly, there can be a photostimulated light image signal, an excited region reflected light image signal, an unexcited region light image signal, an unexcited region reflected light image signal, and the like.

In the present disclosure, the symbol IP representing the imaging plate 10 may be attached to a name of an image signal relating to the imaging plate 10. For example, the excited region light image signal may be referred to as an IP excited region light image signal, the image signal acquired as a result of detection of the reflected light may be referred to as an IP reflected light image signal, the non-photostimulable reflected light image signal may be referred to as an IP non-photostimulable reflected light image signal, and the acted light image signal may be referred to as an IP acted light image signal. The IP acted light image signal may also simply be referred to as an IP image signal. Similarly, there can be an IP photostimulated light image signal, an IP excited region reflected light image signal, an IP unexcited region light image signal, an IP unexcited region reflected light image signal, and the like.

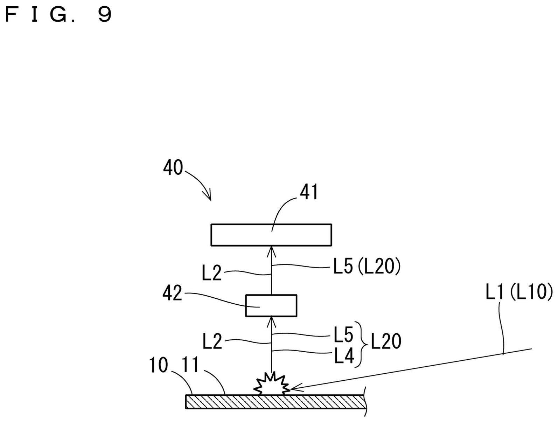

When the optical filter 42 completely blocks the reflected light L4 and transmits only the photostimulated light L5 as illustrated in FIG. 9, the acted light L2 detected by the sensor 41, in other words, the excited region light L20 detected by the sensor 41 is the photostimulated light L5. A luminance value responsive to the intensity of the acted light L2 (i.e., the excited region light L20) output from the sensor 41 is thus a luminance value responsive to the intensity of the photostimulated light L5. In an example of FIG. 9, an image based on the photostimulated light L5 detected by the detector 40 may be referred to as the photostimulated light image, or may be referred to as the IP photostimulated light image. An image signal output from the detector 40 as a result of detection of the photostimulated light L5 may be referred to as the photostimulated light image signal, or may be referred to as the IP photostimulated light image signal.

In this example, not only the imaging plate 10 but also a portion of the holder 20 outside the imaging plate 10 is irradiated with the excitation light L10 in the reading processing. An object irradiated with the excitation light L10 in the reading apparatus 1 is herein referred to as an irradiation object 1200. In this example, the irradiation object 1200 includes the holder 20 and the imaging plate 10 held by the holder 20. A main surface 1200a of the irradiation object 1200 on a side of the support surface 21a is referred to as a support side main surface 1200a. In this example, the support side main surface 1200a includes a surface of the radiograph formation layer 11 of the imaging plate 10, a surface of the fixture 22 of the holder 20, and a region of the support surface 21a of the support plate 21 of the holder 20 not covered with the imaging plate 10 and the fixture 22. A region of the support side main surface 1200a where an image formed by the acted light L2 therefrom is the IP image is referred to as an IP image region R100. It can be said that the IP image region R100 is an IP presence region of the support side main surface 1200a where the imaging plate 10 is present. A main surface of the irradiation object 1200 opposite the support side main surface 1200a matches the back surface 21b of the support plate 21.

Light from the IP image region R100 may be referred to as IP image region light. An image formed by the IP image region light may be referred to as an IP image region light image. An image signal output as a result of detection of the IP image region light may be referred to as an IP image region light image signal. The IP image region light can include only the excited region reflected light L4, can include only the unexcited region reflected light L40, or can include both the excited region reflected light L4 and the unexcited region reflected light L40.

In this example, an irradiation range (also referred to as an excitation light irradiation range) R120 of irradiation with the excitation light L10 on the support side main surface 1200a in the reading processing is a range greater than the IP image region R100 while including the IP image region R100. It can be said that the excitation light irradiation range R120 is a scanning range of scanning with the excitation light L10 in the reading processing. A detection range R110 of detection performed by the detector 40 on the support side main surface 1200a in the reading processing is also a range greater than the IP image region R100 while including the IP image region R100.

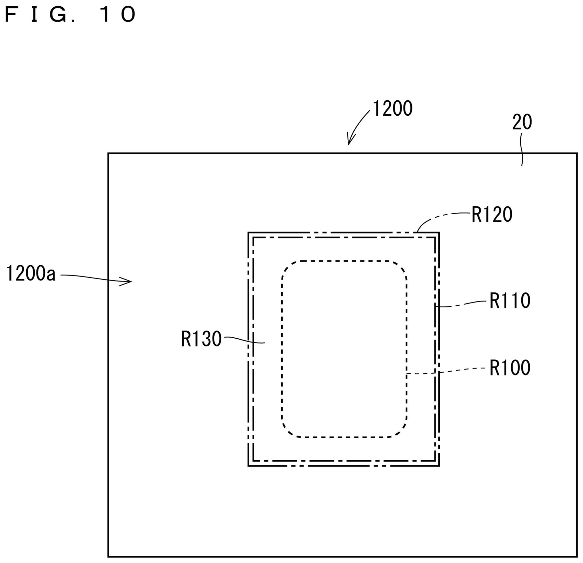

FIG. 10 is a schematic diagram illustrating one example of the excitation light irradiation range R120, the detection range R110, and the IP image region R100. In FIG. 10 and FIGs. 12 and 13 described below, illustration of the imaging plate 10 is omitted. As illustrated in FIG. 10, the excitation light irradiation range R120 and the detection range R110 are each a range greater than the IP image region R100 while including the IP image region R100. The excitation light irradiation range R120 and the detection range R110 have the same size, and are present at the same position, for example. The excitation light irradiation range R120 is illustrated to be slightly greater than the detection range R110 in FIG. 10 for the convenience of description.

As described above, the excitation light irradiation range R120 and the detection range R110 may be ranges matching each other. A configuration in which only the acted light L2 from the excitation light irradiation range R120 is detected is an example of a configuration in which the excitation light irradiation range R120 and the detection range R110 are ranges matching each other.

In the reading processing, the holder 20 holding the imaging plate 10 is moved in the subscannig direction DRs while the light source 30 repeatedly performs the main scanning direction scanning for raster scanning of the excitation light irradiation range R120 with the excitation light L10. The sensor 41 sequentially outputs luminance values responsive to positions of irradiation with the excitation light L10 in the detection range R110 in response to raster scanning with the excitation light L10.

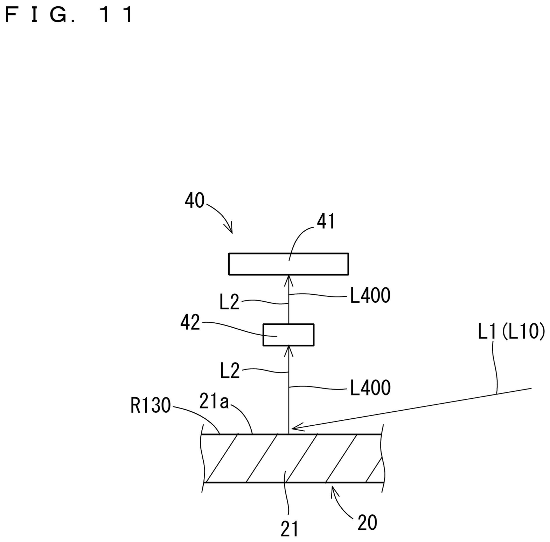

A region outside the IP image region R100 and inside the excitation light irradiation range R120 and the detection range R110 on the support side main surface 1200a is herein referred to as an IP outside region R130. In this example, the excitation light irradiation range R120 and the detection range R110 are each a range greater than the IP image region R100 while including the IP image region R100, so that the sensor 41 detects reflected light L400 of the excitation light L10 from the IP outside region R130. The IP outside region R130 includes at least portion of a surface of the fixture 22 and at least portion of a region of the support surface 21a of the support plate 21 not covered with the imaging plate 10 and the fixture 22, for example. The IP outside region R130 is included in a surface of the holder 20, specifically, a main surface of the holder 20 on a side of the imaging plate 10. FIG. 11 is a schematic diagram illustrating one example of detection of the reflected light L400 performed by the sensor 41. The acted light L2 detected by the detector 41 includes the reflected light L400.

Even when the IP outside region R130 is irradiated with the excitation light L10, the photostimulated light L5 is not emitted from the IP outside region R130. The detector 40 thus detects the reflected light L400 of the excitation light L10 from the IP outside region R130 without detecting the photostimulated light L5 when the IP outside region R130 is irradiated with the excitation light L10. Thus, in the reading processing, an image signal output from the detector 40 as a result of detection of the photostimulated light L5 and the reflected light includes luminance values of a plurality of pixels representing a reflected light image in the IP outside region R130, that is, a reflected light image based on detection of the reflected light L400 of the excitation light L10 from the IP outside region R130. In this example, the whole image (also referred to as an acquired whole image) based on the image signal output from the detector 40 includes not only the detected radiograph but also the reflected light image in the IP outside region R130. When the imaging plate 10 includes the unexposed portion, the acquired whole image includes the detected radiograph, the unexposed region image, and the reflected light image in the IP outside region R130. The reflected light image in the IP outside region R130 is hereinafter also referred to as an IP outside region image.

A region in the detection range R110 excluding the IP image region R100 may be referred to as an IP image region outside region. An image in the IP image region outside region may be referred to as an IP image region outside region image. The detection range R110 is set to include at least the IP image region R100. When the detection range R110 is set to a range being greater than the IP image region R100 while including the IP image region R100, the detection range R110 includes the IP image region R100 and the IP image region outside region. The IP image region R100 is a region of the imaging plate 10 where the excitation light L10 is received, and thus may be referred to as an IP light receiving region. A region in the detection range R110 excluding the IP light receiving region is the IP image region outside region, and thus the IP image region outside region may be referred to as an IP light receiving outside region.

In this example, processing of making the excitation light L10 less likely to be reflected has been performed in the IP outside region R130. For example, black anodizing has been performed in the IP outside region R130. The detector 40 is thus less likely to detect the reflected light L400 of the excitation light L10 in the IP outside region R130 than the reflected light L40 of the excitation light L10 in the unexposed portion of the imaging plate 10. In this example, the excitation light L10 is hardly reflected in the IP outside region R130 due to black anodizing. A luminance value for the unexposed region image output from the detector 40 is equal to or greater than three times a luminance value for the IP outside region image output from the detector 40, for example. Black anodizing may be performed in a region of the surface of the holder 20 other than the IP outside region R130. For example, black anodizing may be performed in all regions on the surface of the holder 20. Processing of making the excitation light L10 less likely to be reflected may be performed at least in a range of irradiation with the excitation light L10 of a portion of the support surface 21a and the fixture 22 of the holder 20 in the detection range R110, for example. When the portion of the support surface 21a and the fixture 22 of the holder 20 in the detection range R110 includes a range not irradiated with the excitation light L10, processing of making light less likely to be reflected may be performed in the range.

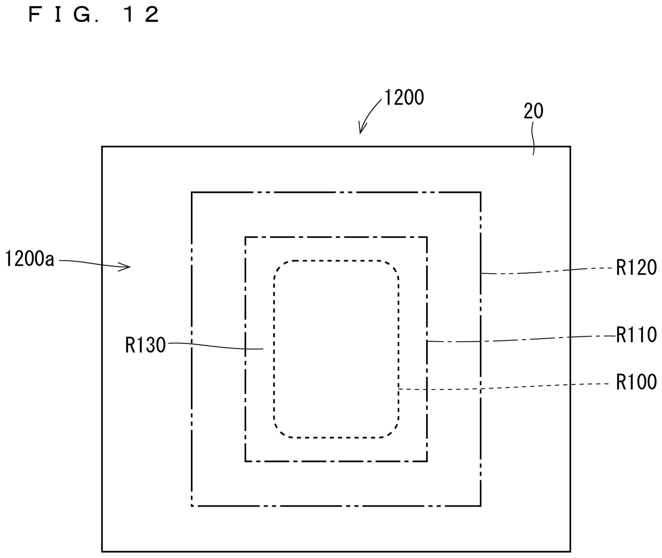

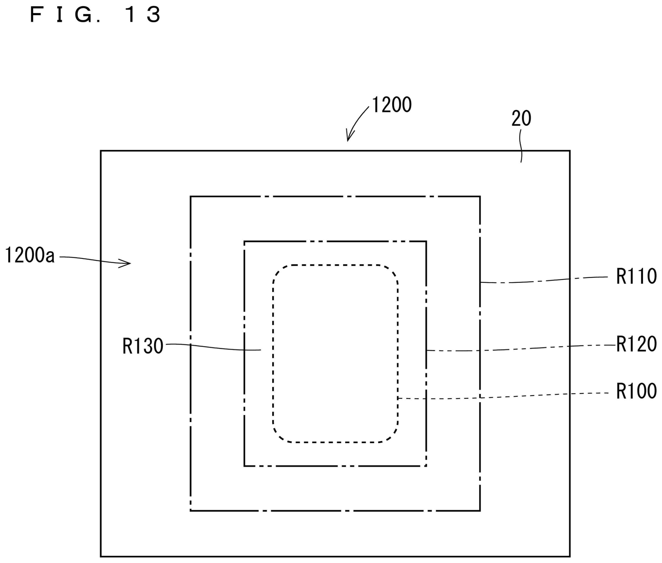

A relationship among the excitation light irradiation range R120, the detection range R110, and the IP image region R100 is not limited to that in the above-mentioned example. FIGs. 12 and 13 are schematic diagrams each illustrating another example of the excitation light irradiation range R120, the detection range R110, and the IP image region R100. In the example of FIG. 12, the excitation light irradiation range R120 is greater than the detection range R110 and the IP image region R100 while including the detection range R110 and the IP image region R100. In the example of FIG. 12, the IP image region outside region inside the detection range R110 falls within the excitation light irradiation range R120. A region inside the detection range R110 and the excitation light irradiation range R120 and outside the IP image region R100 may be referred to as an in-irradiation range IP image region outside region. The in-irradiation range IP image region outside region matches the above-mentioned IP outside region R130. In the example of FIG. 12, the IP image region outside region includes the in-irradiation range IP image region outside region. The IP outside region R130 is hereinafter also referred to as the in-irradiation range IP image region outside region R130.

In the example of FIG. 13, the detection range R110 is greater than the excitation light irradiation range R120 and the IP image region R100 while including the excitation light irradiation range R120 and the IP image region R100.

As in the examples of FIGs. 10, 12, and 13, the excitation light irradiation range R120 and the detection range R110 may match each other, or one of the excitation light irradiation range R120 and the detection range R110 may be greater than the other one of the excitation light irradiation range R120 and the detection range R110.

In the example of FIG. 13, the detection range R110 includes a region (also referred to as an out-of-irradiation range region) on the support side main surface 1200a other than the excitation light irradiation range R120. In the example of FIG. 13, the IP image region outside region inside the detection range R110 includes a region inside the excitation light irradiation range R120 excluding the IP image region R100 and a region inside the detection range R110 excluding the excitation light irradiation range R120. A region in the detection range R110 outside the excitation light irradiation range R120 may be referred to as an out-of-irradiation range IP image region outside region. In the example of FIG. 13, the IP image region outside region inside the detection range R110 includes the in-irradiation range IP image region outside region and the out-of-irradiation range IP image region outside region.

Processing of making the excitation light L10 less likely to be reflected may be performed in the excitation light irradiation range R120, and may be performed in the out-of-irradiation range IP image region outside region of the support side main surface 1200a. A condition of light in the out-of-irradiation range IP image region outside region may be referred to as an out-of-irradiation range IP image region outside region light condition.

In the reading processing, out-of-irradiation range detection processing is performed in the out-of-irradiation range IP image region outside region. In the out-of-irradiation range detection processing, the detector 40 may detect luminance of the out-of-irradiation range IP image region outside region light condition. It is already known that luminance of the out-of-irradiation range IP image region outside region light condition is low, so that luminance values of the out-of-irradiation range IP image region outside region light condition may collectively be set to a predetermined value to detect luminance of the out-of-irradiation range IP image region outside region light condition for efficiency, for example. The predetermined value is a luminance value lower than a luminance value for the IP image. The image signal output from the detector 40 also includes the luminance value of the out-of-irradiation range IP image region outside region light condition.

As described above, an image in the out-of-irradiation range IP image region outside region can be acquired in the reading processing even when light is not emitted from the out-of-irradiation range IP image region outside region. Processing of making the excitation light L10 less likely to be reflected may be performed in the IP image region outside region. In this case, processing of making the excitation light L10 less likely to be reflected may be performed at least in the out-of-irradiation range IP image region outside region.

An image signal acquired as a result of detection of the acted light L2 from the detection range R110 may be referred to as a detection range image signal. An image based on the detection range image signal may be referred to as a detection range image. The detection range image is the above-mentioned acquired whole image.

The image in the IP image region outside region may be referred to as the IP image region outside region image. An image in the in-irradiation range IP image region outside region may be referred to as an in-irradiation range IP image region outside region image. An image in the out-of-irradiation range IP image region outside region may be referred to as an out-of-irradiation range IP image region outside region image. In the case of FIG. 12, the in-irradiation range IP image region outside region image is the IP image region outside region image. In the case of FIG. 13, the in-irradiation range IP image region outside region image and the out-of-irradiation range IP image region outside region image constitute the IP image region outside region image.

When the reflected light L400 having low luminance is generated as a result of irradiation of the in-irradiation range IP image region outside region R130 (i.e., the IP outside region R130) with the excitation light L10, and the detector 40 detects the reflected light L400, the in-irradiation range IP image region outside region image (i.e., the IP outside region image) is a reflected light image. The out-of-irradiation range IP image region outside region image is a dark image from pixels of the detection surface of the sensor 41 through which dark current flows, or a dark image generated by being artificially provided with a low value. The IP image region outside region image is an image including the reflected light image in the in-irradiation range IP image region outside region or including the reflected light image in the in-irradiation range IP image region outside region and the dark image in the out-of-irradiation range IP image region outside region.

### <Erasing Light Source>

In this example, the erasing light source 70 and the erasing control unit 87 that controls the erasing light source 70 constitute an erasing unit 91 that performs erasing processing of erasing the radiograph form the imaging plate 10 as shown in FIG. 6. The erasing light source 70 and the erasing control unit 87 constituting the erasing unit 91 may be contained in a single case to be unitized, or may not be contained in the single case. The erasing light source 70 can irradiate the imaging plate 10 with erasing light L3 to erase the radiograph from the imaging plate 10. The erasing light L3 is visible light, for example. The erasing light L3 may be white light, red visible light, or visible light of another color. The erasing light source 70 may be a light emission diode (an LED), a halogen lamp, or another light source.

As illustrated in FIGs. 4 and 5, the erasing light source 70 can irradiate all the regions of the radiograph formation layer 11 of the imaging plate 10 with the erasing light L3 at one time, for example. In the erasing processing, the radiograph formation layer 11 is irradiated with the erasing light L3 to erase the radiograph from the radiograph formation layer 11.

When the reading processing ends, the driver 50 moves the holder 20 holding the imaging plate 10 to the erasing position. When the holder 20 is moved to the erasing position, the erasing unit 91 performs the erasing processing. In the erasing processing, the erasing light source 70 irradiates all the regions of the radiograph formation layer 11 of the imaging plate 10 with the erasing light L3 through control performed by the erasing control unit 87. The radiograph is thereby erased from the imaging plate 10.

When the radiograph is erased from the imaging plate 10, the driver 50 moves the holder 20 to a discharge position. When the holder 20 is moved to the discharge position, each of the fixing portions 22a of the holder 20 is moved from the close position to the spaced position. The imaging plate 10 from which the radiograph has been erased is then discharged to the outlet 2b. An erased imaging plate 10 hereinafter refers to the imaging plate 10 from which the radiograph has been erased.

### <Image Processing Unit>

The image processing unit 81 of the controller 80 associates luminance values included in the image signal from the sensor 41 with respective pixel positions of the acquired whole image.

The driver 50 herein moves the holder 20 in the subscannig direction DRs in response to repetition of the main scanning direction scanning in the reading processing. Specifically, the driver 50 moves the holder 20 in the subscannig direction DRs while the main scanning direction scanning is performed a predetermined number of times so that all regions of the excitation light irradiation range R120 are irradiated with the excitation light L10. The sensor 41 sequentially outputs luminance values responsive to the positions of irradiation with the excitation light L10 in the detection range R110 in response to raster scanning with the excitation light L10. The reading apparatus 1 operates as described above in the reading processing, so that a position of a pixel of the acquired whole image corresponding to a luminance value output from the sensor 41 at a certain time can be known from the excitation light irradiation range R120, the detection range R110, time taken to perform the main scanning direction scanning at one time, a cycle of repetition of the main scanning direction scanning, and the number of times the main scanning direction scanning is performed in the reading processing. The image processing unit 81 associates the luminance values included in the image signal from the sensor 41 with the respective pixel positions based on the excitation light irradiation range R120, the detection range R110, the time taken to perform the main scanning direction scanning at one time, the cycle of repetition of the main scanning direction scanning, and the number of times the main scanning direction scanning is performed in the reading processing. The detection control unit 85 may associate the luminance values included in the image signal from the sensor 41 with the respective pixel positions.

The image processing unit 81 performs image processing on the image signal from the sensor 41. The image processing unit 81 performs the image processing on the image signal (a detected image signal) from the sensor 41 as the first detector and the second detector. The image processing unit 81 outputs, to the display control unit 82, image information in which luminance values of the image signal after the image processing are associated with respective pixel positions of the acquired whole image, for example. The display control unit 82 causes the display 3 to display the acquired whole image including the detected radiograph based on the image information, for example.

The image processing performed by the image processing unit 81 may include luminance reversal processing. The luminance reversal processing refers to processing of converting the luminance values of the image signal before the luminance reversal processing so that a greater luminance value is converted into a smaller luminance value. A maximum value of a range that the luminance value can take is herein referred to as a maximum luminance value. For example, a value obtained by subtracting a certain luminance value included in the image signal before the luminance reversal processing from the maximum luminance value is used as the certain luminance value after conversion. The luminance reversal processing is performed on the image signal, so that the radiograph based on detection of the excited region light L20 from a portion of the imaging plate 10 in which less energy is stored has a greater luminance value, and the radiograph based on detection of the excited region light L20 from a portion of the imaging plate 10 in which more energy is stored has a smaller luminance value in the image signal after the image processing in contrast to the image signal before the image processing. The luminance value for the unexposed region image and the luminance value for the IP outside region image are greater than the luminance value for the detected radiograph in the image signal after the image processing.

The image processing performed by the image processing unit 81 may not include the luminance reversal processing, or may include processing other than the luminance reversal processing. The image processing may include offset correction and logarithmic transformation as the processing other than the luminance reversal processing, for example.

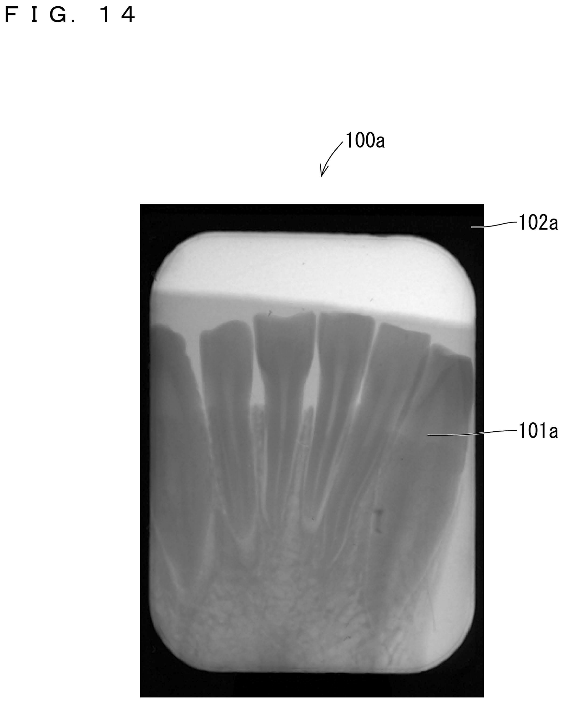

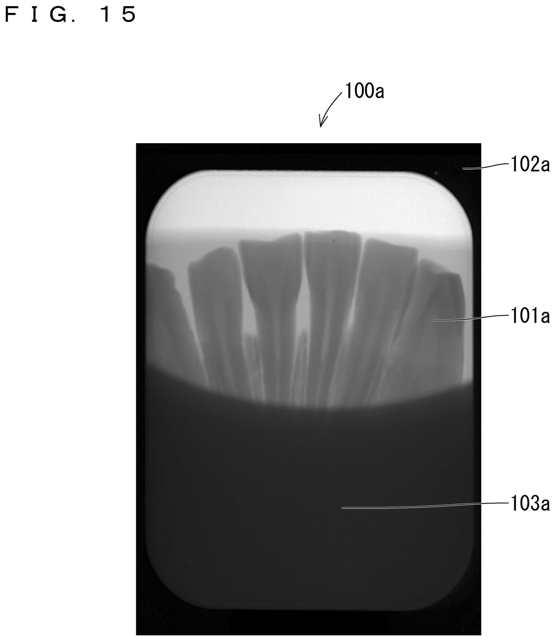

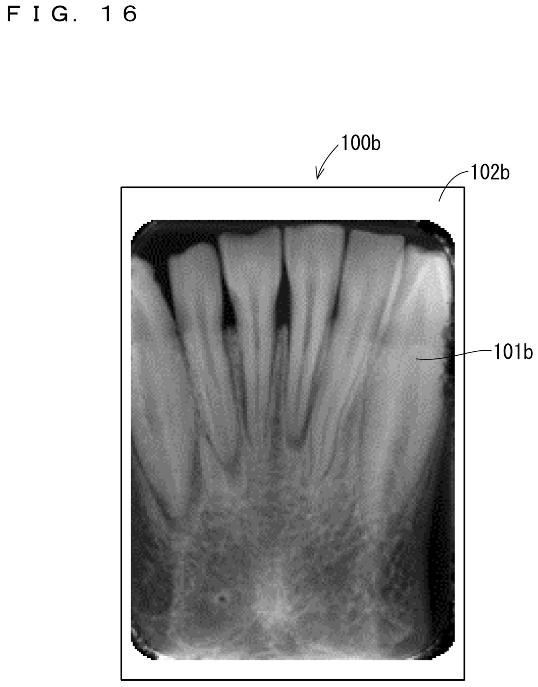

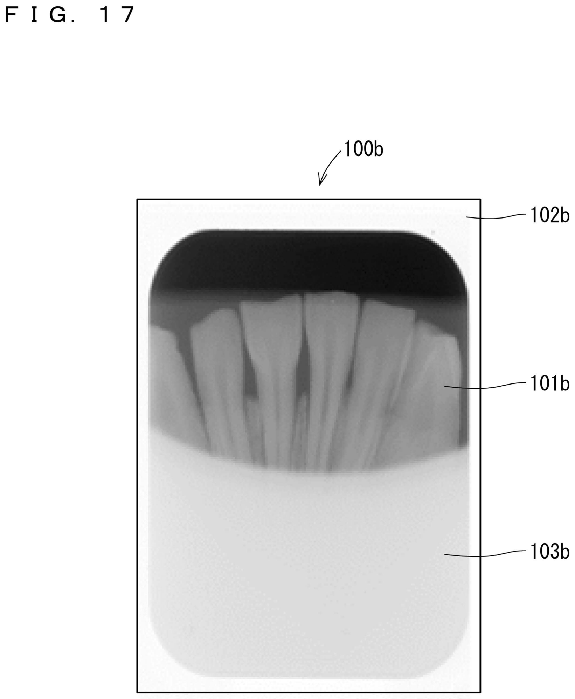

FIGs. 14 and 15 are schematic diagrams each showing one example of the acquired whole image (also referred to as a before-reversal whole image) 100a based on the image signal before the luminance reversal processing. FIGs. 16 and 17 are schematic diagrams each showing one example of the acquired whole image (also referred to as an after-reversal whole image) 100b based on the image signal after the luminance reversal processing. The before-reversal whole images 100a and the after-reversal whole images 100b are shown in grayscale in FIGs. 14 to 17. FIGs. 14 to 17 show a brighter (i.e., white) image as the image has a greater luminance value and a darker (i.e., black) image as the image has a smaller luminance value. The same applies to drawings described below each showing an image acquired by the reading apparatus 1.

An image acquired by scanning the imaging plate 10 used as the light receiver in biological radiography with the acting light L1 to read a radiograph of a biological tissue as with the acquired whole image 100a in FIG. 14 is referred to as a biological radiographically captured image. Scanning with the excitation light L10 as the acting light L1 may be performed. An image formed by the IP acted light L2 (i.e., an image based on detection of the IP acted light L2) in the biological radiographically captured image as with a radiograph 101a in FIG. 14 is referred to as an IP biological radiographically captured image. The biological radiographically captured image and the IP biological radiographically captured image are each an image acquired by biological radiography, and is an acquired image acquired by biological radiography.

FIG. 14 shows the before-reversal whole image 100a including the radiograph (i.e., the detected radiograph or the IP excited region light image) 101a based on detection of the excited region light L20 and an IP image region outside region image 102a. In the example of FIG. 14, the radiograph 101a is the IP image. FIG. 15 shows the before-reversal whole image 100a including the radiograph (i.e., the IP excited region light image) 101a, the IP image region outside region image 102a, and an unexposed region image (i.e., the IP reflected light image or the IP non-photostimulable reflected light image) 103a. In the example of FIG. 15, the radiograph 101a and the unexposed region image 103a constitute the IP image. FIG. 16 shows the after-reversal whole image 100b including a radiograph (i.e., the IP excited region light image) 101b and an IP image region outside region image 102b. In the example of FIG. 16, the radiograph 101b is the IP image. FIG. 17 shows the after-reversal whole image 100b including the radiograph (i.e., the IP excited region light image) 101b, the IP image region outside region image 102b, and an unexposed region image (i.e., the IP reflected light image or the IP non-photostimulable reflected light image) 103b. In the example of FIG. 17, the radiograph 101a and the unexposed region image 103b constitute the IP image. In this example, the detection range R110 of the sensor 41 is rectangular, so that the acquired whole image is rectangular as shown in FIGs. 14 to 17.

When the display control unit 82 causes the display 3 to display the after-reversal whole image 100b based on the image signal after the image processing, the display control unit 82 causes the display 3 to display the after-reversal whole image 100b in grayscale as shown in FIGs. 16 and 17, for example. It can be said that FIGs. 16 and 17 are drawings each showing an example of display of the after-reversal whole image 100b. When the display control unit 82 causes the display 3 to display the before-reversal whole image 100a based on the image signal before the luminance reversal processing, the display control unit 82 causes the display 3 to display the before-reversal whole image 100a in grayscale as shown in FIGs. 14 and 15, for example. It can be said that FIGs. 14 and 15 are drawings each showing an example of display of the before-reversal whole image 100a.

### <Example of Operation of Reading Apparatus>

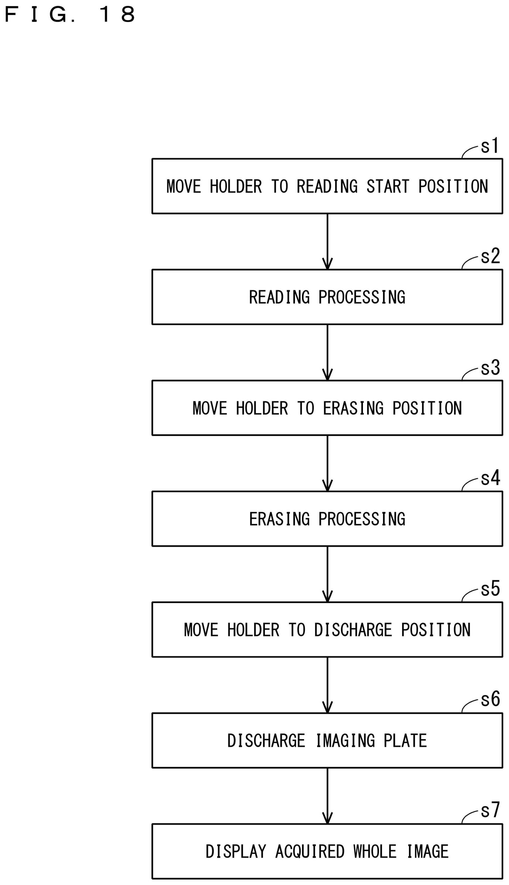

FIG. 18 is a flowchart showing one example of operation of the reading apparatus 1. When the imaging plate 10 inserted through the inlet 2a of the housing 2 is held by the holder 20, and the start button included in the operation unit 4 is operated, step s1 in FIG. 18 is performed. It can be said that the operation on the start button is an operation to provide instructions to start a series of processes shown in FIG. 18.

In step s1, the driver 50 moves the holder 20 to the reading start position through control performed by the drive control unit 83. Next, in step s2, the reading processing of reading the radiograph from the imaging plate 10 is performed. Next, in step s3, the driver 50 moves the holder 20 to the erasing position through control performed by the drive control unit 83. Next, in step s4, the erasing light source 70 irradiates the imaging plate 10 with the erasing light L3 to perform the erasing processing of erasing the radiograph from the imaging plate 10 through control performed by the erasing control unit 87. Next, in step s5, the driver 50 moves the holder 20 to the discharge position through control performed by the drive control unit 83. Next, in step s6, the imaging plate 10 is discharged to the outlet 2b of the housing 2. In step s7, the display 3 displays the acquired whole image through control performed by the display control unit 82. In step s7, the display 3 displays the after-reversal whole image in grayscale as shown in FIGs. 16 and 17, for example. Step s7 may be performed at any time after step s2. For example, step s7 may be performed between step s3 and step s4.

### <Identification of Tilt Angle of Imaging Plate>

The image processing unit 81 may perform tilt angle identification processing of identifying a tilt angle (also referred to as an IP tilt angle) of the imaging plate 10 relative to the reference orientation, for example. The wording of "tilt" can be replaced with wording of "deviate". For example, "tilting" can be replaced with "deviated", and "tilt" can be replaced with "deviation" or "deviated". In the tilt angle identification processing, the image processing unit 81 identifies the IP tilt angle based on the image signal output from the detector 40, specifically, the IP acted light image signal, for example. The image processing unit 81 functions as an identification unit (also referred to as a tilt angle identification unit) that identifies the IP tilt angle. In this example, a rotation angle relative to the reference orientation can be detected by performing principal component analysis in which a region where the imaging plate 10 is present is considered as a two-dimensional feature vector set. While a specific example of the tilt angle identification processing will be described below, the tilt angle identification processing is not limited to that in the example described below.

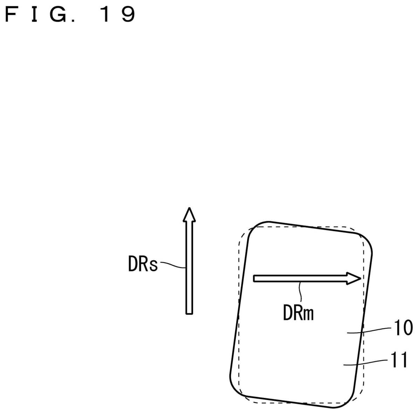

As described above, the reference orientation in this example is an orientation of the imaging plate 10 in which the transverse direction and the longitudinal direction of the imaging plate 10 are respectively parallel to the main scanning direction DRm and the subscannig direction DRs. In other words, the reference orientation is an orientation of the imaging plate 10 in which the transverse direction of the imaging plate 10 is parallel to the main scanning direction DRm and the longitudinal direction of the imaging plate 10 is parallel to the subscannig direction DRs, for example. When the holder 20 does not properly hold the imaging plate 10, the imaging plate 10 can tilt relative to the reference orientation with the longitudinal direction (also referred to as an IP longitudinal direction) of the imaging plate 10 tilting relative to the subscannig direction DRs as illustrated in FIG. 19 when the imaging plate 10 held by the holder 20 is viewed from a side of the front surface thereof. In other words, the imaging plate 10 can tilt relative to the reference orientation with the transverse direction (also referred to as an IP transverse direction) of the imaging plate 10 tilting relative to the main scanning direction DRm. The imaging plate 10 in the reference orientation is indicated by a dashed line in FIG. 19.

In this example, the IP tilt angle, that is, the tilt angle of the imaging plate 10 relative to the reference orientation matches the tilt angle of the IP longitudinal direction relative to the subscannig direction DRs. In other words, the IP tilt angle matches the tilt angle of the IP transverse direction relative to the main scanning direction DRm.

On the other hand, a longitudinal direction of the detection range R110 (see FIG. 10 and the like) of the sensor 41 corresponds to a longitudinal direction of the acquired whole image based on the image signal from the sensor 41. In this example, the longitudinal direction of the detection range R110 is parallel to the subscannig direction DRs, so that it can be said that the longitudinal direction of the acquired whole image corresponds to the subscannig direction DRs. Thus, when the orientation of the imaging plate 10 is the reference orientation, the longitudinal direction of the acquired whole image and a longitudinal direction of a portion (also referred to as an IP corresponding portion) of the acquired whole image corresponding to the imaging plate 10 match each other. On the other hand, when the imaging plate 10 tilts relative to the reference orientation, the longitudinal direction of the IP corresponding portion of the acquired whole image tilts relative to the longitudinal direction of the acquired whole image. In other words, when the imaging plate 10 tilts relative to the reference orientation, a transverse direction of the IP corresponding portion of the acquired whole image tilts relative to a transverse direction of the acquired whole image.

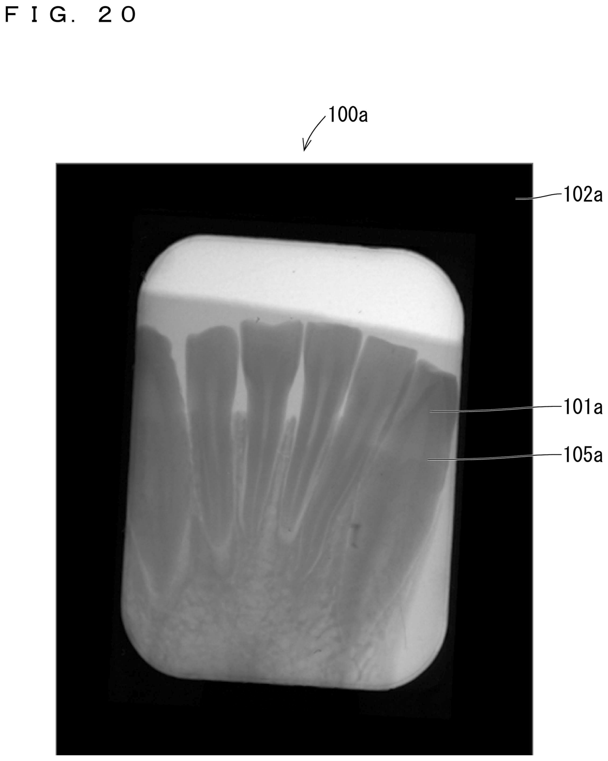

FIG. 20 shows one example of the before-reversal whole image 100a acquired when the imaging plate 10 tilts relative to the reference orientation. The before-reversal whole image 100a shown in FIG. 20 includes a tilting IP corresponding portion 105a.

In this example, the tilt angle of the longitudinal direction of the IP corresponding portion (i.e., the IP image) relative to the longitudinal direction of the acquired whole image matches the IP tilt angle. The image processing unit 81 thus determines the tilt angle of the longitudinal direction of the IP corresponding portion relative to the longitudinal direction of the acquired whole image based on the image signal from the sensor 41. The image processing unit 81 sets the determined tilt angle to the IP tilt angle. Operation of the image processing unit 81 in this case will be described in detail below.

In the tilt angle identification processing, the image processing unit 81 binarizes the before-reversal whole image based on the image signal (also referred to as a before-reversal image signal) before the luminance reversal processing to generate a binarized image. The image processing unit 81 first compares each of luminance values for the before-reversal whole image included in the before-reversal image signal with a preset threshold. The image processing unit 81 replaces a luminance value for the before-reversal whole image equal to or greater than the threshold with "1", and replaces a luminance value for the before-reversal whole image smaller than the threshold with "0". The before-reversal whole image is thereby binarized to acquire the binarized image.

The threshold used for binarization is set to a value greater than a luminance value for the IP image region outside region image included in the before-reversal image signal and smaller than a luminance value for the unexposed region image included in the before-reversal image signal, for example. Consider a case where IL1 is the luminance value for the IP image region outside region image included in the before-reversal image signal, and IL2 is the luminance value for the unexposed region image included in the before-reversal image signal, for example. An inequality IL1 < IL2 holds. In this case, the threshold is set to IL3 that satisfies a relationship indicated by an inequality IL1 < IL3 < IL2, for example. The threshold is set based on the before-reversal image signal acquired by the reading apparatus 1 before actual operation, and is stored in advance in the image processing unit 81 of the reading apparatus 1, for example. The luminance value for the detected radiograph included in the before-reversal image signal is greater than the luminance value for the unexposed region image included in the before-reversal image signal (see FIG. 15), so that the threshold is smaller than the luminance value for the detected radiograph included in the before-reversal image signal.

The threshold is set as described above, so that each of luminance values for a portion of the binarized image corresponding to the IP image region outside region is "0". Each of luminance values for a portion of the binarized image corresponding to the imaging plate 10 is "1" regardless of whether the unexposed portion is included in the imaging plate 10.

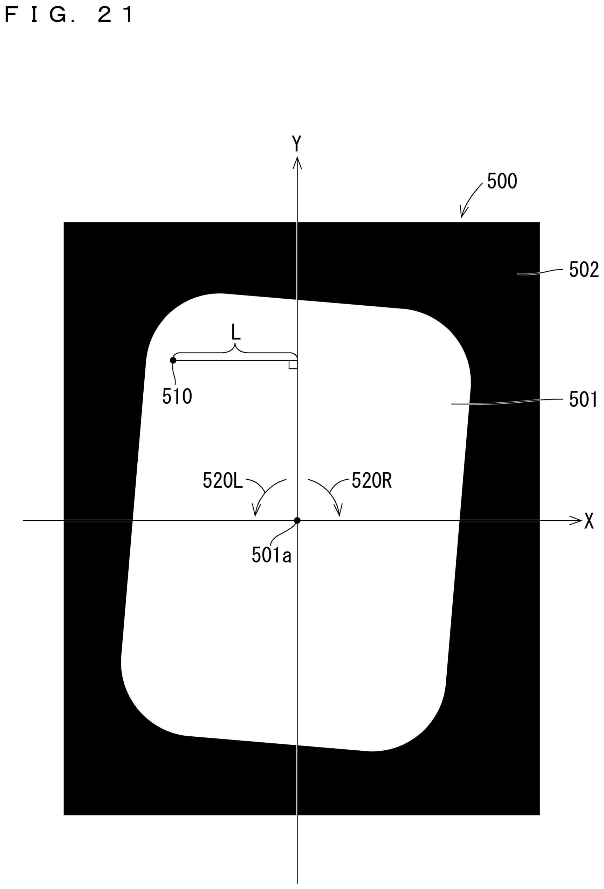

FIG. 21 is a schematic diagram showing one example of a binarized image 500. FIG. 21 shows the binarized image 500 acquired by binarizing the before-reversal whole image 100a shown in FIG. 20. In FIG. 21, a region (also referred to as a high luminance region) 501 of the binarized image 500 where the luminance value is "1" is shown in white, and a region (also referred to as a low luminance region) 502 of the binarized image 500 where the luminance value is "0" is shown in black. As can be understood from FIG. 21, the high luminance region 501 corresponds to the imaging plate 10, and the low luminance region 502 corresponds to the IP image region outside region in the binarized image 500. An outline of the high luminance region 501 has a shape responsive to an outline of the imaging plate 10. An image (also referred to as a high luminance region image) in the high luminance region 501 is the IP image, and an image (also referred to as a low luminance region image) in the low luminance region 502 is the IP image region outside region image.

The shape of the imaging plate 10 is referred to as an imaging plate shape, and data on the imaging plate shape (i.e., data representing the imaging plate shape) is referred to as imaging plate shape data. The imaging plate shape data can include at least one of the size, the shape, and a tilt of the imaging plate 10 and the like. Processing of extracting the imaging plate shape may be referred to as imaging plate shape extraction processing. The imaging plate shape data can be acquired in the imaging plate shape extraction processing. An image, such as the before-reversal whole image 100a in FIG. 20, acquired by radiography of the imaging plate 10 is referred to as an imaging plate captured image.

An image, such as the binarized image 500 in FIG. 21, representing the imaging plate shape acquired by performing processing on an image acquired as a result of detection of the acted light generated by irradiation of the imaging plate 10 with light is referred to as an imaging plate shape image. An image, such as the image in the high luminance region 501 in FIG. 21, formed by extracting the imaging plate shape by performing processing on the IP acted light is referred to as an IP imaging plate shape image. The imaging plate shape image and the IP imaging plate shape image are each an image representing the imaging plate shape extracted by performing processing on the IP acted light image signal, and thus may each be referred to as an imaging plate shape extraction image.

The high luminance region 501 included in the binarized image 500 herein corresponds to the IP corresponding portion 105a included in the before-reversal whole image 100a. In the tilt angle identification processing, the image processing unit 81 performs the principal component analysis in which positions of a plurality of pixels constituting the high luminance region 501 included in the generated binarized image 500 are data to be analyzed to acquire a first principal component axis of the data to be analyzed.

In the principal component analysis, the image processing unit 81 determines a center of gravity 501a (see FIG. 21) of the high luminance region 501 included in the binarized image 500. The image processing unit 81 sets an XY coordinate system with the center of gravity 501a as the origin to the binarized image 500 as shown in FIG. 21. Assume that an X axis is parallel to a transverse direction of the before-reversal whole image 100a, and a Y axis is parallel to a longitudinal direction of the before-reversal whole image 100a in this case. As will be described below, the image processing unit 81 rotates the XY coordinate system about the center of gravity 501a. Assume that an angle of clockwise rotation of the XY coordinate system is a positive angle, and an angle of counterclockwise rotation of the XY coordinate system is a negative angle in this example. An orientation of the XY coordinate system in which the X axis is parallel to the transverse direction of the before-reversal whole image 100a and the Y axis is parallel to the longitudinal direction of the before-reversal whole image 100a is referred to as an initial orientation.

When setting the XY coordinate system in the initial orientation to the binarized image 500, the image processing unit 81 determines a length L of a perpendicular from a position 510 of each of the plurality of pixels constituting the high luminance region 501 to the Y axis. Next, the image processing unit 81 determines a variance of a plurality of lengths L determined for respective pixels constituting the high luminance region 501. The variance is referred to as a variance in the initial orientation.

The image processing unit 81 performs clockwise processing of rotating the XY coordinate system about the center of gravity 501a clockwise 520R from the initial orientation by 0.1 degrees at a time, and determining the variance of the plurality of lengths L each time the XY coordinate system is rotated by 0.1 degrees. In the clockwise processing, the image processing unit 81 eventually rotates the XY coordinate system clockwise 520R by 90 degrees, for example. The image processing unit 81 also performs counterclockwise processing of rotating the XY coordinate system about the center of gravity 501a counterclockwise 520L from the initial orientation by 0.1 degrees at a time, and determining the variance of the plurality of lengths L each time the XY coordinate system is rotated by 0.1 degrees. In the counterclockwise processing, the image processing unit 81 eventually rotates the XY coordinate system counterclockwise 520L by 90 degrees, for example.

When performing the clockwise processing and the counterclockwise processing, the image processing unit 81 identifies a minimum value of the variances determined by the clockwise processing and the counterclockwise processing and the variance in the initial orientation. The image processing unit 81 sets the Y axis of the XY coordinate system when the identified minimum value is acquired to the first principal component axis. It can be said that the first principal component axis is an axis to minimize the variance of the lengths of the perpendiculars from respective positions of the plurality of pixels constituting the high luminance region 501. An axis being perpendicular to the first principal component axis and passing through the center of gravity 501a is hereinafter also referred to as a second principal component axis. The XY coordinate system when the minimum value of the variances determined by the clockwise processing and the counterclockwise processing and the variance in the initial orientation is determined is also referred to as an XY coordinate system in a minimum variance orientation.

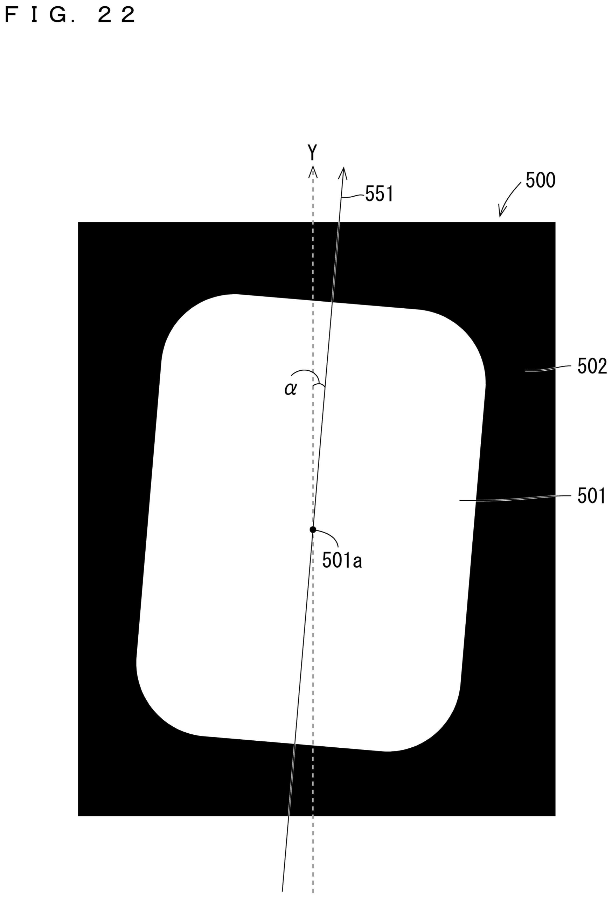

FIG. 22 is a schematic diagram showing a first principal component axis 551 acquired from the binarized image 500 shown in FIG. 21. The Y axis of the XY coordinate system in the initial orientation is indicated by a dashed line in FIG. 22. As shown in FIG. 22, the first principal component axis 551 matches a longitudinal direction of the high luminance region 501. When determining the first principal component axis 551, the image processing unit 81 determines the rotation angle α of the XY coordinate system having the Y axis matching the first principal component axis 551, that is, the XY coordinate system in the minimum variance orientation from the initial orientation. The image processing unit 81 sets the determined rotation angle α to the tilt angle α of the longitudinal direction of the IP corresponding portion relative to the longitudinal direction of the acquired whole image. The Y axis of the XY coordinate system in the initial orientation corresponds to the longitudinal direction of the acquired whole image, and the first principal component axis 551 (i.e., the Y axis of the XY coordinate system in the minimum variance orientation) corresponds to the longitudinal direction of the IP corresponding portion included in the acquired whole image. It can thus be said that the rotation angle α is the tilt angle of the longitudinal direction of the IP corresponding portion relative to the longitudinal direction of the acquired whole image. The rotation angle α has a positive value when the XY coordinate system in the minimum variance orientation is acquired by the clockwise processing, and has a negative value when the XY coordinate system in the minimum variance orientation is acquired by the counterclockwise processing. The rotation angle α is zero when the XY coordinate system in the minimum variance orientation matches the XY coordinate system in the initial orientation.

The image processing unit 81 determines the tilt angle α of the longitudinal direction of the IP corresponding portion relative to the longitudinal direction of the acquired whole image from the binarized image 500 of the acquired whole image as described above, and sets the determined tilt angle α to the IP tilt angle α. When the imaging plate 10 is viewed from a side of the front surface thereof in plan view, the imaging plate 10 tilts clockwise relative to the reference orientation when the IP tilt angle α is a positive angle, and tilts counterclockwise relative to the reference orientation when the IP tilt angle α is a negative angle. As for the tilt of the imaging plate 10, a clockwise tilt hereinafter simply refers to a clockwise tilt when the imaging plate 10 is viewed from a side of the front surface thereof, and a counterclockwise tilt hereinafter simply refers to a counterclockwise tilt when the imaging plate 10 is viewed from a side of the front surface thereof.

The tilt angle identification processing as described above may be performed during the above-mentioned series of processes shown in FIG. 18, or may be performed at a different time from the processing shown in FIG. 18. The IP tilt angle determined by the image processing unit 81 may be displayed by the display 3. In this case, the display 3 may display the IP tilt angle simultaneously with the acquired whole image in the above-mentioned step s7, or may display the IP tilt angle when the acquired whole image is not displayed.

Instead of calculating variances as described above each time, a covariance σXY may be determined in addition to variances σX2 and σY2 in the initial orientation, and may be used to calculate a variance after rotation.

As described above, in this example, the image processing unit 81 identifies the IP tilt angle based on the image signal as a result of detection of the emitted light L2 and the reflected light from the imaging plate 10, and thus can properly identify the IP tilt angle.

Consider a case where the sensor 41 cannot detect the reflected light, and the imaging plate 10 includes the unexposed portion, for example. In this case, luminance values for the unexposed region image and the IP image region outside region image included in the before-reversal whole image based on the image signal from the sensor 41 are each zero. Thus, in the binarized image 500 acquired by binarizing the before-reversal whole image, luminance values for portions corresponding to the unexposed region image and the IP image region outside region image are each "0", and the portions corresponding to the unexposed region image and the IP image region outside region image are each the low luminance region 502. When the imaging plate 10 includes the unexposed portion, the high luminance region 501 of the binarized image 500 does not include the portion corresponding to the unexposed portion, and the high luminance region 501 does not correspond to the IP corresponding portion included in the before-reversal whole image. That is to say, when the imaging plate 10 includes the unexposed portion, the outline of the high luminance region 501 does not have the shape responsive to the outline of the imaging plate 10. Thus, when performing the principal component analysis on the high luminance region 501, the image processing unit 81 sometimes cannot determine the first principal component axis corresponding to the longitudinal direction of the imaging plate 10, and cannot properly determine the tilt angle of the longitudinal direction of the IP corresponding portion relative to the longitudinal direction of the acquired whole image.

In contrast, in this example, the sensor 41 can detect the reflected light to some extent, and the image processing unit 81 binarizes the before-reversal whole image based on the image signal as a result of detection of the photostimulated light L5 and the reflected light from the imaging plate 10 to generate the binarized image 500. Thus, in the binarized image 500, the high luminance region 501 corresponds to the IP corresponding portion (i.e., the IP image) included in the before-reversal whole image as shown in FIGs. 21 and 22 even when the imaging plate 10 includes the unexposed portion. That is to say, the outline of the high luminance region 501 has the shape responsive to the outline of the imaging plate 10. For example, the binarized image in which the unexposed region is included in the IP image region as shown in FIGs. 21 and 22 can be acquired by binarization processing even from the before-reversal whole image 100a including the unexposed region image 103a as shown in FIG. 15. Thus, when performing the principal component analysis on the high luminance region 501, the image processing unit 81 can determine the first principal component axis corresponding to the longitudinal direction of the imaging plate 10, and can properly determine the tilt angle of the longitudinal direction of the IP corresponding portion relative to the longitudinal direction of the acquired whole image. The image processing unit 81 can thus properly identify the IP tilt angle. An image, such as the image in the high luminance region 501, acquired by performing the binarization processing on the IP image may be referred to as an IP binarized image.

### <Identification of Size of Imaging Plate>

The image processing unit 81 may perform size identification processing of identifying the size (also referred to as an IP size) of the imaging plate 10, for example. In the size identification processing, the image processing unit 81 identifies the IP size based on the image signal output from the detector 40, for example. The image processing unit 81 functions as an identification unit (also referred to as a size identification unit) that identifies the IP size. While a specific example of the size identification processing will be described below, the size identification processing is not limited to that in the example described below.

The image processing unit 81 binarizes the before-reversal whole image based on the before-reversal image signal to generate the binarized image 500 similarly to the foregoing, for example. The image processing unit 81 identifies the IP size based on the generated binarized image 500. For example, the image processing unit 81 numerically identifies a size in the longitudinal direction (also referred to as a longitudinal size) of the imaging plate 10 and a size in the transverse direction (also referred to as a transverse size) of the imaging plate 10.

When identifying the IP size, the image processing unit 81 performs the principal component analysis in which the positions of the plurality of pixels constituting the high luminance region 501 included in the binarized image 500 are the data to be analyzed to acquire the first principal component axis 551 of the data to be analyzed similarly to the foregoing. The image processing unit 81 acquires a second principal component axis 552 being perpendicular to the first principal component axis 551 and passing through the center of gravity 501a.

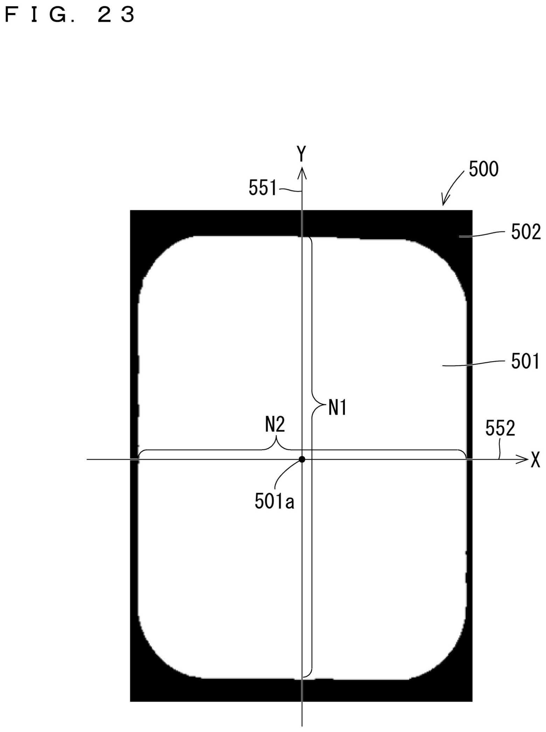

FIG. 23 is a schematic diagram showing one example of the first principal component axis 551 and the second principal component axis 552. FIG. 23 shows the binarized image 500 acquired by binarizing the before-reversal whole image 100a shown in FIG. 14 described above and the first principal component axis 551 and the second principal component axis 552 acquired based on the binarized image 500. The first principal component axis 551 is parallel to the longitudinal direction of the high luminance region 501 corresponding to the imaging plate 10. The second principal component axis 552 is parallel to the transverse direction of the high luminance region 501.

As shown in FIG. 23, the image processing unit 81 acquires the number of pixels (also referred to as the number of pixels in the longitudinal direction) N1 along the first principal component axis 551 of the high luminance region 501. The image processing unit 81 also acquires the number of pixels (also referred to as the number of pixels in the transverse direction) N2 along the second principal component axis 552 of the high luminance region 501. The image processing unit 81 acquires the longitudinal size of the imaging plate 10 based on the number of pixels N1 in the longitudinal direction, and acquires the transverse size of the imaging plate 10 based on the number of pixels N2 in the transverse direction.

A square region M mm on a side in the detection range R110 of the sensor 41 herein corresponds to a single pixel of the acquired whole image and the binarized image 500 in this example. M mm is approximately 0.03 mm, for example. The image processing unit 81 sets a length obtained by multiplying the number of pixels N1 in the longitudinal direction by M mm to the longitudinal size of the imaging plate 10. The image processing unit 81 also sets a length obtained by multiplying the number of pixels N2 in the transverse direction by M mm to the transverse size of the imaging plate 10. The longitudinal size and the transverse size of the imaging plate 10 determined by the image processing unit 81 are hereinafter also referred to as an identified longitudinal size and an identified transverse size.

The image processing unit 81 may numerically identify the area of the main surface (also referred to as a main surface area) of the imaging plate 10 in the size identification processing. In this case, the image processing unit 81 may set a value obtained by multiplying the square of M mm by the total number of pixels constituting the high luminance region 501 to the main surface area of the imaging plate 10. It can be said that the main surface area is the area of the front surface of the imaging plate 10, and is the area of the back surface of the imaging plate 10. The main surface area of the imaging plate 10 determined by the image processing unit 81 is hereinafter also referred to as an identified main surface area or an identified area.

The image processing unit 81 may identify a type of the size of the imaging plate 10 in the size identification processing. A plurality of types of sizes are prepared as the size of the imaging plate 10 in this example. In the reading apparatus 1, the holder 20 can hold each of imaging plates 10 of the plurality of types of sizes. The reading apparatus 1 can read a radiograph from each of the imaging plates 10 of the plurality of types of sizes.

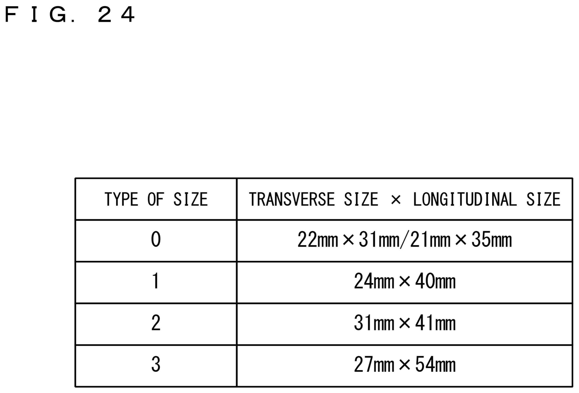

FIG. 24 shows one example of the types of sizes (also referred to as types of IP sizes) of the imaging plate 10. Four types of sizes, including Size 0, Size 1, Size 2, and Size 3, are prepared as the types of IP sizes, for example. The transverse size and the longitudinal size of the imaging plate 10 of each of the types of sizes are herein respectively referred to as a nominal transverse size and a nominal longitudinal size. Sizes commonly used by many manufacturing entities and/or sizes determined by public standards may be used as the nominal transverse size and the nominal longitudinal size.

For example, the nominal transverse size and the nominal longitudinal size for Size 0 are respectively 22 mm and 31 mm according to the International Organization for Standardization (ISO) standard. The nominal transverse size and the nominal longitudinal size for Size 0 are respectively 21 mm and 35 mm in some cases. The nominal transverse size and the nominal longitudinal size for Size 1 are respectively 24 mm and 40 mm. The nominal transverse size and the nominal longitudinal size for Size 2 are respectively 31 mm and 41 mm. The nominal transverse size and the nominal longitudinal size for Size 3 are respectively 27 mm and 54 mm. A value obtained by multiplying the nominal transverse size and the nominal longitudinal size is referred to as a nominal main surface area or a nominal area.

The image processing unit 81 identifies the type of the size of the imaging plate 10 based on the identified transverse size, the identified longitudinal size, and the identified main surface area of the imaging plate 10, for example. For example, when the identified transverse size is close to the nominal transverse size for Size 0, the identified longitudinal size is close to the nominal longitudinal size for Size 0, and the identified area is close to the nominal area for Size 0, the image processing unit 81 determines that the type of the IP size is Size 0.

The image processing unit 81 determines whether the identified transverse size is close to the nominal transverse size for Size 0 using a first threshold slightly smaller than the nominal transverse size for Size 0 and a second threshold slightly greater than the nominal transverse size for Size 0, for example. The image processing unit 81 determines that the identified transverse size is close to the nominal transverse size for Size 0 when the identified transverse size is greater than the first threshold and is smaller than the second threshold, for example.

The image processing unit 81 determines whether the identified longitudinal size is close to the nominal longitudinal size for Size 0 using a third threshold slightly smaller than the nominal longitudinal size for Size 0 and a fourth threshold slightly greater than the nominal longitudinal size for Size 0, for example. The image processing unit 81 determines that the identified longitudinal size is close to the nominal longitudinal size for Size 0 when the identified longitudinal size is greater than the third threshold and is smaller than the fourth threshold, for example.

The image processing unit 81 determines whether the identified area is close to the nominal area for Size 0 using a fifth threshold slightly smaller than the nominal area for Size 0 and a sixth threshold slightly greater than the nominal area for Size 0, for example. The image processing unit 81 determines that the identified area is close to the nominal area for Size 0 when the identified area is greater than the fifth threshold and is smaller than the sixth threshold, for example.

For example, when the identified transverse size is close to the nominal transverse size for Size 1, the identified longitudinal size is close to the nominal longitudinal size for Size 1, and the identified main surface area is close to the nominal main surface area for Size 1, the image processing unit 81 similarly determines that the type of the IP size is Size 1.

For example, when the identified transverse size is close to the nominal transverse size for Size 2, the identified longitudinal size is close to the nominal longitudinal size for Size 2, and the identified main surface area is close to the nominal main surface area for Size 2, the image processing unit 81 similarly determines that the type of the IP size is Size 2.

For example, when the identified transverse size is close to the nominal transverse size for Size 3, the identified longitudinal size is close to the nominal longitudinal size for Size 3, and the identified main surface area is close to the nominal main surface area for Size 3, the image processing unit 81 similarly determines that the type of the IP size is Size 3.

The size identification processing as described above may be performed during the above-mentioned series of processes shown in FIG. 18, or may be performed at a different time from the processing shown in FIG. 18. The display 3 may display at least one of the transverse size, the longitudinal size, the main surface area, and the type of the IP size identified by the image processing unit 81, for example. In this case, the display 3 may display at least one of the transverse size, the longitudinal size, the main surface area, and the type of the IP size simultaneously with the acquired whole image in the above-mentioned step s7, or may display at least one of the transverse size, the longitudinal size, the main surface area, and the type of the IP size when the acquired whole image is not displayed, for example. The display 3 may display at least one of the transverse size, the longitudinal size, the main surface area, and the type of the IP size simultaneously with the IP tilt angle, or may display at least one of the transverse size, the longitudinal size, the main surface area, and the type of the IP size when the IP tilt angle is not displayed. The display 3 may simultaneously display at least two of the transverse size, the longitudinal size, the main surface area, and the type of the IP size.

As described above, in this example, the image processing unit 81 identifies the IP size based on the image signal as a result of detection of the emitted light L2 and the reflected light from the imaging plate 10, and thus can properly identify the IP size as in a case where the IP tilt angle is identified. As descried above, even when the imaging plate 10 includes the unexposed portion, the high luminance region 501 of the binarized image 500 corresponds to the IP corresponding portion of the before-reversal whole image, so that the image processing unit 81 can properly identify the transverse size, the longitudinal size, the main surface area, and the type of the IP size of the imaging plate 10.

An example of setting of the excitation light irradiation range R120 will be described in connection with description of the nominal transverse size and the nominal longitudinal size.

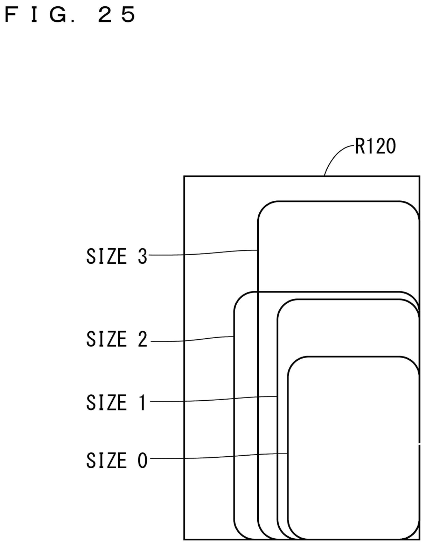

FIG. 25 schematically shows imaging plates 10 of Sizes 0 to 3. Positions of four sides of each of the imaging plates 10 in plan view of the imaging plate 10 are herein represented by upper, lower, left, and right positions. The holder 20 is commonly used for the imaging plates 10 of the plurality of sizes, for example. The holder 20 holds each of the imaging plates 10 so that left sides or right sides of the imaging plates 10 of the plurality of sizes are located in the same line, and upper sides or lower sides of the imaging plates 10 of the plurality of sizes are located in the same line as shown in FIG. 25, for example.

The longitudinal size of the imaging plate 10 for Size 3 is greater than the longitudinal size of the imaging plate 10 for Size 2, but the transverse size of the imaging plate 10 for Size 3 is smaller than the transverse size of the imaging plate 10 for Size 2. The excitation light irradiation range R120 is set to have a longitudinal size equal to or greater than a maximum longitudinal size of longitudinal sizes for the plurality of IP sizes and have a transverse size equal to or greater than a maximum transverse size of transverse sizes for the plurality of IP sizes to suit each of the imaging plates 10 of the plurality of sizes as described above, for example. The excitation light irradiation range R120 set as described above may be used for the imaging plate 10 of any of the plurality of IP sizes regardless of the IP size of the imaging plate 10.

The excitation light irradiation range R120 may be changed for each of the imaging plates 10. In this case, the excitation light irradiation range R120 may be set to at least have a longitudinal size equal to or greater than the maximum longitudinal size of the longitudinal sizes for the plurality of IP sizes and have a transverse size equal to or greater than the maximum transverse size of the transverse sizes for the plurality of IP sizes.

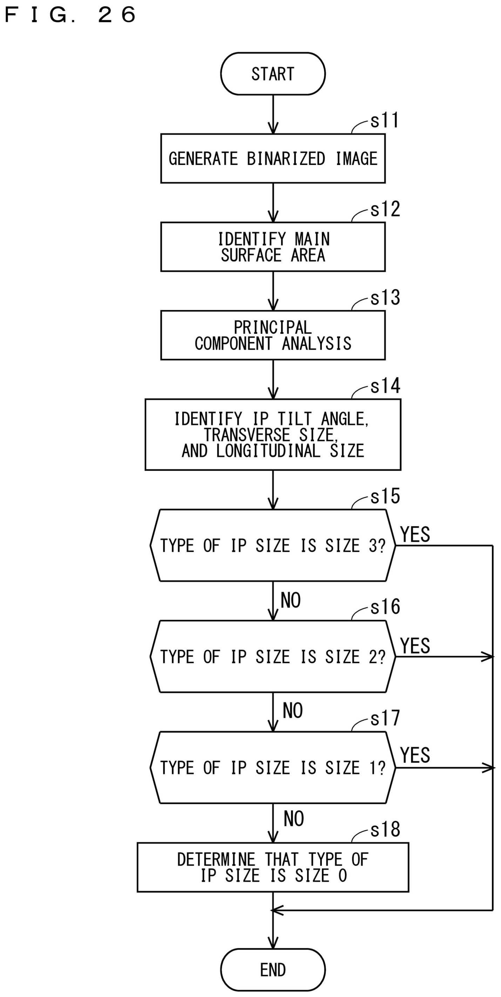

FIG. 26 is a flowchart showing one example of a series of operations of the image processing unit 81 when the image processing unit 81 identifies the IP tilt angle and the IP size. A series of processes shown in FIG. 26 may be performed during the above-mentioned series of processes shown in FIG. 18, or may be performed at a different time from the processing shown in FIG. 18.

As shown in FIG. 26, in step s11, the image processing unit 81 binarizes the before-reversal whole image based on the before-reversal image signal to generate the binarized image 500. Next, in step s12, the image processing unit 81 identifies the main surface area of the imaging plate 10 based on the binarized image 500 as described above. Next, in step s13, the image processing unit 81 performs the principal component analysis in which the positions of the plurality of pixels constituting the high luminance region 501 included in the binarized image 500 are the data to be analyzed to acquire the first principal component axis and the second principal component axis of the data to be analyzed.

Next, in step s14, the image processing unit 81 identifies the IP tilt angle and the transverse size and the longitudinal size of the imaging plate 10 based on the binarized image 500, the first principal component axis, and the second principal component axis as described above. Next, in step s15, the image processing unit 81 determines whether the type of the IP size is Size 3 based on the identified transverse size, the identified longitudinal size, and the identified area. In step s15, the image processing unit 81 determines that the type of the IP size is Size 3 when the identified transverse size is close to the nominal transverse size for Size 3, the identified longitudinal size is close to the nominal longitudinal size for Size 3, and the identified area is close to the nominal area for Size 3 as described above, for example.

When affirmative determination is made in step s15, processing shown in FIG. 26 ends. On the other hand, when negative determination is made in step s15, step s16 is performed. In step s16, the image processing unit 81 determines whether the type of the IP size is Size 2 based on the identified transverse size, the identified longitudinal size, and the identified area. In step s16, the image processing unit 81 determines that the type of the IP size is Size 2 when the identified transverse size is close to the nominal transverse size for Size 2, the identified longitudinal size is close to the nominal longitudinal size for Size 2, and the identified area is close to the nominal area for Size 2 as described above, for example.

When affirmative determination is made in step s16, processing shown in FIG. 26 ends. On the other hand, when negative determination is made in step s16, step s17 is performed. In step s17, the image processing unit 81 determines whether the type of the IP size is Size 1 based on the identified transverse size, the identified longitudinal size, and the identified area. In step s17, the image processing unit 81 determines that the type of the IP size is Size 1 when the identified transverse size is close to the nominal transverse size for Size 1, the identified longitudinal size is close to the nominal longitudinal size for Size 1, and the identified area is close to the nominal area for Size 1 as described above, for example.

When affirmative determination is made in step s17, processing shown in FIG. 26 ends. On the other hand, when negative determination is made in step s17, step s18 is performed. In step s18, the image processing unit 81 determines that the type of the IP size is Size 0. After step s18 is performed, processing shown in FIG. 26 ends. In step s18, the image processing unit 81 may determine whether the type of the IP size is Size 0 based on the identified transverse size, the identified longitudinal size, and the identified area as in steps s15 to s 17.

While the image processing unit 81 identifies the type of the IP size based on the identified transverse size, the identified longitudinal size, and the identified area in the above-mentioned example, the type of the IP size can be identified based on one of the identified transverse size, the identified longitudinal size, and the identified area. As shown in FIG. 24, the nominal transverse size and the nominal longitudinal size each differ among the plurality of types of sizes in this example. The nominal area also differs among the plurality of types of sizes. The image processing unit 81 may set one of Sizes 0 to 3 having the nominal transverse size closest to the identified transverse size to the type of the size of the imaging plate 10. The image processing unit 81 may set one of Sizes 0 to 3 having the nominal longitudinal size closest to the identified longitudinal size to the type of the size of the imaging plate 10. The image processing unit 81 may set one of Sizes 0 to 3 having the nominal area closest to the identified area to the type of the size of the imaging plate 10.

The image processing unit 81 may identify the type of the IP size based on two of the identified transverse size, the identified longitudinal size, and the identified area. For example, when the identified transverse size is close to the nominal transverse size for Size 1, and the identified longitudinal size is close to the nominal longitudinal size for Size 1, the image processing unit 81 may determine that the type of the IP size is Size 1.

In the size identification processing, the longitudinal size of the imaging plate 10 may not be identified, the transverse size of the imaging plate 10 may not be identified, and the main surface area of the imaging plate 10 may not be identified. The type of the size of the imaging plate 10 may not be identified in the size identification processing.

The reading apparatus 1 may have a configuration in which the user can identify the IP size through selection in addition to or in place of a configuration in which the image processing unit 81 identifies the IP size. The reading apparatus 1 may be configured to be switchable between the configuration in which the image processing unit 81 identifies the IP size and the configuration in which the user identifies the IP size through selection. In this case, whether to receive selection by the user is determined between steps s13 and s14, and, when affirmative determination is made, the selection by the user is received, and then the image processing unit 81 identifies the IP tilt angle without identifying the IP size, for example.

### <Irradiation of Erased Imaging Plate with Excitation Light>

In the reading apparatus 1, the light source 30 may irradiate the erased imaging plate 10 with the excitation light L10, and the detector 40 may detect the reflected light L40 from the erased imaging plate 10 to acquire a reflected light image in which the imaging plate 10 appears. In this case, the detector 40 also detects the reflected light L400 from the in-irradiation range IP image region outside region R130 in this example. In this example, the detector 40 detects the reflected light of the excitation light L10 from the erased imaging plate 10 and the in-irradiation range IP image region outside region R130, and outputs an image signal as a result of detection. An example of operation of the reading apparatus 1 that irradiates the erased imaging plate 10 with the excitation light L10 will be described below.

An image signal as a result of detection of the photostimulated light L5 or the photostimulated light L5 and the reflected light (excited region light L20) when the imaging plate 10 on which the radiograph is recorded is held as with the image signal having been described so far is hereinafter referred to as a light emission-time image signal. A whole image including the radiograph, that is, a whole image based on the light emission-time image signal as with the acquired whole image having been described so far is referred to as a light emission-time whole image. The light emission-time whole image is one example of the biological radiographically captured image. The before-reversal whole image and the after-reversal whole image described above are respectively referred to as a before-reversal light emission-time whole image and an after-reversal light emission-time whole image.

Portions of the light emission-time image signal, the light emission-time whole image, the before-reversal light emission-time whole image, and the after-reversal light emission-time whole image representing the image of the imaging plate 10, that is, the IP image may respectively be referred to as a light emission-time IP image signal, a light emission-time IP image, a before-reversal light emission-time IP image, and an after-reversal light emission-time IP image. The light emission-time IP image is one example of the IP biological radiographically captured image. When the imaging plate 10 includes the cone cut, the light emission-time IP image includes the IP excited region light image and the IP reflected light image (i.e., the IP non-photostimulable reflected light image). The light emission-time image signal may be defined as an image signal as a result of detection (acquisition of the biological radiographically captured image) when the imaging plate 10 held to read the radiograph is irradiated with the excitation light L10, that is, in a reading mode of reading the radiograph as in a case where step s2 is performed. In this case, when the cone cut extends across the whole region of the imaging plate 10, the light emission-time IP image (IP biological radiographically captured image) includes only the IP reflected light image (i.e., the IP non-photostimulable reflected light image).

An image signal as a result of detection of reflected light of light when the erased imaging plate 10 is held is referred to as an erasing-time image signal. A whole image based on the erasing-time image signal is referred to as an erasing-time whole image. In this example, the erasing-time whole image includes not only the reflected light image of the imaging plate 10, that is, the reflected light image based on detection of the reflected light L40 of the excitation light L10 from the imaging plate 10 but also the IP image region outside region image, and does not include the radiograph. It can be said that the erasing-time whole image is the reflected light image in the detection range R110 of the sensor 41. The reflected light image of the erased imaging plate 10, that is, the IP reflected light image representing the whole image of the imaging plate 10 is sometimes particularly referred to as an IP whole reflected light image. The reading apparatus 1 will be described below based on the assumption that the whole image based on the image signal output from the detector 40 as a result of detection of light is the acquired whole image. In description made below, the acquired whole image includes the light emission-time whole image and the erasing-time whole image.

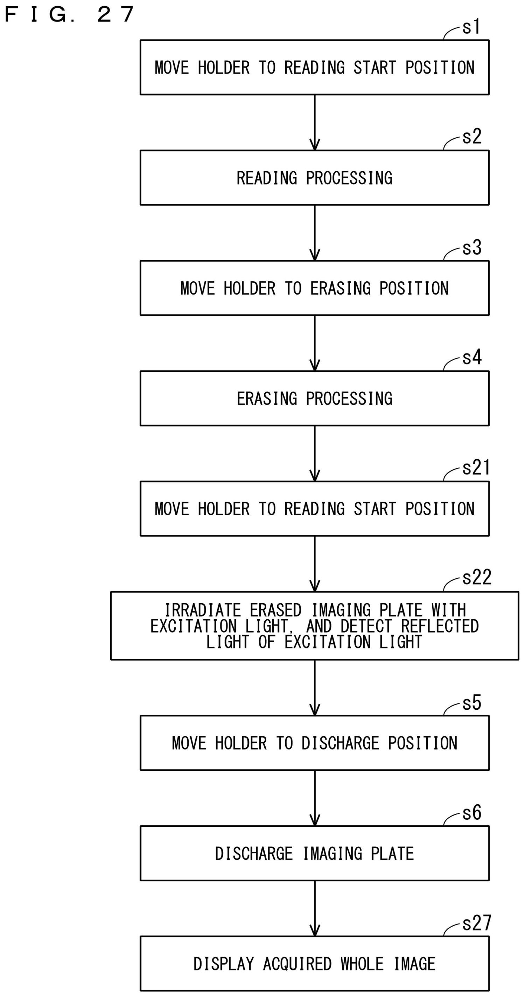

FIG. 27 is a flowchart showing one example of operation of the reading apparatus 1 in this example. When the start button included in the operation unit 4 is operated, the reading apparatus 1 performs the above-mentioned steps s1 to s4 as shown in FIG. 27. In the reading processing in step s2, the detector 40 outputs the light emission-time image signal as a result of detection of the emitted light L2 and the reflected light.

After the erasing processing in step s4, the driver 50 moves the holder 20 holding the erased imaging plate 10 to the reading start position in step s21. Step s22 is performed next. In step s22, the light source 30 irradiates the front surface of the erased imaging plate 10 and the IP image region outside region with the excitation light L10. The detector 40 detects the reflected light of the excitation light L10 from the front surface of the erased imaging plate 10 and the IP image region outside region, and outputs the erasing-time image signal as a result of detection. The erasing-time image signal is a gray-scale image signal as with the light emission-time image signal, for example.

After step s22, the above-mentioned steps s5 and s6 are performed to discharge the erased imaging plate 10 to the outlet 2b of the housing 2. Next, in step s27, the display control unit 82 causes the display 3 to simultaneously and separately display the light emission-time whole image based on the light emission-time image signal acquired in step s2 and the erasing-time whole image based on the erasing-time image signal acquired in step s22. In step s27, the image processing unit 81 displays the light emission-time whole image and the erasing-time whole image in grayscale, for example.

Step s27 may be performed at any time after step s22. For example, step s27 may be performed between step s22 and step s5. The light emission-time whole image and the erasing-time whole image may not simultaneously be displayed. The light emission-time whole image may not be displayed in step s27. At least one of the size identification processing and the tilt angle identification processing described above may be performed during a series of processes shown in FIG. 27, or may be performed at a different time from the processing shown in FIG. 27.

The erasing-time image signal output from the detector 40 includes luminance values of a plurality of pixels constituting the IP whole reflected light image (i.e., the IP image) and luminance values of a plurality of pixels constituting the IP image region outside region image. The luminance values included in the erasing-time image signal are greater when the reflected light detected by the detector 40 has a higher intensity. Thus, when the reflected light has a higher intensity in a certain region of the erased imaging plate 10, for example, luminance of the reflected light image in the certain region included in the erasing-time image signal is greater.

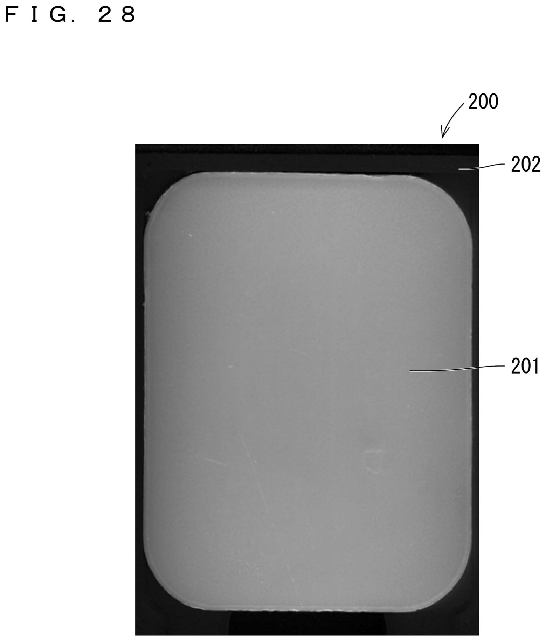

FIG. 28 is a schematic diagram showing one example of the erasing-time whole image 200. As shown in FIG. 28, the erasing-time whole image 200 includes the IP whole reflected light image 201 and the IP image region outside region image 202. In the example of FIG. 28, the front surface of the imaging plate 10 appears in the IP whole reflected light image 201. The IP whole reflected light image 201 is a portion of the erasing-time whole image 200 corresponding to the imaging plate 10, so that the IP whole reflected light image is also referred to as the IP corresponding portion. The IP whole reflected light image 201 is also the IP image, and is also the IP non-photostimulable reflected light image.

The image processing unit 81 performs image processing on the erasing-time image signal. In this example, the luminance reversal processing is not performed in the image processing performed on the erasing-time image signal, for example, in contrast to the image processing performed on the light emission-time image signal. Thus, when the reflected light has a higher intensity in a certain region of the imaging plate 10, a luminance value of the reflected light image in the certain region included in the erasing-time image signal after the image processing is greater as with that included in the erasing-time image signal before the image processing. On the other hand, when the reflected light has a lower intensity in a certain region of the imaging plate 10, the reflected light image in the certain region has a smaller luminance value. The erasing-time whole image 200 based on the erasing-time image signal on which the image processing not including the luminance reversal processing has been performed is hereinafter also referred to as a before-reversal erasing-time whole image 200.

The luminance reversal processing may be performed in the image processing performed on the erasing-time image signal. In this case, the erasing-time whole image based on the erasing-time image signal on which the image processing including the luminance reversal processing has been performed may be referred to as an after-reversal erasing-time whole image. The erasing-time whole image may include both the before-reversal erasing-time whole image and the after-reversal erasing-time whole image. The after-reversal erasing-time whole image may be used in place of the before-reversal erasing-time whole image 200.

Portions of the erasing-time image signal, the erasing-time whole image, the before-reversal erasing-time whole image, and the after-reversal erasing-time whole image representing the image of the imaging plate 10 may respectively be referred to as an erasing-time IP image signal, an erasing-time IP image, a before-reversal erasing-time IP image, and an after-reversal erasing-time IP image.

In step s27, the display control unit 82 may cause the display 3 to display the after-reversal light emission-time whole image 100b and the before-reversal erasing-time whole image 200 in grayscale, for example. In this case, the display 3 may display the after-reversal light emission-time whole image 100b in grayscale as shown in FIGs. 16 and 17 described above, and display the before-reversal erasing-time whole image 200 in grayscale as shown in FIG. 28 described above.

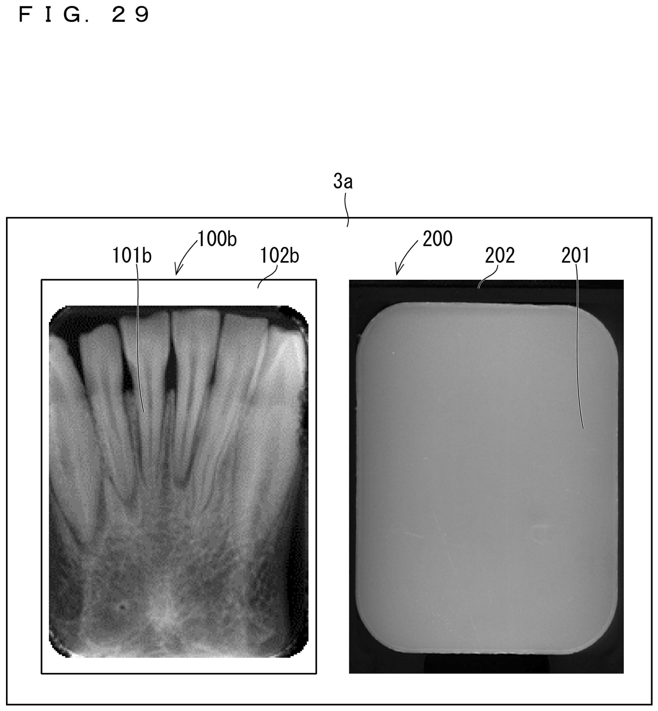

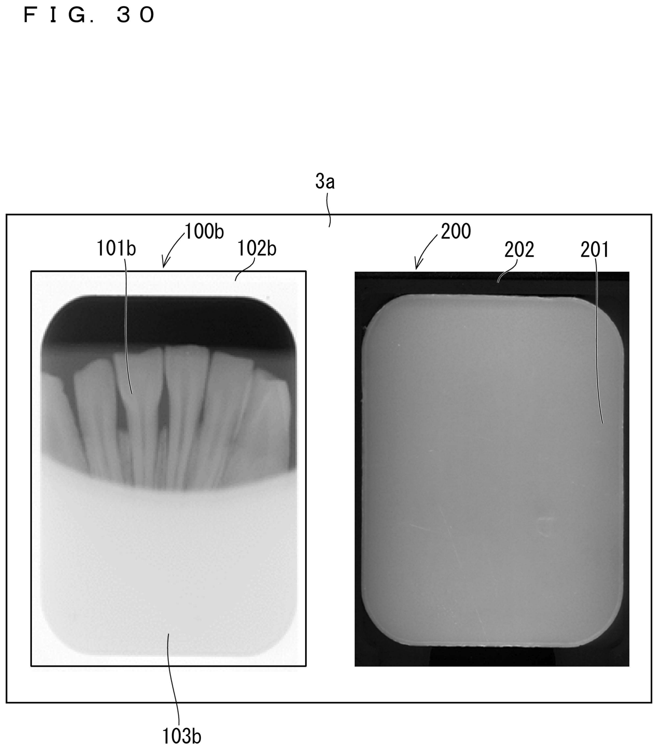

FIGs. 29 and 30 are schematic diagrams each showing one example of display of the after-reversal light emission-time whole image 100b and the before-reversal erasing-time whole image 200 on a display surface 3a of the display 3. FIG. 29 shows the after-reversal light emission-time whole image 100b and the before-reversal erasing-time whole image 200 when the imaging plate 10 does not include the unexposed portion. FIG. 30 shows the after-reversal light emission-time whole image 100b and the before-reversal erasing-time whole image 200 when the imaging plate 10 includes the unexposed portion.

As shown in FIGs. 29 and 30, the display 3 may display the after-reversal light emission-time whole image 100b and the before-reversal erasing-time whole image 200 in the same size and side by side, for example. In this example, an image having a greater luminance value is displayed by the display 3 to be brighter, so that a portion where the teeth appear (i.e., the IP excited region light image) and the unexposed region image (i.e., the IP reflected light image or the IP non-photostimulable reflected light image) 103b of the after-reversal light emission-time whole image 100b are displayed to be brighter, for example. A portion where the imaging plate 10 appears of the before-reversal erasing-time whole image 200 is displayed to be brighter.

A position of an outer edge of the radiograph 101b relative to an outer edge of the after-reversal light emission-time whole image 100b and a position of an outer edge of the IP whole reflected light image 201 relative to an outer edge of the before-reversal erasing-time whole image 200 preferably correspond to each other in display. As described above, a position of an outer edge of the IP biological radiographically captured image relative to an outer edge of the biological radiographically captured image and a position of an outer edge of the IP imaging plate shape image relative to an outer edge of the imaging plate shape image may correspond to each other. A match between a position of radiography of the biological radiographically captured image and a position of radiography of the imaging plate shape image facilitates corresponding arrangements in the image processing. Even if there is a difference between these positions of radiography, knowing and calculating the difference allow for the corresponding arrangements in the image processing.

In place of the before-reversal erasing-time whole image 200 displayed side by side with the after-reversal light emission-time whole image 100b, an image formed by extracting only the IP whole reflected light image 201, that is, the IP imaging plate shape image may be displayed side by side with the after-reversal light emission-time whole image 100b.

As shown in FIGs. 29 and 30, the before-reversal erasing-time whole image 200 when the imaging plate 10 includes the unexposed portion and the before-reversal erasing-time whole image 200 when the imaging plate 10 does not include the unexposed portion are the same. The user can check the before-reversal erasing-time whole image 200 (i.e., the erasing-time whole image) displayed by the display 3 for appearance of the imaging plate 10. A method of displaying the after-reversal light emission-time whole image 100b and the before-reversal erasing-time whole image 200 is not limited to that in the examples of FIGs. 29 and 30.

In the above-mentioned example of FIG. 27, the detector 40 detects the excited region light L20 from the imaging plate 10 excited by the excitation light L10 from the light source 30, and outputs the light emission-time image signal as a result of detection. Then, after the radiograph is erased from the imaging plate 10, the erased imaging plate 10 is irradiated with the excitation light L10 from the light source 30, and the detector 40 detects the reflected light of the excitation light L10 from the imaging plate 10. The reading apparatus 1 can thus easily acquire both the radiograph recorded on the imaging plate 10 and the reflected light image of the imaging plate 10 using the same light source 30 and the same detector 40.

When the light emission-time whole image and the erasing-time whole image are simultaneously and separately displayed as in the examples of FIGs. 29 and 30, the user can easily compare the light emission-time whole image and the erasing-time whole image. That is to say, the user can easily compare the radiograph read from the imaging plate 10 and the appearance of the imaging plate 10. The user can thus easily identify the unexposed portion that can be included in the imaging plate 10, for example. Description will be made in this respect below.

When the imaging plate 10 includes the unexposed portion, the radiograph is not present in a region of the light emission-time whole image corresponding to the unexposed portion. However, it is difficult for the user to determine, only from display of the light emission-time whole image, whether the radiograph is not present in the region due to failure of the reading apparatus 1 although the radiograph is actually present in the region or the radiograph is not present in the region as the imaging plate 10 includes the unexposed portion. In contrast, when the light emission-time whole image and the erasing-time whole image are simultaneously and separately displayed as in the example of FIG. 30, the user can check display of the light emission-time whole image for the radiograph read from the imaging plate 10, check display of the erasing-time whole image for the appearance of the imaging plate 10, and compare them to easily identify the unexposed portion that can be included in the imaging plate 10. The user can also easily identify a range of the unexposed portion that can be included in the imaging plate 10.

While the image processing unit 81 identifies the IP tilt angle based on the light emission-time whole image in the above-mentioned example, the IP tilt angle may be identified based on the erasing-time whole image (i.e., the reflected light image based on the erasing-time image signal). The image processing unit 81 may similarly identify the IP size based on the erasing-time whole image.

A configuration in which the light emission-time whole image and the erasing-time whole image are simultaneously and separately displayed as in the examples of FIGs. 29 and 30 may be modified to a configuration in which the light emission-time whole image and an image acquired by binarizing the light emission-time whole image are simultaneously and separately displayed by replacing the erasing-time whole image with an image such as the binarized image 500 in FIG. 21. It can be said that the erasing-time whole image is the imaging plate shape extraction image as the erasing-time whole image is an image representing the imaging plate shape extracted by performing processing on the IP acted light image signal as with the image such as the binarized image 500.

The image processing unit 81 can identify the IP tilt angle and the IP size based on the erasing-time whole image as in a case where the IP tilt angle and the IP size are identified based on the light emission-time whole image. Specifically, the image processing unit 81 binarizes the before-reversal erasing-time whole image 200 to generate a binarized image, for example. The binarized image is also referred to as a second binarized image. The binarized image acquired by binarizing the before-reversal erasing-time whole image 200 is one example of an imaging plate shape radiograph. A threshold used when the before-reversal erasing-time whole image 200 is binarized is set to be greater than a luminance value for the IP image region outside region image 202 included in the before-reversal erasing-time whole image 200 and smaller than a luminance value for the IP whole reflected light image 201 as the IP image included in the before-reversal erasing-time whole image 200, for example. Thus, the second binarized image is similar to the binarized image of the before-reversal light emission-time whole image, and a region corresponding to the IP image region outside region and a region corresponding to the imaging plate 10 of the second binarized image are respectively the low luminance region and the high luminance region. An outline of the high luminance region of the second binarized image has a shape responsive to the outline of the imaging plate 10 regardless of an exposed or unexposed state of the imaging plate 10 before erasing. The image processing unit 81 can identify the IP tilt angle, the transverse size, the longitudinal size, and the main surface area of the imaging plate 10, and the type of the IP size based on the second binarized image similarly to the foregoing. The IP tilt angle and the IP size are identified by the image processing unit 81 as the identification unit. As processing, processing similar to that in steps S11 to S18 may be performed after step S27.

As described above, even when the erasing-time image signal representing the reflected light image of the imaging plate 10 is used to identify the IP tilt angle, the IP tilt angle can properly be identified as in a case where the light emission-time image signal is used. Even when the erasing-time image signal is used to identify the IP size, the IP size can properly be identified as in a case where the light emission-time image signal is used.

### <Correction of Tilt of IP Corresponding Portion>

When the imaging plate 10 tilts relative to the reference orientation, the IP corresponding portion (i.e., the IP image) tilts in the acquired whole image as shown in FIG. 20 and the like. Specifically, when the imaging plate 10 tilts clockwise relative to the reference orientation, the longitudinal direction of the IP corresponding portion tilts clockwise relative to a direction (the longitudinal direction of the before-reversal whole image 100a in FIG. 20) corresponding to the subscannig direction DRs in the acquired whole image. On the other hand, when the imaging plate 10 tilts counterclockwise relative to the reference orientation, the longitudinal direction of the IP corresponding portion tilts counterclockwise relative to the direction corresponding to the subscannig direction DRs in the acquired whole image. When the acquired whole image is displayed with the IP corresponding portion tilting, the user can have difficulty viewing the tilting IP corresponding portion.

The image processing unit 81 may thus perform tilt correction processing of correcting a tilt of the IP corresponding portion on the acquired whole image based on the IP tilt angle α identified based on the light emission-time whole image or the erasing-time whole image. When the acquired whole image as a target of correction is the light emission-time whole image based on detection of the emitted light L2, the tilt of the IP corresponding portion is corrected to correct a tilt of the radiograph included in the IP corresponding portion in the tilt correction processing. On the other hand, when the acquired whole image as the target of correction is the erasing-time whole image based on detection of the reflected light, a tilt of the IP whole reflected light image in which the imaging plate 10 appears is corrected in the tilt correction processing. The image processing unit 81 may perform the tilt correction processing on the acquired whole image based on the image signal before the luminance reversal processing, or may perform the tilt correction processing on the acquired whole image based on the image signal after the luminance reversal processing. The display 3 may display the acquired whole image after the tilt correction processing. The tilt correction processing may be performed during the series of processes shown in FIG. 18, or may be performed at a different time from the processing shown in FIG. 18. The tilt correction processing may be performed during the series of processes shown in FIG. 27, or may be performed at a different time from the processing shown in FIG. 27. It can be said that the tilt correction processing is processing of correcting the tilt of the IP corresponding portion (i.e., the IP image) in the acquired whole image in response to the tilt of the imaging plate 10 relative to the reference orientation.

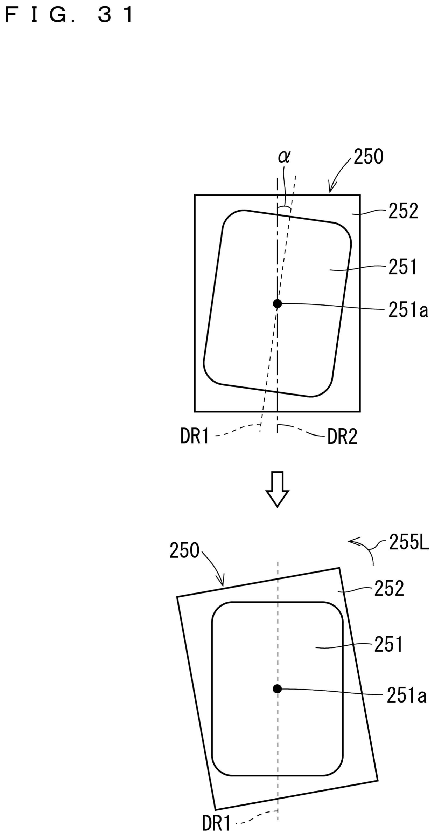

FIG. 31 is a schematic diagram for explaining one example of the tilt correction processing. An acquired whole image 250 before the tilt correction processing is shown at the top of FIG. 29, and an acquired whole image 250 after the tilt correction processing is shown at the bottom of FIG. 29. The acquired whole image 250 as the target of correction includes an IP corresponding portion (i.e., the IP image) 251 and an IP image region outside region image 252. In FIG. 31, a longitudinal direction DR1 of the IP corresponding portion 251 is indicated by a dashed line, and a direction DR2 corresponding to the subscannig direction DRs in the acquired whole image 250 is indicated by an alternate long and short dashed line.

In the tilt correction processing, the image processing unit 81 determines a center of gravity 251a of the IP corresponding portion 251 included in the acquired whole image 250 as the target of correction. The center of gravity 251a of the IP corresponding portion 251 herein matches a center of gravity of the high luminance region of the binarized image of the acquired whole image 250. The image processing unit 81 generates the binarized image of the acquired whole image 250, and determines the center of gravity of the high luminance region of the generated binarized image to determine the center of gravity 251a of the IP corresponding portion 251 of the acquired whole image 250. Next, the image processing unit 81 rotates the acquired whole image 250 about the determined center of gravity 251a by the IP tilt angle α. In this case, when the IP tilt angle α is a positive angle, the image processing unit 81 rotates the acquired whole image 250 counterclockwise 255L by the IP tilt angle α as shown in FIG. 31. On the other hand, when the IP tilt angle α is a negative angle, the image processing unit 81 rotates the acquired whole image 250 clockwise by the IP tilt angle α. The tilt of the IP corresponding portion 251 is thereby corrected, so that the longitudinal direction DR1 of the IP corresponding portion 251 is parallel to the direction DR2 corresponding to the subscannig direction DRs in the acquired whole image 250.

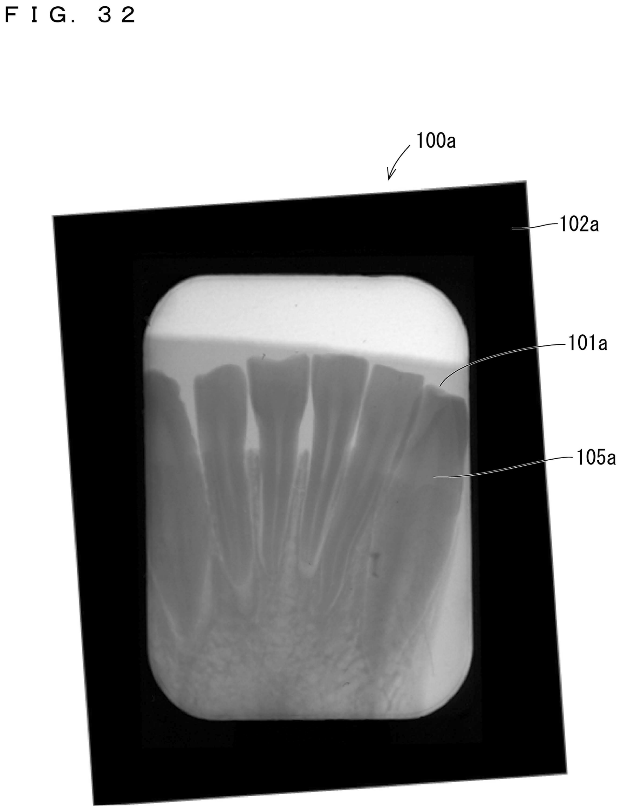

FIG. 32 is a schematic diagram showing one example of the tilt correction processing having been performed on the before-reversal light emission-time whole image 100a shown in FIG. 20 described above. As can be understood from comparison between FIGs. 20 and 32, the tilt of the radiograph 101a included in the IP corresponding portion (i.e., the IP image) 105a is properly corrected by the tilt correction processing performed on the before-reversal light emission-time whole image 100a. The orientation of the IP corresponding portion 105a in the before-reversal light emission-time whole image 100a on which the tilt correction processing has been performed is the same as the orientation of the IP corresponding portion 105a in the before-reversal light emission-time whole image 100a acquired when the orientation of the imaging plate 10 is the reference orientation. The display 3 may display the before-reversal light emission-time whole image 100a after the tilt correction processing in grayscale as in FIG. 32, for example. Display of the acquired whole image hereinafter includes display of the acquired whole image after the tilt correction processing.

As described above, the image processing unit 81 corrects the tilt of the IP corresponding portion of the acquired whole image based on the IP tilt angle to acquire the IP corresponding portion whose tilt has been properly corrected. In this case, the image processing unit 81 functions as a correction processing unit that corrects a tilt of an image (the IP acted light image) of the imaging plate 10. For example, when the tilt correction processing is performed on the light emission-time whole image, the radiograph whose tilt has been properly corrected can be acquired. When the tilt correction processing is performed on the erasing-time whole image, the IP whole reflected light image whose tilt has been properly corrected can be acquired.

### <Cutting-out Processing on Acquired Whole Image>

The image processing unit 81 may perform cutting-out processing of determining a cutout image to be cut out from the light emission-time whole image including the radiograph based on the IP tilt angle and the IP size, and cutting out the determined cutout image from the light emission-time whole image. A desired cutout image responsive to the IP tilt angle and the IP size can be acquired from the light emission-time whole image by the cutting-out processing. The image processing unit 81 functions as a cutting-out unit that performs the cutting-out processing. The cutting-out processing may be performed during the series of processes shown in FIG. 18, or may be performed at a different time from the processing shown in FIG. 18. The cutting-out processing may be performed during the series of processes shown in FIG. 27, or may be performed at a different time from the processing shown in FIG. 27.

In the cutting-out processing, the IP corresponding portion (i.e., the IP image) of the light emission-time whole image may be determined as the cutout image, for example. In this case, the image processing unit 81 determines the IP corresponding portion of the light emission-time whole image as the cutout image based on the type of the IP size and the IP tilt angle identified based on the light emission-time whole image or the erasing-time whole image, for example. One example of operation of the image processing unit 81 when the image processing unit 81 determines the IP corresponding portion of the light emission-time whole image as the cutout image will be described below. The type of the IP size identified by the image processing unit 81 is hereinafter also referred to as an identified size Z. In this example, Z has a value of any of 0, 1, 2, and 3.

In the cutting-out processing, the image processing unit 81 sets a cutout frame to the light emission-time whole image based on the type of the IP size and the IP tilt angle, for example. The image processing unit 81 determines a portion within the cutout frame of the light emission-time whole image as the cutout image.

The shape of the cutout frame is similar to a nominal outline of the imaging plate 10 of the identified size Z. In this example, the imaging plate 10 has a rectangular outline with four rounded corners, and thus the cutout frame has a rectangular shape with four rounded corners. The size in the transverse direction of the cutout frame has a value responsive to the nominal transverse size (also referred to as a nominal transverse size for the identified size Z) of the imaging plate 10 of the identified size Z, and the size in the longitudinal direction of the cutout frame has a value responsive to the nominal longitudinal size (also referred to as a nominal longitudinal size for the identified size Z) of the imaging plate 10 of the identified size Z.

The image processing unit 81 herein knows in advance, for each type of the IP size, the number of pixels in the acquired whole image corresponding to each of the nominal transverse size and the nominal longitudinal size. When P1 pixels correspond to the nominal transverse size for the identified size Z, and P2 pixels correspond to the nominal longitudinal size for the identified size Z, the image processing unit 81 sets the size in the transverse direction of the cutout frame to a length of P1 pixels, and sets the size in the longitudinal direction of the cutout frame to a length of P2 pixels. When equations P1 = 800 and P2 = 1100 hold, for example, the size in the transverse direction of the cutout frame is set to a length of 800 pixels, and the size in the longitudinal direction of the cutout frame is set to a length of 1100 pixels.

When determining the outline and the size of the cutout frame, the image processing unit 81 disposes the cutout frame on the light emission-time whole image so that the center of the cutout frame matches the center of gravity of the IP corresponding portion of the light emission-time whole image, and the longitudinal direction and the transverse direction of the cutout frame are respectively parallel to the longitudinal direction and the transverse direction of the light emission-time whole image. As described above, the center of gravity of the IP corresponding portion of the light emission-time whole image matches the center of gravity of the high luminance region of the binarized image of the light emission-time whole image.

Next, the image processing unit 81 rotates the cutout frame disposed on the light emission-time whole image about the center of gravity of the IP corresponding portion by the IP tilt angle. In this case, the image processing unit 81 rotates the cutout frame clockwise when the IP tilt angle is a positive angle, and rotates the cutout frame counterclockwise when the IP tilt angle is a negative angle. The portion within the cutout frame of the light emission-time whole image thus matches the IP corresponding portion. The image processing unit 81 determines a portion within the cutout frame rotated by the IP tilt angle of the light emission-time whole image as the cutout image. The image processing unit 81 then cuts out the determined cutout image from the light emission-time whole image. The IP corresponding portion is thereby cut out from the light emission-time whole image to acquire an image of only a portion corresponding to the imaging plate 10. The cutting-out processing may be performed on the before-reversal light emission-time whole image or on the after-reversal light emission-time whole image. For the purpose of determining a cutout range, the nominal size may not necessarily be applied to the cutout frame, and an imaging plate range acquired as a result of binarization may be applied to the cutout frame.

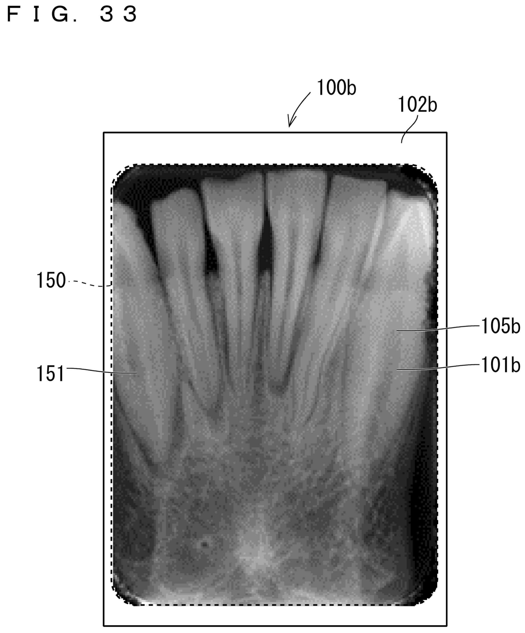

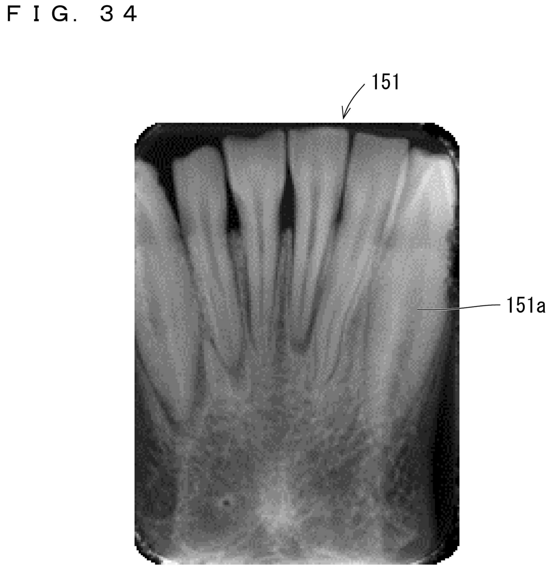

FIG. 33 is a schematic diagram showing one example of setting of a cutout frame 150 to the after-reversal light emission-time whole image 100b shown in FIG. 16 described above. The biological radiographically captured image, such as the after-reversal light emission-time whole image 100b, as a target of cutting-out includes the IP image region and the IP image region outside region image. FIG. 34 is a schematic diagram showing one example of cutting-out of the cutout image 151 within the cutout frame 150 of the after-reversal light emission-time whole image 100b from the after-reversal light emission-time whole image 100b in the example of FIG. 33. As shown in FIG. 34, the IP corresponding portion 105b is properly cut out from the after-reversal light emission-time whole image 100b. The cutout image 151 includes a radiograph 151a that is the same as the radiograph (i.e., the excited region light image) 101b included in the after-reversal light emission-time whole image 100b.

Application of the imaging plate shape data to the biological radiographically captured image to cut out the IP corresponding portion 105b from the after-reversal light emission-time whole image 100b is referred to as imaging plate shape application processing. The imaging plate shape application processing includes positioning of the imaging plate shape for cutting-out from the biological radiographically captured image, setting of the cutout frame, and extraction of the IP biological radiographically captured image as the cutout image from the biological radiographically captured image as a result of cutting-out.

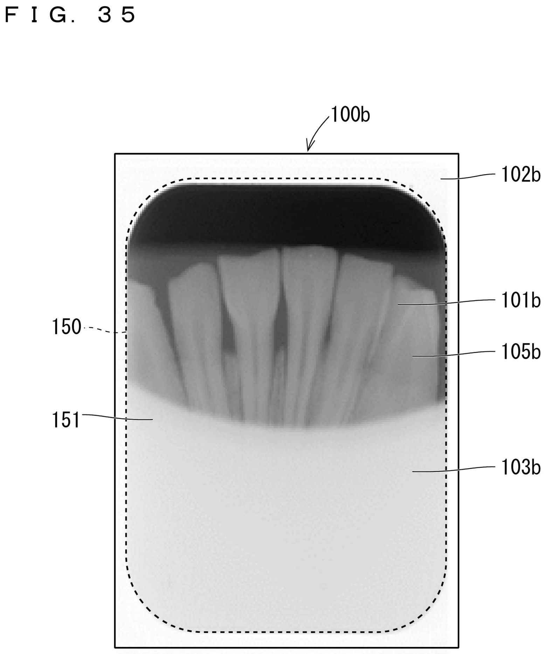

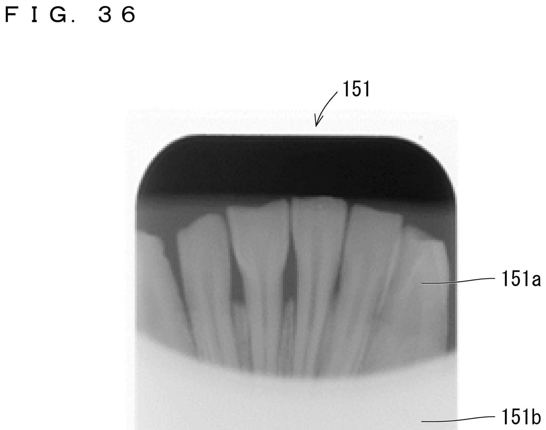

FIG. 35 is a schematic diagram showing one example of setting of the cutout frame 150 to the after-reversal light emission-time whole image 100b shown in FIG. 17 described above. FIG. 36 is a schematic diagram showing one example of cutting-out of the cutout image 151 within the cutout frame 150 of the after-reversal light emission-time whole image 100b from the after-reversal light emission-time whole image 100b in the example of FIG. 35. As shown in FIG. 36, the IP corresponding portion 105b is properly cut out from the after-reversal light emission-time whole image 100b even when the after-reversal light emission-time whole image 100b includes the unexposed region image 103b. The cutout image 151 includes the radiograph 151a that is the same as the radiograph 101b included in the after-reversal light emission-time whole image 100b and an unexposed region image 151b that is the same as the unexposed region image (i.e., the IP reflected light image or the IP non-photostimulable reflected light image) 103b included in the after-reversal light emission-time whole image 100b.

When the cutting-out processing is performed, the display 3 may display the cutout image 151 cut out from the light emission-time whole image under control performed by the display control unit 82. In this case, the display 3 may display the cutout image 151 in grayscale as shown in FIGs. 34 and 36. It can be said that FIGs. 34 and 36 are schematic diagrams each showing an example of display of the cutout image 151. When the cutout image 151 is displayed, the user can check only a necessary image of the light emission-time whole image. When the IP corresponding portion is cut out from the light emission-time whole image as the cutout image 151 and displayed as in this example, the user can check only the IP corresponding portion that is necessary of the light emission-time whole image. The user can thus easily identify the unexposed portion that can be included in the imaging plate 10 by checking display of the cutout image 151 as shown in FIG. 36, for example. The user can also easily identify a range of the unexposed portion that can be included in the imaging plate 10.

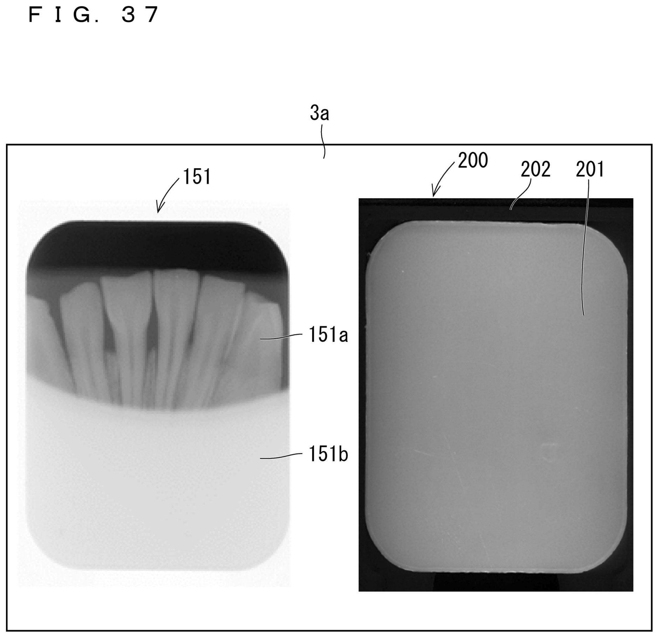

The display 3 may simultaneously and separately display the cutout image 151 cut out from the light emission-time whole image and the erasing-time whole image 200 under control performed by the display control unit 82. FIG. 37 is a schematic diagram showing one example of display of the cutout image 151 and the erasing-time whole image 200 in grayscale on the display surface 3a of the display 3. The cutout image 151 including the unexposed region image 151b is shown in FIG. 37. As shown in FIG. 37, the display 3 may display the cutout image 151 and the erasing-time whole image 200 so that the cutout image 151 and the IP whole reflected light image (i.e., the IP corresponding portion or the IP image) 201 of the erasing-time whole image 200 have the same size, for example. The display 3 may display the cutout image 151 and the erasing-time whole image 200 side by side, for example. The display 3 simultaneously and separately displays the cutout image 151 and the erasing-time whole image 200, so that the user can easily compare the cutout image 151 and the erasing-time whole image 200. Thus, by comparing the IP whole reflected light image 201 included in the erasing-time whole image 200 and the cutout image 151, the user can easily determine whether the IP corresponding portion has properly been cut out from the light emission-time whole image, for example.

The cutout frame 150, that is, the cutout range may be determined based on the erasing-time whole image 200. In this case, a range of the IP non-photostimulable reflected light image (i.e., the IP whole reflected light image) of the erasing-time whole image 200 may be identified by image processing, such as boundary processing, to determine the cutout range, and the determined cutout range may be set to the light emission-time whole image to generate the cutout image 151, for example. In this case, acquisition of the IP binarized image may be used in the image processing, such as the boundary processing. Holding of the imaging plate 10 by the holder 20 in the same orientation during light emission and during erasing allows for positional application.

A configuration in which the cutout image and the erasing-time whole image are simultaneously and separately displayed as in the example of FIG. 37 may be modified to a configuration in which the cutout image and the image acquired by binarizing the erasing-time whole image are simultaneously and separately displayed by replacing the erasing-time whole image with the image such as the binarized image 500 in FIG. 21.

As in the above-mentioned tilt correction processing, the image processing unit 81 may correct a tilt of the cutout image 151 cut out from the light emission-time whole image based on the IP tilt angle identified based on the light emission-time whole image or the erasing-time whole image. Thus, when the cutout image 151 after correction is displayed, the user can easily view the cutout image 151, for example. Assume hereinafter that the tilt correction processing includes correction of the tilt of the cutout image 151.

The image processing unit 81 corrects the tilt of the cutout image 151 as in the above-mentioned tilt correction processing performed on the light emission-time whole image, for example. The image processing unit 81 still functions as the correction processing unit that corrects the tilt of the image (the IP acted light image) of the imaging plate 10. Specifically, the image processing unit 81 first determines a center of gravity of the cutout image 151. The center of gravity of the cutout image 151 matches the center of gravity of the high luminance region of the binarized image of the light emission-time whole image. The image processing unit 81 thus determines the center of gravity of the high luminance region of the binarized image of the light emission-time whole image to determine the center of gravity of the cutout image 151. Next, the image processing unit 81 rotates the cutout image 151 about the determined center of gravity by the IP tilt angle. In this case, when the IP tilt angle is a positive angle, the image processing unit 81 rotates the cutout image 151 counterclockwise by the IP tilt angle. On the other hand, when the IP tilt angle is a negative angle, the image processing unit 81 rotates the cutout image 151 clockwise by the IP tilt angle. The tilt of the cutout image 151 is thereby corrected. The tilt of the radiograph based on the light emission-time image signal included in the cutout image 151 is thereby corrected.

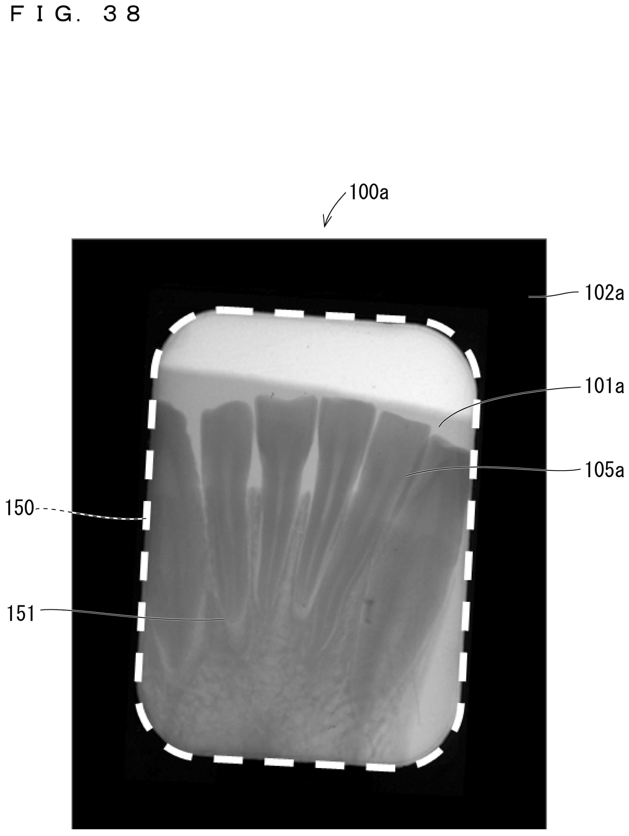

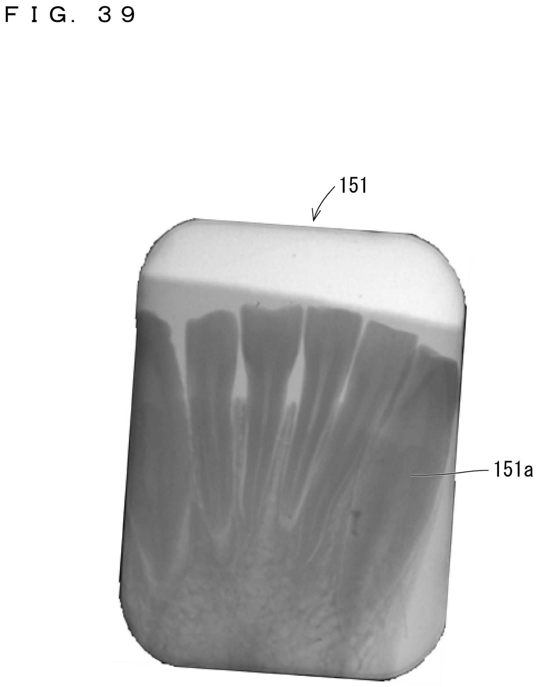

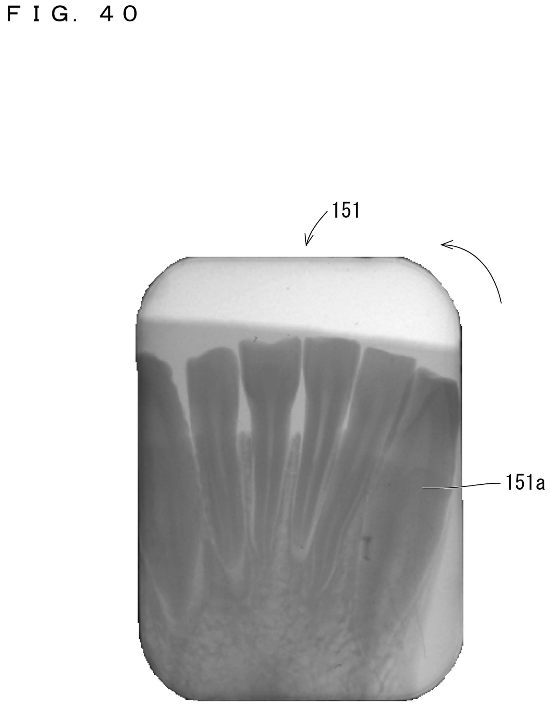

FIGs. 38 to 40 are schematic diagrams showing one example of the cutting-out processing performed on the light emission-time whole image and the tilt correction processing performed on the cutout image 151 after the cutting-out processing. In the example of FIGs. 38 to 40, the cutting-out processing is performed on the before-reversal light emission-time whole image 100a shown in FIG. 20 described above. As shown in FIG. 38, the image processing unit 81 sets the cutout frame 150 to the before-reversal light emission-time whole image 100a as described above. Next, the image processing unit 81 cuts out the cutout image 151 within the cutout frame 150 of the before-reversal light emission-time whole image 100a from the before-reversal light emission-time whole image 100a as shown in FIG. 39. The image processing unit 81 then corrects the tilt of the cut out cutout image 151 based on the IP tilt angle as shown in FIG. 40. The tilt of the cutout image 151 responsive to the tilt of the imaging plate 10 relative to the reference orientation is thereby properly corrected. The orientation of the cutout image 151 whose tilt has been corrected is the same as the orientation of the cutout image 151 obtained when the orientation of the imaging plate 10 is the reference orientation.

While the image processing unit 81 sets the IP corresponding portion of the light emission-time whole image to the cutout image 151 in the above-mentioned example, a portion of the IP corresponding portion may be set to the cutout image 151. One example of operation of the image processing unit 81 in this case will be described below.

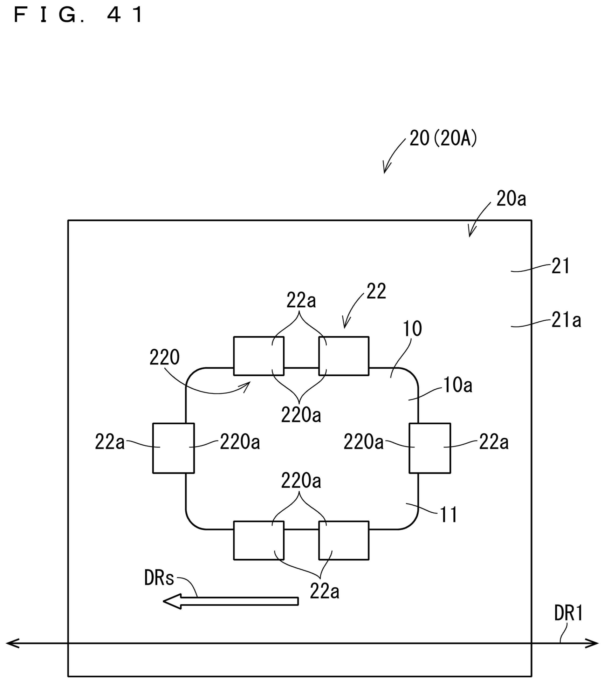

FIG. 41 is a schematic diagram illustrating another example of the holder 20 that holds the imaging plate 10. FIG. 41 illustrates a state of each of the fixing portions 22a of the fixture 22 of the holder 20 being in the close position. In the holder 20 (also referred to as a holder 20A) illustrated in FIG. 41, the fixture 22 has an overlapping portion 220 that covers a peripheral edge portion 10a of the imaging plate 10 when each of the fixing portions 22a is in the close position. The fixing portions 22a have respective overlapping portions 220a that cover the peripheral edge portion 10a of the imaging plate 10 when being in the close position. The overlapping portions 220a of the fixing portions 22a constitute the overlapping portion 220 of the fixture 22.

When the holder 20A holds the imaging plate 10, the peripheral edge portion 10a of the imaging plate 10 is covered with the overlapping portion 220 of the fixture 22, so that the light emission-time whole image in which the overlapping portion 220 appears can be acquired in the reading apparatus 1. From a perspective of the imaging plate 10, the overlapping portion 220 may be considered as a grasped portion of the imaging plate 10.

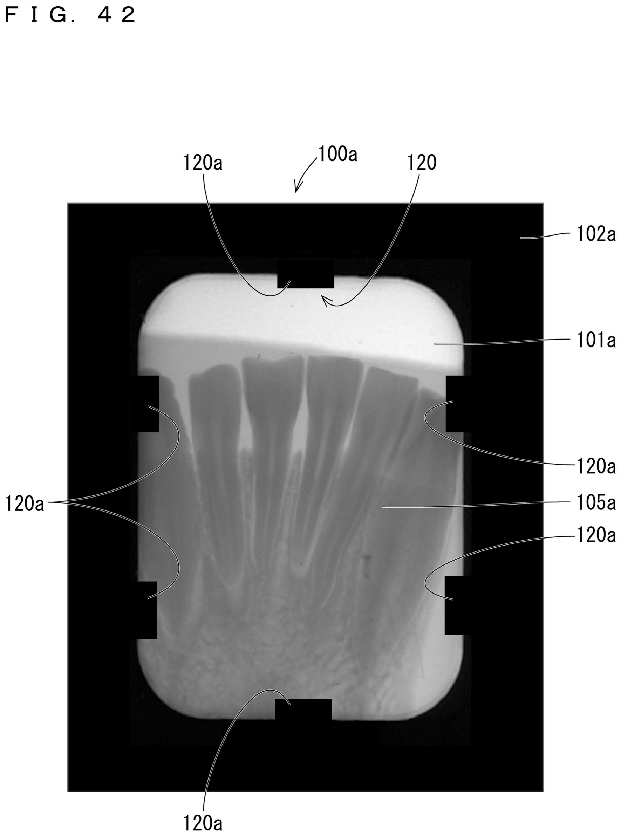

FIG. 42 is a schematic diagram showing one example of the before-reversal light emission-time whole image 100a acquired when the imaging plate 10 is held by the holder 20A. The before-reversal light emission-time whole image 100a shown in FIG. 42 includes an image 120 (also referred to as an overlapping portion image 120) of the overlapping portion 220 of the fixture 22. The overlapping portion image 120 is an image based on detection of the reflected light of the excitation light L10 from the overlapping portion 220. The overlapping portion image 120 includes images 120a (also referred to as overlapping portion images 120a) of the overlapping portions 220a of the fixing portions (also referred to as grasping portions) 22a. In this example, black anodizing has been performed on surfaces of the fixing portions 22a, for example. The overlapping portion image 120 included in the before-reversal light emission-time whole image 100a thus has a small luminance value. The overlapping portion images 120a are included in the IP image region outside region image 102a, and are not included in the radiograph 101a forming the IP image (IP image region light image).

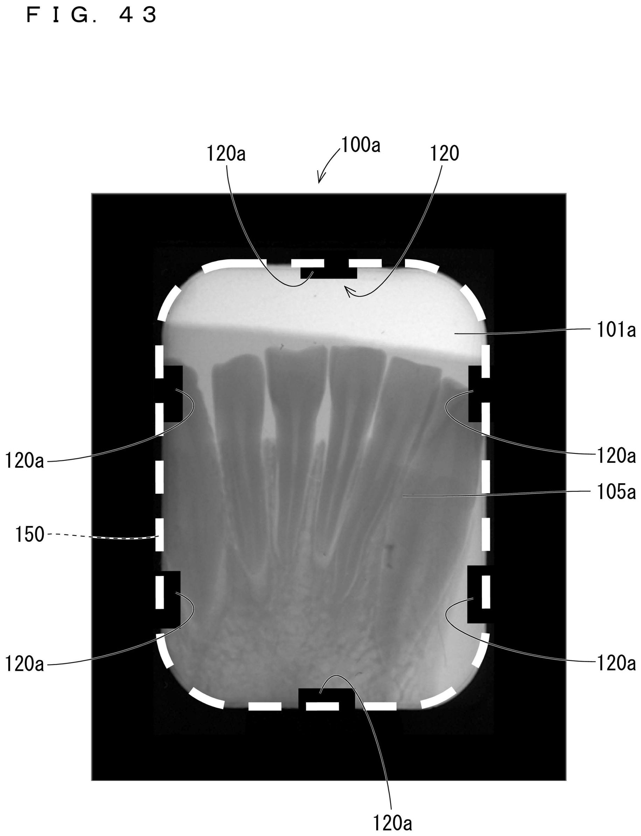

When the overlapping portion image 120 is included in the light emission-time whole image, the image processing unit 81 may determine a portion of the IP corresponding portion as the cutout image 151 so that the cutout image 151 does not include the overlapping portion image 120 (i.e., the overlapping portion images 120a) in the light emission-time whole image. In this case, the image processing unit 81 sets the cutout frame 150 to the light emission-time whole image based on the type of the IP size and the IP tilt angle similarly to the foregoing, for example. An outline of the cutout frame 150 set as described above has a shape responsive to the outline of the imaging plate 10 (i.e., a contour of the imaging plate 10), so that the overlapping portion image 120 is included within the cutout frame 150 at this time point. FIG. 43 is a schematic diagram showing one example of setting of the cutout frame 150 to the before-reversal light emission-time whole image 100a shown in FIG. 42 as described above. The overlapping portion images 120a constituting the overlapping portion image 120 are included within the cutout frame 150 set to the before-reversal light emission-time whole image 100a.

While the before-reversal light emission-time whole image 100a shown in FIG. 42 includes an IP image (i.e., a partially missing IP image compared with that in FIG. 32) in a state of the fixing portions 22a overlapping the imaging plate 10, the cutout frame 150 responsive to the outline of the imaging plate 10 can be set as described below, for example. For example, the first principal component axis and the second principal component axis are determined by a method similar to the method described with reference to FIGs. 21 to 23. The first principal component axis and the second principal component axis can be determined similarly to the foregoing while the overlapping portion images 120a appear in the IP image but the amount of appearance is not large enough to interfere with calculation. A maximum value of a width in the longitudinal direction of the high luminance region 501 and a maximum value of a width in the transverse direction of the high luminance region 501 are determined respectively in a direction along the first principal component axis and a direction along the second principal component axis. The maximum value of the width in the longitudinal direction of the high luminance region 501 is set to a longitudinal width of the cutout frame 150, and the maximum value of the width in the transverse direction of the high luminance region 501 is set to a transverse width of the cutout frame 150. Alternatively, when the transverse sizes and the longitudinal sizes of the imaging plates 10 of the plurality of types of sizes are respectively stored as the nominal transverse sizes and the nominal longitudinal sizes as described above, the shapes of the imaging plates 10 of the respective sizes may be stored, any of the sizes that the determined maximum values suit may be checked, and the cutout frame may be set according to the shape of the imaging plate 10 of the size.

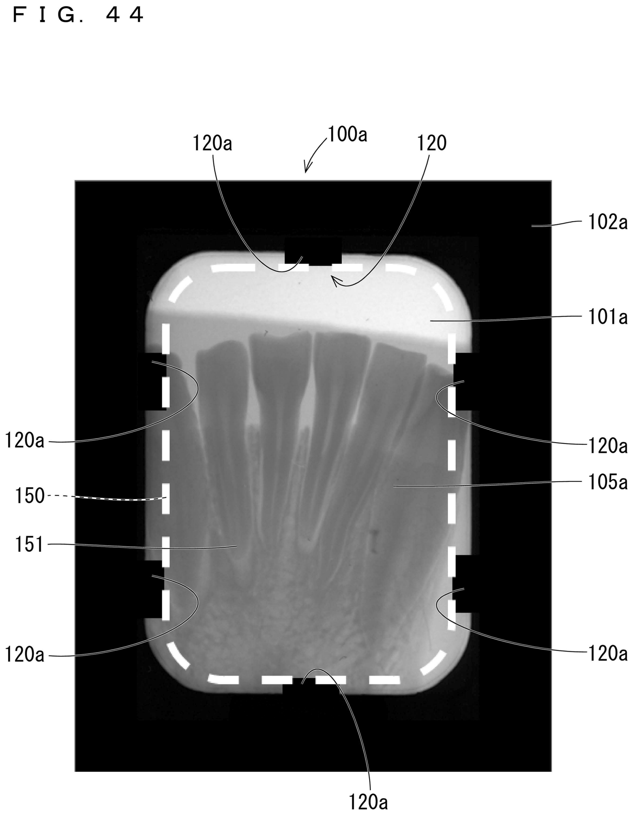

After setting the cutout frame 150 to the light emission-time whole image as described above, the image processing unit 81 reduces the size of the cutout frame 150 in a similar shape so that the overlapping portion image 120 is not included within the cutout frame 150. In this case, the image processing unit 81 reduces the size of the cutout frame 150 in the similar shape so that the overlapping portion image 120 is not included within the cutout frame 150, and the cutout frame 150 after size reduction is as large as possible. The image processing unit 81 can identify positions and ranges of the overlapping portion images 120a in the light emission-time whole image based on the binarized image of the light emission-time whole image, for example. The image processing unit 81 reduces the size of the cutout frame 150 in the similar shape so that the overlapping portion image 120 is not included within the cutout frame 150, and the cutout frame 150 after size reduction is as large as possible based on the identified positions and ranges of the overlapping portion images 120a. The image processing unit 81 determines a portion within the cutout frame 150 after size reduction of the light emission-time whole image as the cutout image 151. The cutout image 151 not including the overlapping portion image 120 but including a large portion of the IP corresponding portion 105a can thereby be acquired. FIG. 44 is a schematic diagram showing one example of size reduction of the cutout frame 150 shown in FIG. 43. As shown in FIG. 44, the image processing unit 81 determines a portion within the cutout frame 150 after size reduction of the before-reversal light emission-time whole image 100a as the cutout image 151.

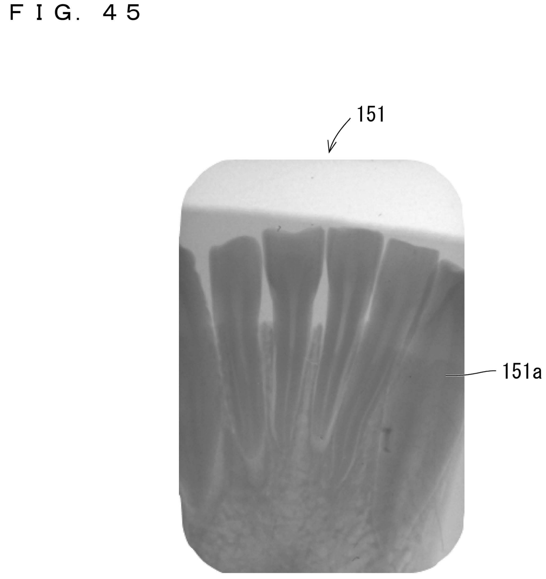

When determining the cutout image 151, the image processing unit 81 cuts out the cutout image 151 from the light emission-time whole image. FIG. 45 is a schematic diagram showing one example of cutting-out of the cutout image 151 shown in FIG. 44 from the before-reversal whole image 100a.

As described above, when the image processing unit 81 determines a portion of the IP corresponding portion as the cutout image 151 so that the cutout image 151 does not include the overlapping portion image 120, the cutout image 151 in which the overlapping portion 220 of the fixture 22 does not appear can be acquired. Thus, when the cutout image 151 is displayed, the user can check only a portion in which an image useful for a diagnosis is left as widely as possible without being distracted by the image of the overlapping portion 220, for example.

An end portion of the surface on a side of the radiograph formation layer 11 of the imaging plate 10 in plan view is sometimes considered as a non-effective region, and most of a region in the center of the surface is sometimes considered as an effective region. For example, in FIG. 44, a region inside a boundary indicated by the cutout frame 150 is considered as the effective region, and a region outside the boundary is considered as the non-effective region. This is because pressure is sometimes applied to the end portion (non-effective region) of the imaging plate 10 in a manufacturing process, and, in this case, good storage of radiographs cannot be ensured in the non-effective region. Only an image in the effective region can be checked by displaying the cutout image 151 in which the overlapping portion 220 of the fixture 22 does not appear.

Even when the cutout image 151 in which the overlapping portion 220 does not appear is cut out from the light emission-time whole image, the image processing unit 81 may correct the tilt of the cut out cutout image 151 based on the IP tilt angle as described above. In this case, the display control unit 82 may cause the display 3 to display the cutout image 151 whose tilt has been corrected.

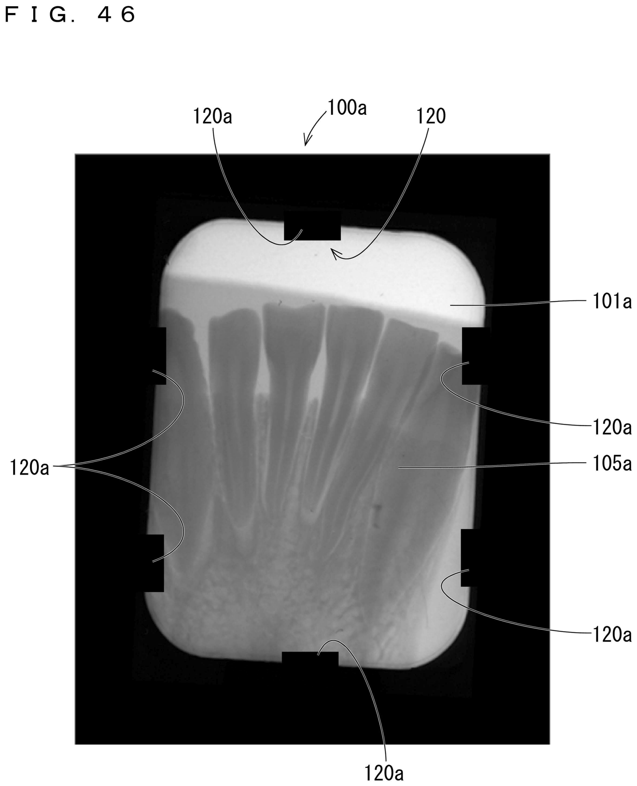

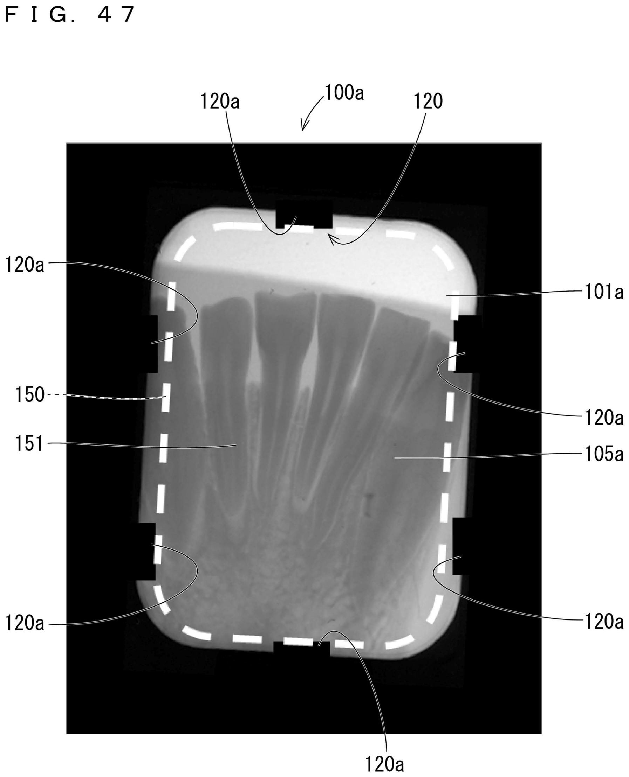

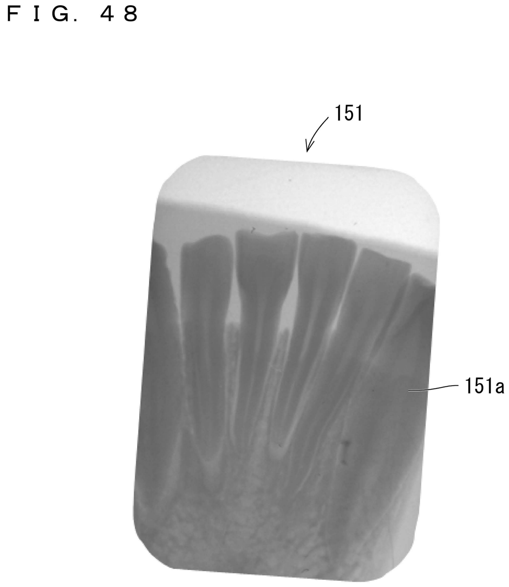

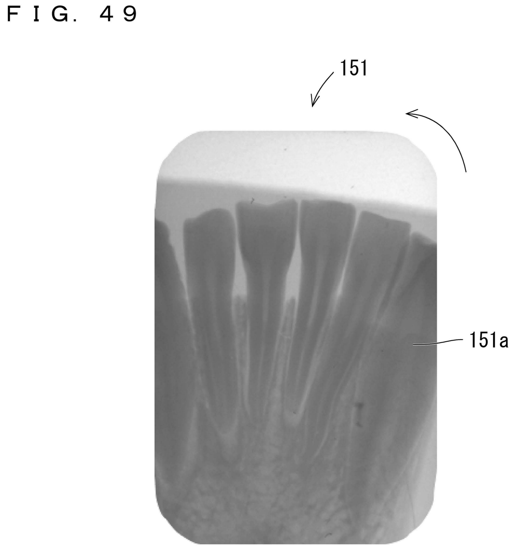

FIG. 46 is a schematic diagram showing another example of the before-reversal light emission-time whole image 100a acquired when the imaging plate 10 is held by the holder 20A. FIGs. 47 to 49 are schematic diagrams showing one example of the cutting-out processing performed on the before-reversal light emission-time whole image 100a shown in FIG. 46 and the tilt correction processing performed on the cutout image 151 after the cutting-out processing. The image processing unit 81 sets the cutout frame 150 to the before-reversal light emission-time whole image 100a as described above, and then reduces the size of the cutout frame 150 in the similar shape so that the overlapping portion image 120 is not included within the cutout frame 150, and the cutout frame 150 after size reduction is as large as possible as shown in FIG. 47. Next, the image processing unit 81 cuts out the cutout image 151 within the cutout frame 150 of the before-reversal light emission-time whole image 100a from the before-reversal light emission-time whole image 100a as shown in FIG. 48. The image processing unit 81 then corrects the tilt of the cut out cutout image 151 based on the IP tilt angle as shown in FIG. 49. The tilt of the cutout image 151 responsive to the tilt of the imaging plate 10 relative to the reference orientation is thereby properly corrected.

As described above, the image processing unit 81 determines at least portion of the IP corresponding portion of the light emission-time whole image as the cutout image based on the IP size and the IP tilt angle, so that at least portion of the IP corresponding portion can properly be cut out from the light emission-time whole image. The fixing portions 22a are portions being in contact with the end portion of the imaging plate 10 to fix the imaging plate 10, and thus the overlapping portion image 120 is typically present only in the end portion of the whole image. The cutout image may thus be determined to be an image in a region of a central portion obtained by removing the end portion including the overlapping portion image 120.

Even when the light emission-time whole image does not include the overlapping portion image 120, the image processing unit 81 may reduce the size of the cutout frame 150 set to the light emission-time whole image in the similar shape, and determine the portion within the cutout frame 150 after size reduction of the light emission-time whole image as the cutout image 151. Also in this case, a portion of the IP corresponding portion (i.e., the IP image) of the light emission-time whole image is determined as the cutout image 151. The image processing unit 81 may increase the size of the cutout frame 150 set to the light emission-time whole image, for example, in a similar shape, and determine a portion within the cutout frame 150 after a size increase of the light emission-time whole image as the cutout image 151. In this case, the IP corresponding portion and a portion around the IP corresponding portion (e.g., a portion of the IP image region outside region image or at least portion of the overlapping portion image 120) are determined as the cutout image 151 in the light emission-time whole image.

In the cutting-out processing, the image processing unit 81 may determine a cutout image (also referred to as a second cutout image) to be cut out from the erasing-time whole image including the IP whole reflected light image based on the IP tilt angle and the IP size, and cut out the determined second cutout image from the erasing-time whole image. In this case, the image processing unit 81 may determine at least portion of the IP corresponding portion (i.e., the IP whole reflected light image) of the erasing-time whole image as the second cutout image. When the erasing-time whole image includes the image of the overlapping portion 220, the image processing unit 81 may determine the second cutout image so that the second cutout image does not include the image of the overlapping portion 220. The display control unit 82 may cause the display 3 to display the second cutout image cut out from the erasing-time whole image. In this case, the display 3 may simultaneously and separately display the second cutout image and the cutout image (also referred to as a first cutout image) 151 cut out from the light emission-time whole image. The image processing unit 81 may also correct a tilt of the cut out second cutout image based on the IP tilt angle. The display control unit 82 may cause the display 3 to display the second cutout image whose tilt has been corrected. In this case, the display 3 may simultaneously and separately display the second cutout image whose tilt has been corrected and the first cutout image whose tilt has been corrected.

When the imaging plate 10 hardly tilts relative to the reference orientation, the image processing unit 81 may determine the first cutout image and the second cutout image based on the IP size without using the IP tilt angle. In this case, processing of rotating the cutout frame in response to the IP tilt angle is not necessary in the cutting-out processing. Display of the cutout image hereinafter includes display of the cutout image whose tilt has been corrected.

As described above, the image processing unit 81 sets the cutout range of the IP biological radiographically captured image being the image based on detection of the IP acted light from the biological radiographically captured image. The image in the portion corresponding to the imaging plate of the biological radiographically captured image can thereby properly be cut out.

### <Identification of Unexposed Region Image>

The image processing unit 81 may perform unexposure identification processing of identifying the unexposed region image of the first cutout image 151 or the unexposed region image of the light emission-time whole image. The unexposure identification processing may be performed during the series of processes shown in FIG. 18, or may be performed at a different time from the processing shown in FIG. 18. The unexposure identification processing may be performed during the series of processes shown in FIG. 27, or may be performed at a different time from the processing shown in FIG. 27. The image processing unit 81 functions as an identification unit (also referred to as an unexposed region image identification unit) that identifies the unexposed region image. Identification of the unexposed region image facilitates identification of a biological image region in the acquired image acquired by biological radiography.

When at least portion of the IP corresponding portion is set to the first cutout image 151 as in the examples of FIGs. 39, 45, and the like, the image processing unit 81 generates a binarized image acquired by binarizing the first cutout image 151 in the unexposure identification processing, for example. The first cutout image 151 cut out from the after-reversal light emission-time whole image 100b as in FIGs. 34 and 36 described above is herein referred to as an after-reversal first cutout image 151. The first cutout image 151 cut out from the before-reversal light emission-time whole image 100a as in FIGs. 45 and 48 described above is herein referred to as a before-reversal first cutout image 151.

A threshold used to binarize the before-reversal first cutout image 151 is set to a value smaller than a minimum luminance value for the radiograph included in the before-reversal first cutout image 151 and greater than a luminance value for the unexposed region image included in the before-reversal first cutout image 151, for example. Consider a case where IL4 is the minimum luminance value for the radiograph included in the before-reversal first cutout image 151, and IL2 is the luminance value for the unexposed region image included in the before-reversal first cutout image 151, for example. An inequality IL2 < IL4 holds. In this case, the threshold is set to IL50 that satisfies a relationship indicated by an inequality IL2 < IL5 < IL4, for example. A portion corresponding to the unexposed region image and a portion corresponding to the radiograph of a binarized image of the before-reversal first cutout image 151 being at least portion of the IP corresponding portion are thus respectively the low luminance region and the high luminance region. The image processing unit 81 can properly identify the unexposed region image of the before-reversal first cutout image 151 by identifying the low luminance region of the binarized image of the before-reversal first cutout image 151. When the imaging plate 10 does not include the unexposed portion, the binarized image of the before-reversal first cutout image 151 does not include the low luminance region.

A threshold used to binarize the after-reversal first cutout image 151 is set to a value greater than a maximum luminance value for the radiograph included in the after-reversal first cutout image 151 and smaller than a luminance value for the unexposed region image included in the after-reversal first cutout image 151, for example. A portion corresponding to the unexposed region image and a portion corresponding to the radiograph of a binarized image of the after-reversal first cutout image 151 being at least portion of the IP corresponding portion are thus respectively the high luminance region and the low luminance region. The image processing unit 81 can properly identify the unexposed region image of the after-reversal first cutout image 151 by identifying the high luminance region of the binarized image of the after-reversal first cutout image 151.

As described above, the image processing unit 81 can properly identify the unexposed region image of the first cutout image 151 when at least portion of the IP corresponding portion is cut out from the light emission-time whole image as the first cutout image 151.

In the unexposure identification processing, the image processing unit 81 may identify the unexposed region image of the first cutout image 151 including the IP image region outside region image. In this case, the image processing unit 81 may identify the unexposed region image of the first cutout image 151 including the first cutout image 151 included in the IP image region outside region image. When identifying the unexposed region image of the first cutout image 151 including the IP image region outside region image, the image processing unit 81 ternarizes the first cutout image 151 to generate a ternarized image.

The image processing unit 81 first compares each of luminance values of a plurality of pixels constituting the first cutout image 151 with a lower threshold and an upper threshold set in advance. The upper threshold is a value greater than the lower threshold. As for each of the luminance values of the plurality of pixels constituting the first cutout image 151, the image processing unit 81 replaces a luminance value smaller than the lower threshold with a first value, replaces a value equal to or greater than the lower threshold and smaller than the upper threshold with a second value, and replaces a luminance value equal to or greater than the upper threshold with a third value. Herein, the third value is greater than the second value, and the second value is greater than the first value. The first cutout image 151 is thereby ternarized to acquire the ternarized image. A region of the ternarized image where the luminance value is the third value is hereinafter also referred to as the high luminance region, a region of the ternarized image where the luminance value is the second value is hereinafter also referred to as a medium luminance region, and a region of the ternarized image where the luminance value is the first value is hereinafter also referred to as the low luminance region.

The lower threshold (also referred to as a first threshold) used to ternarize the before-reversal first cutout image 151 is set to a value greater than the luminance value for the IP image region outside region image included in the before-reversal first cutout image 151 and smaller than the luminance value for the unexposed region image included in the before-reversal first cutout image 151, for example. The upper threshold (also referred to as a second threshold) used to ternarize the before-reversal first cutout image 151 is set to a value greater than the luminance value for the unexposed region image included in the before-reversal first cutout image 151 and smaller than the minimum luminance value for the radiograph included in the before-reversal first cutout image 151, for example. Consider a case where the minimum luminance value for the radiograph included in the before-reversal first cutout image 151 is 10000, the luminance value for the unexposed region image included in the before-reversal first cutout image 151 is 3000, and the luminance value for the IP image region outside region image included in the before-reversal first cutout image 151 is 1000, for example. In this case, the lower threshold is set to 2000, and the upper threshold is set to 5000, for example. A portion corresponding to the IP image region outside region image, a portion corresponding to the unexposed region image, and a portion corresponding to the radiograph of a ternarized image of the before-reversal first cutout image 151 are thus respectively the low luminance region, the medium luminance region, and the high luminance region. The image processing unit 81 can properly identify the unexposed region image of the before-reversal first cutout image 151 by identifying the medium luminance region of the ternarized image of the before-reversal first cutout image 151. When the imaging plate 10 does not include the unexposed portion, the ternarized image of the before-reversal first cutout image 151 does not include the medium luminance region.

The lower threshold (also referred to as a first threshold) used to ternarize the after-reversal first cutout image 151 is set to a value greater than the maximum luminance value for the radiograph included in the after-reversal first cutout image 151 and smaller than the luminance value for the unexposed region image included in the after-reversal first cutout image 151, for example. The upper threshold (also referred to as a second threshold) used to ternarize the after-reversal first cutout image 151 is set to a value greater than the luminance value for the unexposed region image included in the after-reversal first cutout image 151 and smaller than the luminance value for the IP image region outside region image included in the after-reversal first cutout image 151, for example. A portion corresponding to the IP image region outside region image, a portion corresponding to the unexposed region image, and a portion corresponding to the radiograph of a ternarized image of the after-reversal first cutout image 151 are thus respectively the high luminance region, the medium luminance region, and the low luminance region. The image processing unit 81 can properly identify the unexposed region image of the after-reversal first cutout image 151 by identifying the medium luminance region of the ternarized image of the after-reversal first cutout image 151.

As described above, the image processing unit 81 can properly identify the unexposed region image of the first cutout image 151 including the IP image region outside region image.

In the unexposure identification processing, the image processing unit 81 may identify the unexposed region image of the light emission-time whole image. In this case, the image processing unit 81 ternarizes the light emission-time whole image to generate a ternarized image as in a case where the unexposed region image of the first cutout image 151 including the IP image region outside region image is identified. A lower threshold and an upper threshold used to ternarize the before-reversal light emission-time whole image 100a are set similarly to the lower threshold and the upper threshold used to ternarize the before-reversal first cutout image 151. The image processing unit 81 can properly identify the unexposed region image of the before-reversal light emission-time whole image 100a by identifying the medium luminance region of the ternarized image of the before-reversal light emission-time whole image 100a. A lower threshold and an upper threshold used to ternarize the after-reversal light emission-time whole image 100b are set similarly to the lower threshold and the upper threshold used to ternarize the after-reversal first cutout image 151. The image processing unit 81 can properly identify the unexposed region image of the after-reversal light emission-time whole image 100b by identifying the medium luminance region of the ternarized image of the after-reversal light emission-time whole image 100b.

When the image processing unit 81 identifies the unexposed region image of the first cutout image 151 or the light emission-time whole image, the display control unit 82 may cause the display 3 to display unexposure notification information 161 to provide notification that the unexposed region image is present in the first cutout image 151 or the light emission-time whole image. The user can thus easily recognize that the unexposed region image is present in the first cutout image 151 or the light emission-time whole image. The user can thus easily recognize that the imaging plate 10 includes the unexposed portion.

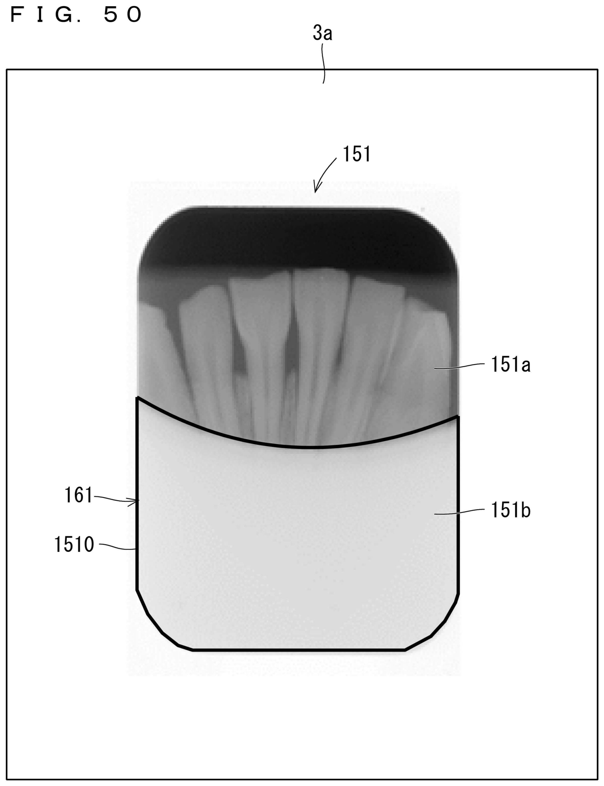

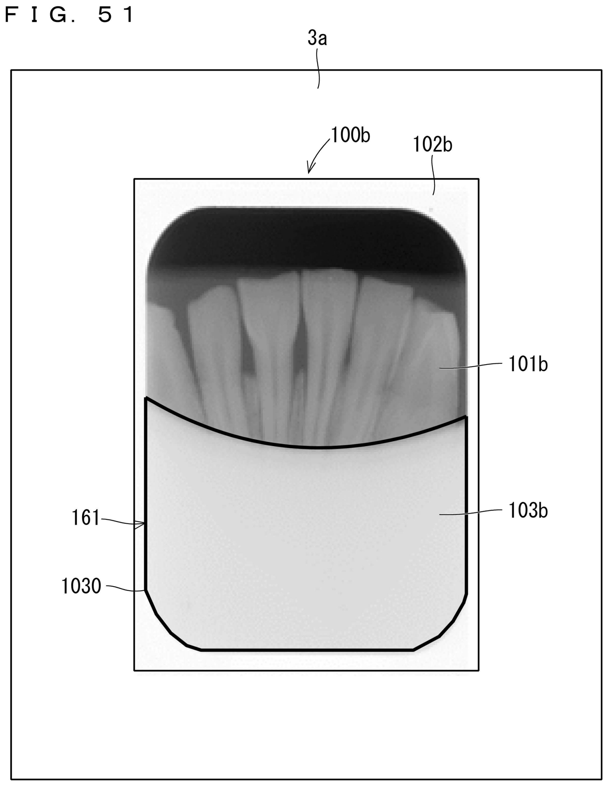

FIGs. 50 and 51 are schematic diagrams each showing an example of display of the unexposure notification information 161. In the example of FIG. 50, the first cutout image 151 including the radiograph 151a and the unexposed region image 151b and the unexposure notification information 161 are displayed on the display surface 3a of the display 3. In the example of FIG. 51, the after-reversal light emission-time whole image 100b and the unexposure notification information 161 are displayed on the display surface 3a. In the example of FIG. 50, a border line 1510 that borders a range of the unexposed region image 151b is displayed as the unexposure notification information 161 to provide notification that the unexposed region image 151b is present in the first cutout image 151. Similarly, in the example of FIG. 51, a border line 1030 that borders a range of the unexposed region image 103b is displayed as the unexposure notification information 161 to provide notification that the unexposed region image 103b is present in the after-reversal light emission-time whole image 100b. The border lines 1510 and 1030 may each be displayed in at least one color.

The unexposure notification information 161 is not limited to that in the examples of FIGs. 50 and 51. For example, hatching lines, such as diagonal lines, attached to the unexposed region image may be displayed as the unexposure notification information 161. The unexposed region image may be displayed in at least one color to display the unexposure notification information 161.

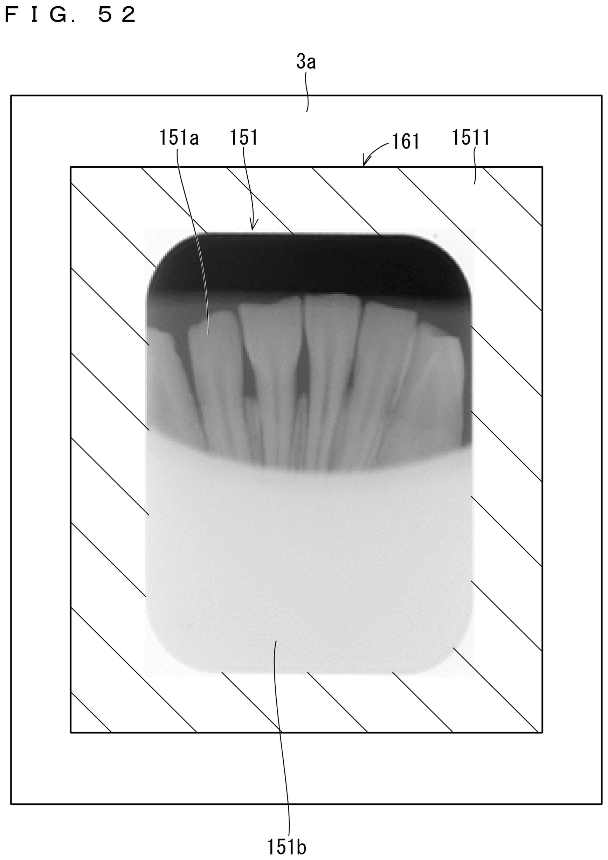

As shown in FIG. 52, a frame-like graphic 1511 surrounding the first cutout image 151 may be displayed as the unexposure notification information 161. While the frame-like graphic 1511 is hatched in the example of FIG. 52 for the convenience of description, the frame-like graphic 1511 may not be hatched or may be hatched. The frame-like graphic 1511 may be displayed in at least one color. Similarly, when the unexposed region image is identified in the light emission-time whole image, a frame-like graphic surrounding the light emission-time whole image may be displayed as the unexposure notification information 161. The unexposure notification information 161 may be displayed in at least one of characters and a symbol.

When the first cutout image 151 or the light emission-time whole image does not include the unexposed region image, that is, the image processing unit 81 does not identify the unexposed region image of the first cutout image 151 or the light emission-time whole image, the display control unit 82 may cause the display 3 to display notification information 162 to provide notification that the unexposed region image is not present in the first cutout image 151 or the light emission-time whole image. The user can thus easily recognize that the imaging plate 10 does not include the unexposed portion.

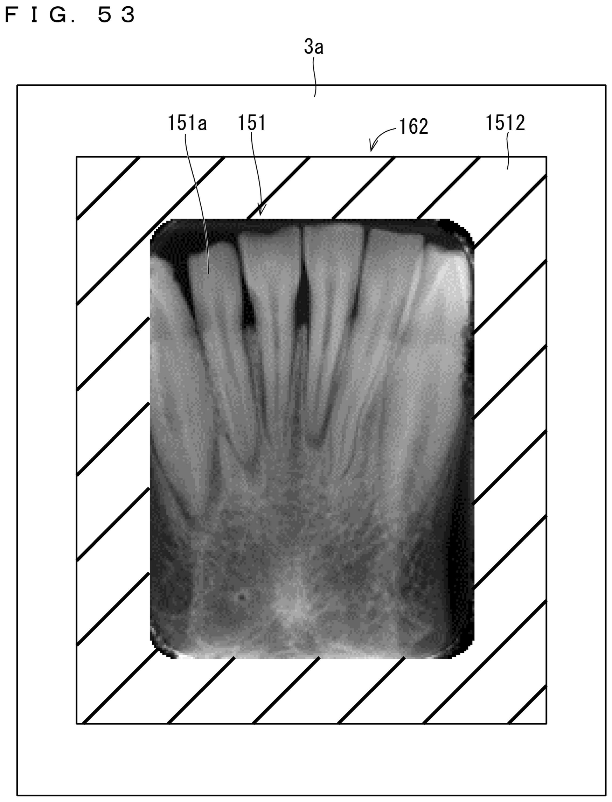

FIG. 53 is a schematic diagram showing an example of display of the notification information 162. FIG. 53 shows an example of display of the notification information 162 to provide notification that the unexposed region image is not present in the first cutout image 151. In the example of FIG. 53, a frame-like graphic 1512 surrounding the first cutout image 151 not including the unexposed region image is displayed as the notification information 162. While the frame-like graphic 1512 is hatched in the example of FIG. 53 for the convenience of description, the frame-like graphic 1512 may not be hatched or may be hatched. The frame-like graphic 1512 may be displayed in at least one color. When the frame-like graphic 1511 in FIG. 52 is displayed as the unexposure notification information 161, the frame-like graphic 1511 as the unexposure notification information 161 and the frame-like graphic 1512 as the notification information 162 are displayed in different ways. In this case, the frame-like graphic 1511 and the frame-like graphic 1512 may be displayed in different colors. The frame-like graphic 1511 and the frame-like graphic 1512 may be hatched in different ways. Similarly, when the unexposed region image is not present in the light emission-time whole image, a frame-like graphic surrounding the light emission-time whole image may be displayed as the notification information 162. The notification information 162 may be displayed in at least one of characters and a symbol. When the unexposed region image is present, notification of the presence of the unexposed region image is displayed in a different way from that in a case where the frame-like graphic 1512 is displayed, and thus it can be said that not providing notification of the presence of the unexposed region image means providing notification of the absence of the unexposed region image.

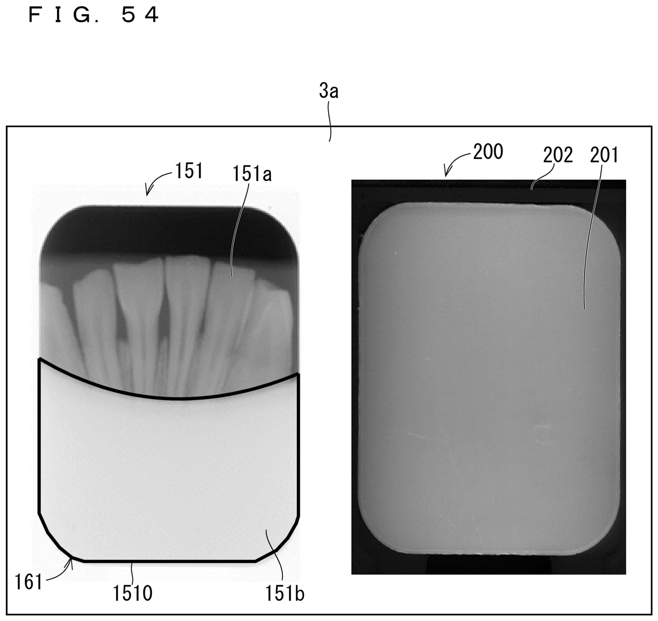

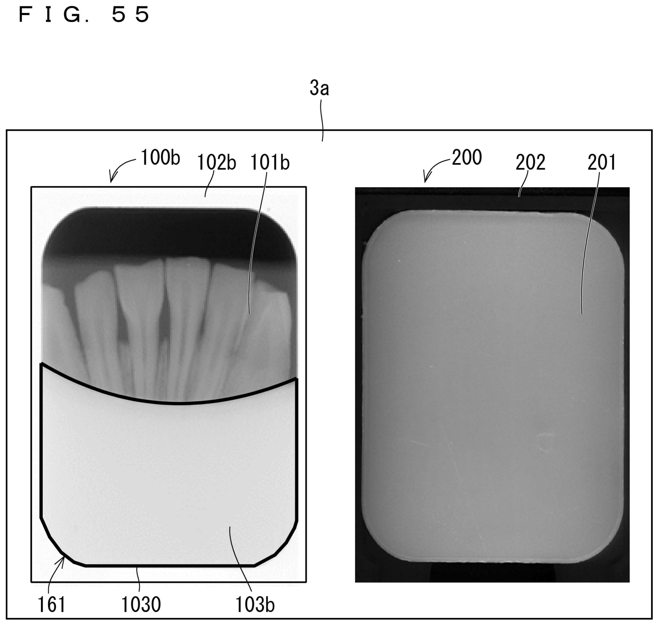

When the display control unit 82 causes the display 3 to simultaneously and separately display the first cutout image 151 and the erasing-time whole image 200, and the first cutout image 151 includes the unexposed region image as shown in FIG. 37 described above, the display control unit 82 may cause the display 3 to display the unexposure notification information 161 together as shown in FIG. 54. In this case, the second cutout image may be displayed in place of the erasing-time whole image 200. When the display control unit 82 causes the display 3 to simultaneously and separately display the light emission-time whole image and the erasing-time whole image 200, and the light emission-time whole image includes the unexposed region image as shown in FIG. 30 described above, the display control unit 82 may cause the display 3 to display the unexposure notification information 161 together as shown in FIG. 55. In this case, the second cutout image may be displayed in place of the erasing-time whole image 200.

When the display control unit 82 causes the display 3 to simultaneously and separately display the first cutout image 151 not including the unexposed region image and the erasing-time whole image 200, the display control unit 82 may cause the display 3 to display the notification information 162 together. In this case, the second cutout image may be displayed in place of the erasing-time whole image 200. Similarly, when the display control unit 82 causes the display 3 to simultaneously and separately display the light emission-time whole image not including the unexposed region image and the erasing-time whole image 200, the display control unit 82 may cause the display 3 to display the notification information 162 together. In this case, the second cutout image may be displayed in place of the erasing-time whole image 200.

As described above, the detector 40 according to this example can detect not only the emitted light L2 from the imaging plate 10 but also the reflected light of the excitation light L10 from the imaging plate 10 to some extent. The reading apparatus 1 can thus acquire the radiograph based on detection of the emitted light L2 and the reflected light image (e.g., the unexposed region image) based on detection of the reflected light. Usability of the reading apparatus 1 is thereby improved.

For example, the reading apparatus 1 can properly identify the IP size, and properly identify the IP tilt angle as described above based on the radiograph based on detection of the emitted light L2 from the imaging plate 10 and the light emission-time whole image including the reflected light image based on detection of the reflected light from the imaging plate 10.

For example, the reading apparatus 1 simultaneously and separately displays the radiograph based on detection of the emitted light L2 from the imaging plate 10 and the reflected light image based on detection of the reflected light from the imaging plate 10 as shown in FIGs. 29, 30, 37, 54, 55, and the like described above, so that the user can easily compare the radiograph read from the imaging plate 10 and the appearance of the imaging plate 10. The user can thus easily identify the unexposed portion that can be included in the imaging plate 10, for example.

### <Identification of Abnormality of Surface of Imaging Plate>

When the IP whole reflected light image as the reflected light image based on detection of the reflected light from the imaging plate 10 is displayed as shown in FIGs. 28 to 30, 54, 55, and the like described above, the user can easily identify the presence or absence of any abnormality of the surface of the imaging plate 10, for example. The abnormality of the surface of the imaging plate 10 includes a scratch, a depression, a chip, contamination, and adhesion of foreign matter, for example.

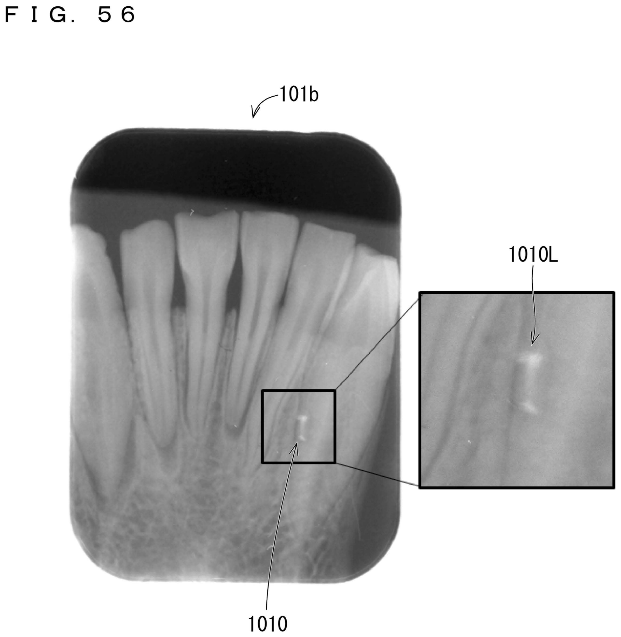

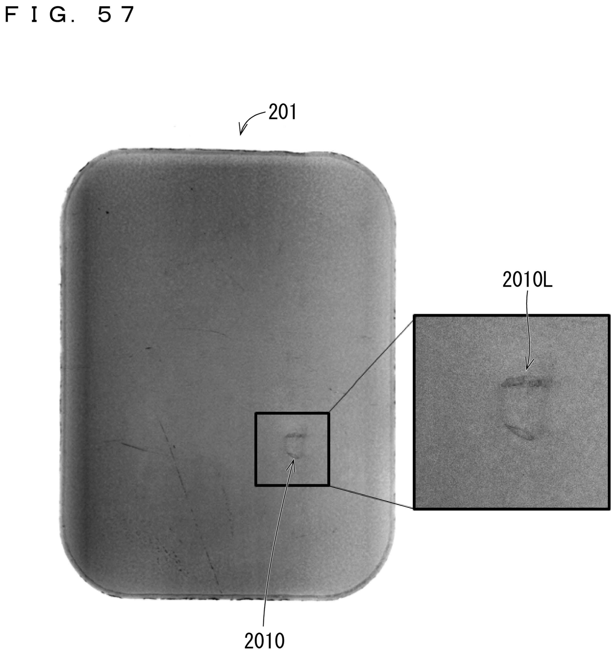

FIGs. 56 and 57 are schematic diagrams respectively showing one example of the radiograph 101b included in the after-reversal light emission-time whole image 100b acquired by the reading apparatus 1 and one example of the IP whole reflected light image 201 included in the before-reversal erasing-time whole image 200 acquired by the reading apparatus 1 when the front surface of the imaging plate 10 has an abnormality. The before-reversal erasing-time whole image 200 is used as the erasing-time whole image in the shown examples. FIG. 56 also shows an enlarged view 1010L of an abnormal region image 1010 in which the abnormality of the surface of the imaging plate 10 appears in the radiograph 101b. FIG. 57 also shows an enlarged view 2010L of an abnormal region image 2010 in which the abnormality of the surface of the imaging plate 10 appears in the IP whole reflected light image 201. FIGs. 56 and 57 respectively show the radiograph 101b and the IP whole reflected light image 201 when the front surface of the imaging plate 10 has a scratch.

As shown in FIGs. 56 and 57, a luminance value for the abnormal region image 1010 in which the abnormality of the surface of the imaging plate 10 appears is sometimes different from a luminance value for a portion around the abnormal region image 1010 in each of the radiograph 101b and the IP whole reflected light image 201. On the other hand, when any lesion, such as a carious lesion, occurs in teeth as the imaging object, a luminance value for a region in which a portion of the teeth where the lesion occurs is sometimes different from a luminance value for a portion around the region in the radiograph 101b. Furthermore, in contrast to the example of FIG. 56, the luminance value for the abnormal region image 1010 can be close to the luminance value for a region in which the teeth appear in the radiograph 101b. In view of these points, it is not easy for the user to identify the abnormality of the surface of the imaging plate 10 based on the radiograph 101b displayed by the display 3.

In contrast, the teeth do not appear in the IP whole reflected light image 201 of the imaging plate 10 in which the radiograph is not stored as the latent image. The user can thus easily identify the abnormality of the surface of the imaging plate 10 based on the IP whole reflected light image 201 displayed by the display 3. That is to say, the user can easily identify the abnormality of the surface of the imaging plate 10 by recognizing the abnormal region image 1010 included in the IP whole reflected light image 201 displayed by the display 3. The user may identify the abnormality of the surface of the imaging plate 10 based on the erasing-time whole image 200 including the IP whole reflected light image 201 displayed by the display 3. The user may also identify the abnormality of the surface of the imaging plate 10 based on the second cutout image including at least portion of the IP whole reflected light image 201 displayed by the display 3.

When the display 3 being controlled by the display control unit 82 simultaneously and separately displays the radiograph and the IP whole reflected light image, the user can easily identify a region corresponding to the abnormality of the surface of the imaging plate 10 in the radiograph. Thus, when a dentist is the user, for example, the dentist can easily exclude the region corresponding to the abnormality of the surface of the imaging plate 10 in the radiograph from information to make diagnostic determination.

The light source 30 functions as a first light source which irradiates the imaging plate 10 with the excitation light L10. The detector 40 functions as a first detector that detects the emitted light L2 caused by the excitation light L10 from the imaging plate 10, and outputs a first image signal as a result of detection of the emitted light L2.

The construction of the present embodiment irradiates the imaging plate 10 with light as the acting light L1 in order to generate reflected light L4 from the imaging plate 10. The acting light L1 can be thought as to be irradiation light L1. The light source 30 such as a light source which can also irradiate the excitation light L10 acting as the irradiation light L1 to generate reflected light L4 can be used. In this case, the light source 30 as the first light source functions also as the second light source. The first light source and the second light source can comprise separate bodies as described later.

The detector 40 such as a detector which can also detect the reflection light L4 of the irradiation light L1 can be used. In this case, the detector 40 as the first detector functions also as the second detector which outputs the second image signal as a result of detection of the reflected light L4. The first detector and the second detector can comprise separate bodies as described later. In above case, the optical filter 42 such as a filter of which transmittance of the emitted light L2 and the reflected light L4 is high and transmittance of other light is low can be preferably used.

After the light source 30 as the first light source irradiates the imaging plate 10 with the excitation light L10 and the detector 40 as the first detector detects the emitted light L2, the light source 30 as the second light source can irradiate the excitation light L10 as the irradiation light L1 and the detector 40 as the second detector can detect the reflected light L4. And also between the detection of the emitted light L2 and the detection of the reflected light L4, an irradiation of the erasing light L3 can be inserted. Or, the imaging plate 10 which does not store the radiograph as the latent image before X-ray exposure can be irradiated by the excitation light L10 as the irradiation light L1 to detect the reflected light L4 to secure the second image signal in advance.

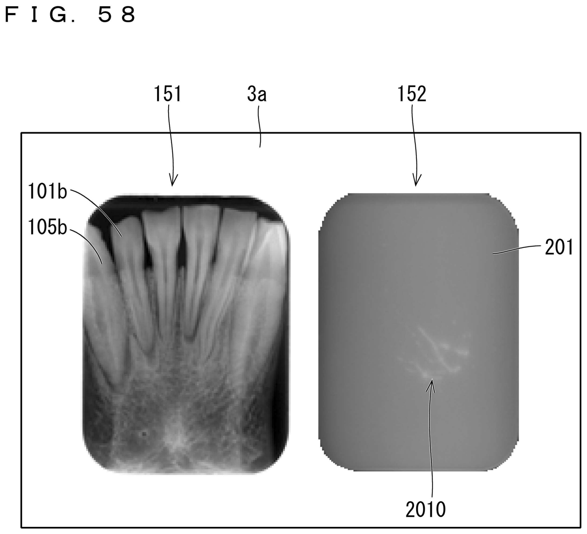

FIG. 58 is a schematic diagram showing an example of display of the first cutout image 151 and the second cutout image 152 of the imaging plate 10 having the front surface having the abnormality. In the example of FIG. 58, the IP corresponding portion 105b of the after-reversal light emission-time whole image 100b is set to the first cutout image 151, and the erased IP reflected light image (i.e., the IP corresponding portion) 201 of the before-reversal erasing-time whole image 200 is set to the second cutout image 152. The second cutout image 152 includes the abnormal region image 2010 in which the abnormality of the surface of the imaging plate 10 appears.

The first cutout image 151 is an example of an image that contains a radiograph generated from the first image signal. The radiograph can occupy the whole image area. The second cutout image 152 is an example of an image that contains an IP reflected light image generated from the second image signal. The IP reflected light image can occupy the whole image area.

The abnormality of the surface of the imaging plate 10 is identified by generation of IP reflected light image 201 by the image processing unit 81. Hereby the image processing unit 81 can identify the position and the shape of the abnormal region image 2010 in the IP reflected light image 201. Herein the generation of IP reflected light image 201 causes the identification by exaggeration.

As shown in FIG. 56, one of the first cutout image 151 and the second cutout image 152 can be placed next to the other. This placement makes it easy to compare both. The row of the placement next to each other can be left right, or up down, or other row.

The image processing unit 81 which executes processing of generation of the IP reflected light image 201 can be regarded as an identification unit. Or the image processing unit 81 and the display control unit 82 executing processing of generation of the IP reflected light image and of the placement next to each other can be regarded as an identification unit. In this case, the generation of the IP reflected light image 201 and of the placement next to each other can be thought to be identification of the position and the shape of the abnormal region image 2010 in the radiograph.

The display of one of the first cutout image 151 and the second cutout image 152 next to the other is one example of abnormal region display which is a display of the position and the shape of the abnormal region against a radiograph generated by processing of the first image signal.

When display as shown in FIG. 58 is performed, the user identifies the abnormal region image 2010 in the erased IP reflected light image 201 included in the displayed second cutout image 152, for example. The user identifies the region corresponding to the abnormality of the imaging plate 10 in the radiograph 101b included in the first cutout image 151 based on the abnormal region image 2010 identified in the erased IP reflected light image 201.

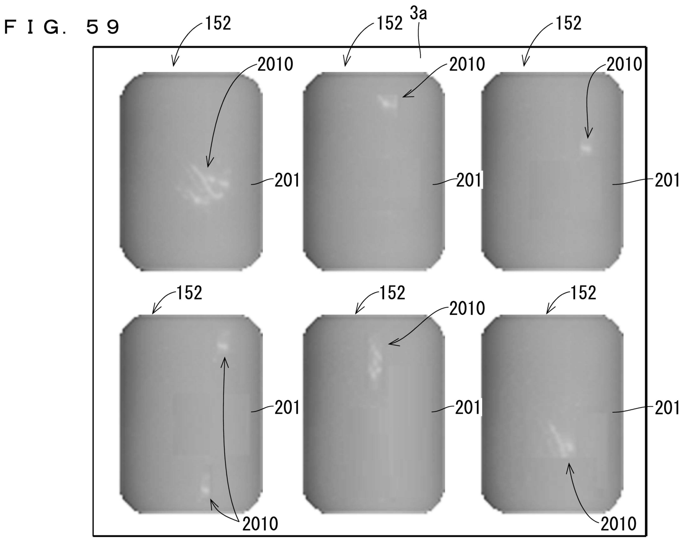

The display control unit 82 may cause the display 3 to simultaneously and separately display IP whole reflected light images of a plurality of imaging plates 10. FIG. 59 is a schematic diagram showing one example of such display. In the example of FIG. 59, a plurality of second cutout images 152 in which front surfaces of the respective imaging plates 10 appear are displayed on the display surface 3a. In the example of FIG. 59, the IP whole reflected light images 201 of before-reversal erasing-time whole images 200 are set to the second cutout images 152. As shown in FIG. 59, the IP whole reflected light images 201 in which the front surfaces of the respective imaging plates 10 appear are simultaneously and separately displayed, so that the user can easily identify, from among the imaging plates 10, an imaging plate 10 having the abnormality in a relatively wide range. The user may discard the imaging plate 10 having the abnormality in the wide range, for example. A plurality of erasing-time whole images in which the front surfaces of the respective imaging plates 10 appear may simultaneously and separately be displayed by the display 3.

When the operation unit 4 receives a predetermined operation from the user, the display control unit 82 may cause the display 3 to display a plurality of IP whole reflected light images 201 acquired in the past as shown in FIG. 59. When the operation unit 4 receives a predetermined operation from the user, the reading apparatus 1 may set an operation mode of the reading apparatus 1 to a quality check mode to check the quality of the imaging plates 10, and the IP whole reflected light images 201 in which the respective imaging plates 10 appear may be acquired and displayed as shown in FIG. 59 in the quality check mode. The imaging plates 10 are sequentially inserted into the reading apparatus 1 in the quality check mode by the user. When a single imaging plate 10 is inserted through the inlet 2a, the reading apparatus 1 in the quality check mode performs steps s3, s4, s21, and s22 in FIG. 27 described above to acquire the erasing-time whole image in which a front surface of the inserted imaging plate 10 appears, for example. The reading apparatus 1 performs steps s5 and s6 in FIG. 27 to discharge the processed imaging plate 10. When the processed imaging plate 10 is discharged from the reading apparatus 1, a next imaging plate 10 is inserted into the reading apparatus 1. The reading apparatus 1 in the quality check mode similarly operates to acquire an erasing-time whole image in which a front surface of the next imaging plate 10 appears, and discharges the processed imaging plate 10. Insertion of the imaging plate 10 into the reading apparatus 1 and discharge of the imaging plate 10 from the reading apparatus 1 are hereinafter similarly repeated, so that the reading apparatus 1 in the quality check mode acquires the erasing-time whole images in which the respective imaging plates 10 appear. The display control unit 82 then causes the display 3 to perform display as shown in FIG. 59 based on the acquired erasing-time whole images. The user identifies and discards the imaging plate 10 having the abnormality in the wide range based on display in FIG. 59, for example.

While the user identifies the abnormality of the surface of the imaging plate 10 based on the IP reflected light image displayed by the display 3 in the above-mentioned example, the image processing unit 81 may perform abnormality identification processing of identifying the abnormality of the surface of the imaging plate 10. In this case, the image processing unit 81 functions as an identification unit (also referred to as an abnormality identification unit) that identifies the abnormality of the surface of the imaging plate 10.

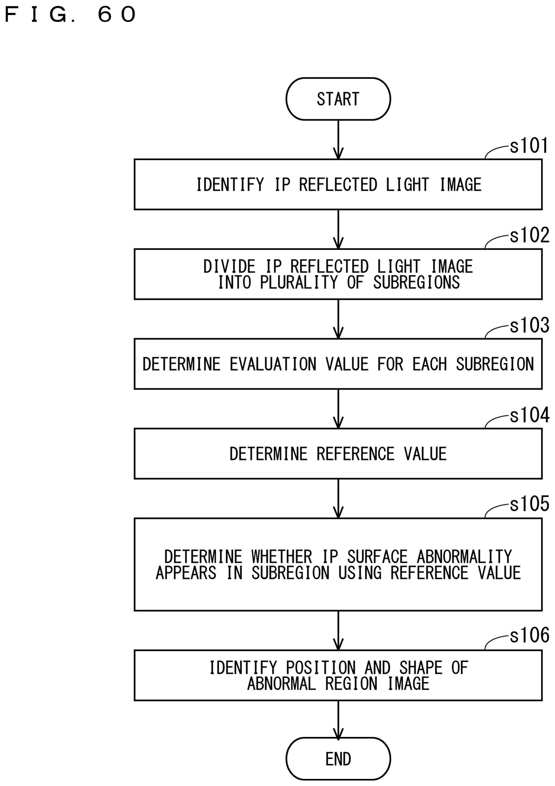

FIG. 60 is a flowchart showing one example of the abnormality identification processing. The image processing unit 81 identifies a position and a shape of the abnormal region image of the erasing-time whole image based on the erasing-time image signal to identify the abnormality of the surface of the imaging plate 10, for example. The abnormality of the surface of the imaging plate 10 is hereinafter also referred to as an IP surface abnormality.

As shown in FIG. 60, in step s101, the image processing unit 81 identifies the IP reflected light image (i.e., the IP corresponding portion) of the erasing-time whole image based on the erasing-time image signal acquired in the above-mentioned step s22. The image processing unit 81 identifies the IP reflected light image of the before-reversal erasing-time whole image, for example. As described above, the image processing unit 81 can determine the IP reflected light image of the erasing-time whole image as the second cutout image, and thus can similarly identify the IP reflected light image of the before-reversal erasing-time whole image.

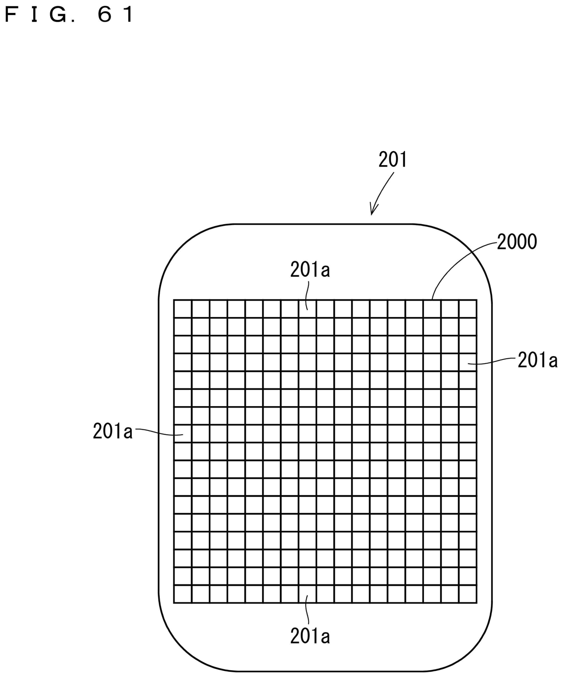

Next, in step s102, the image processing unit 81 divides the IP reflected light image identified in step s101 into a plurality of subregions. FIG. 61 is a schematic diagram showing one example of dividing the IP reflected light image 201 into a plurality of subregions 201a. When the imaging plate 10 is in clinical use, only a region of a portion of the radiograph formation layer 11 of the imaging plate 10 is used in some cases. The region is referred to as a usage target region. In step s102, the image processing unit 81 divides an identified region 2000 of the IP reflected light image 201 corresponding to the usage target region of the imaging plate 10 into the plurality of subregions 201a, for example. In this case, the image processing unit 81 is to identify the abnormal region image 2010 in the identified region 2000 of the IP reflected light image 201. Each of the subregions 201a is composed of a plurality of pixels, for example. Each of the subregions 201a is square, for example.

As shown in FIG. 61, the image processing unit 81 divides the identified region 2000 into the subregions 201a in a matrix, for example. Assume herein that a transverse direction and a longitudinal direction of the IP reflected light image 201 are respectively a row direction and a column direction. The image processing unit 81 divides the identified region 2000 in a matrix so that P subregions 201a are arranged in the row direction and Q subregions 201a are arranged in the column direction. FIG. 61 shows an example of a case where an equation P = Q = 17 holds. The image processing unit 81 may divide the whole IP reflected light image 201 into the subregions 201a.

Next, in step s103, the image processing unit 81 determines, for each of the subregions 201a, an evaluation value representing the possibility that the IP surface abnormality appears in the subregion 201a. It can be said that the evaluation value is a value representing the possibility that the subregion 201a is an image in a region of the imaging plate 10 having any abnormality.

In step s103, the image processing unit 81 determines an average value and a standard deviation of luminance values of a plurality of pixels constituting the subregion 201a, for example. The image processing unit 81 determines a value obtained by dividing the average value by the standard deviation as the evaluation value. The image processing unit 81 determines the evaluation value for each of the subregions 201a as described above. In this example, as the evaluation value increases, the possibility that the IP surface abnormality appears in the subregion 201a corresponding to the evaluation value increases. A method of determining the evaluation value is not limited to this method.

Next, in step s104, the image processing unit 81 determines a reference value to be compared with the evaluation value for each of the subregions 201a. The image processing unit 81 determines an average value of evaluation values determined for the respective subregions 201a in step s103 as the reference value, for example. A method of determining the reference value is not limited to this method.

Next, in step s105, the image processing unit 81 performs, for each of the subregions 201a, determination processing of determining whether the IP surface abnormality appears in the subregion 201a using the reference value. In the determination processing, the image processing unit 81 compares the reference value and the evaluation value for each of the subregions 201a, for example. When the evaluation value for the subregion 201a exceeds the reference value (e.g., the evaluation value is smaller than the reference value), the image processing unit 81 determines that the IP surface abnormality appears in the subregion 201a. On the other hand, when the evaluation value for the subregion 201a does not exceed the reference value (e.g., the evaluation value is equal to or greater than the reference value), the image processing unit 81 determines that the IP surface abnormality does not appear in the subregion 201a. The subregion 201a determined as the subregion 201a in which the IP surface abnormality appears is hereinafter referred to as an abnormal subregion 201a. When the surface of the imaging plate 10 has any abnormality, at least one abnormal subregion 201a is identified in step s105.

Next, in step s106, the image processing unit 81 identifies a position and a shape of the abnormal region image 2010 (see FIGs. 55 to 57) in which the IP surface abnormality appears in the IP reflected light image 201 of the before-reversal erasing-time whole image 200 based on a result of the determination processing performed for each of the subregions 201a in step s105. The image processing unit 81 sets an image in a partial region composed of at least one abnormal subregion 201a identified in step s105 in the IP reflected light image 201 to the abnormal region image 2010. The image processing unit 801 sets a position and a shape of the partial region composed of the at least one abnormal subregion 201a identified in step s105 to the position and the shape of the abnormal region image 2010. The image processing unit 801 can identify the position and the shape of the abnormal region image 2010 based on a position and a shape of each abnormal subregion 201a. When the position and the shape of the abnormal region image 2010 are identified, the abnormality identification processing ends. Identification of the position and the shape of the abnormal region image 2010 can be regarded as identification by extraction. The position and the shape of the abnormal region image 2010 identified by the image processing unit 81 are hereinafter also referred to as an abnormal region image position and an abnormal region image shape.

When it is determined that the IP surface abnormality appears in none of the subregions 201a in step s105, the abnormality identification processing ends without performing step s106. That is to say, when it is determined that the IP surface abnormality does not appear in the identified region 2000 of the IP reflected light image 201, the abnormality identification processing ends without performing step s106.

As described above, the image processing unit 81 can properly identify any abnormality of the surface of the imaging plate 10 based on the image signal as a result of detection of the reflected light of the excitation light L10 from the imaging plate 10.

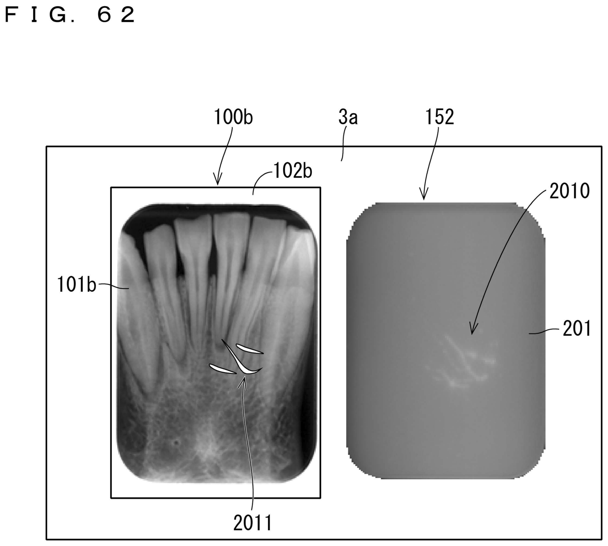

When the display control unit 82 causes the display 3 to display the detected radiograph, the abnormal region image shape may be superimposed on the detected radiograph at a position of the detected radiograph corresponding to the abnormal region image position. FIG. 62 is a schematic diagram showing an example of display of the display 3 in this case. The superimpose display is one example of abnormal region display which is a display of the position and the shape of the abnormal region against a radiograph generated by processing of the first image signal.

In the example of FIG. 62, the after-reversal light emission-time whole image 100b including the radiograph 101b and the second cutout image 152 cut out from the before-reversal erasing-time whole image 200 are simultaneously and separately displayed on the display surface 3a. In the example of FIG. 62, the IP reflected light image 201 of the before-reversal erasing-time whole image 200 is set to the second cutout image 152, and the second cutout image 152 includes the abnormal region image 2010. In the example of FIG. 62, the abnormal region image shape (also referred to as an abnormal region image shape image) 2011 is superimposed on the radiograph 101b at the position of the radiograph 101b corresponding to the abnormal region image position. A relative position of the abnormal region image 2010 in the IP reflected light image 201 and a relative position of the abnormal region image shape 2011 in the radiograph 101b are the same.

When the abnormal region image shape 2011 is superimposed on the radiograph 101b at the position of the radiograph 101b corresponding to the abnormal region image position as in the example of FIG. 62, the user can easily identify a region corresponding to the abnormality of the surface of the imaging plate 10 in the radiograph 101b. Thus, when the dentist is the user, for example, the dentist can easily exclude the region corresponding to the abnormality of the surface of the imaging plate 10 in the radiograph 101b from the information to make diagnostic determination.

In a case where the abnormal region image shape 2011 is displayed on the radiograph 101b when the IP reflected light image 201 is displayed as in the example of FIG. 62, the user can easily compare the region corresponding to the abnormality of the surface of the imaging plate 10 in the radiograph 101b and the abnormal region image 2010 in the IP reflected light image 201. The user can thus determine whether the position and the shape of the abnormal region image 2010 are properly identified by the reading apparatus 1, for example. The IP reflected light image 201 may not be displayed when the abnormal region image shape 2011 is superimposed on the radiograph 101b.

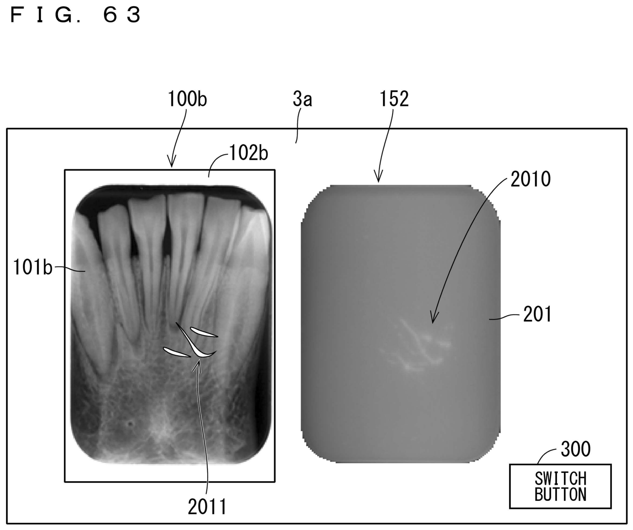

The reading apparatus 1 may switch between display and hiding of the abnormal region image shape 2011 in response to instructions from the user. In this case, the display 3 may display a switch button 300 to switch between display and hiding of the abnormal region image shape 2011. FIG. 63 is a schematic diagram showing an example of display of the display 3 in this case.

In the example of FIG. 63, a touch sensor included in the operation unit 4 and the display 3 constitute a touch panel display. The switch button 300 is a software button, and the operation unit 4 can receive a touch operation of the user on the switch button 300. Each time the touch operation is performed on the switch button 300, switching between display and hiding of the abnormal region image shape 2011 is made. When the operation unit 4 receives the user operation on the switch button 300 in a state of the abnormal region image shape 2011 being displayed as in FIG. 63, the abnormal region image shape 2011 is hidden. On the other hand, when the operation unit 4 receives the user operation on the switch button 300 in a state of the abnormal region image shape 2011 being hidden, the abnormal region image shape 2011 is displayed as in FIG. 63.

As described above, when switching between display and hiding of the abnormal region image shape 2011 is made in response to the instructions from the user, usability of the reading apparatus 1 is improved.

In the abnormality identification processing, only the presence or absence of any abnormality of the surface of the imaging plate 10 may be identified, and the position and the shape of the abnormal region image 2010 may not be identified.

As described above, the reading apparatus 1 can acquire the radiograph based on detection of the emitted light L2 and the reflected light image based on detection of the reflected light, so that usability of the reading apparatus 1 is improved.

Luminance information (i.e., a luminance value) for the abnormal subregion 201a may be used to correct the detected radiograph. After identifying the position and the shape of the abnormal region image 2010 in step s106, the image processing unit 81 acquires, from the image signal from the detector 40, luminance information (i.e., a luminance value) for the abnormal region image 2010 and luminance information for an image outside the abnormal region image 2010 of the second cutout image 152. The image processing unit 81 uses the absolute value of a difference between the luminance information for the image outside the abnormal region image 2010 and the luminance information for the abnormal region image 2010 as a correction value when the detected radiograph is corrected. For example, when a luminance value for a region (an abnormality corresponding region) corresponding to the abnormality of the surface of the imaging plate 10 falls below a luminance value for the other region in the detected radiograph, the image processing unit 81 adds the correction value to the luminance value for the abnormality corresponding region. On the other hand, when the luminance value for the abnormality corresponding region exceeds the luminance value for the other region in the detected radiograph, the image processing unit 81 subtracts the correction value from the luminance value for the abnormality corresponding region. Such adjustment of the luminance information is referred to as abnormality corresponding region luminance adjustment processing. A proper radiograph can be acquired by the abnormality corresponding region luminance adjustment processing. A portion of the detected radiograph on which the abnormal region image shape 2011 is superimposed is the abnormality corresponding region.

### <Identification of Reverse Setting of IP>

When the IP reflected light image is displayed, the user can identify reverse setting of the imaging plate 10 due to unusual insertion of the imaging plate 10 into the reading apparatus 1 with the back surface thereof facing forward based on display of the IP reflected light image, for example. As described above, the surface of the imaging plate 10 on a side of the radiograph formation layer 11 is the front surface, and the surface of the imaging plate 10 opposite the front surface not on a side of the radiograph formation layer 11 is the back surface. While the user basically inserts the imaging plate 10 into the reading apparatus 1 with the front surface thereof facing forward in this example, the user sometimes inserts the imaging plate 10 into the reading apparatus 1 with the back surface thereof facing forward by mistake. When the imaging plate 10 is set properly by being inserted with the front surface thereof facing forward, the support plate 21 of the holder 20 supports the back surface of the imaging plate 10 as described above. The front surface of the imaging plate 10, in other words, the radiograph formation layer 11 of the imaging plate 10 is thus properly irradiated with the excitation light L10 as described above. On the other hand, when the imaging plate 10 is set in reverse by being inserted with the back surface thereof facing forward by mistake, the support plate 21 supports the front surface of the imaging plate 10, that is, the radiograph formation layer 11 of the imaging plate 10. The back surface of the imaging plate 10 is thus irradiated with the excitation light L10. The detector 40 detects the reflected light of the excitation light L10 from the back surface of the imaging plate 10 and the IP outside region R130, and outputs an image signal as a result of detection.

An image signal as a result of detection of light from the imaging plate 10 when the imaging plate 10 is set in reverse by being inserted into the reading apparatus 1 with the back surface thereof facing forward is hereinafter also referred to as a back insertion-time image signal. In the present embodiment, the back insertion-time image signal is acquired as a result of detection of the reflected light from the imaging plate 10. A whole image based on the back insertion-time image signal is referred to as a back insertion-time whole image. The back insertion-time whole image does not include the radiograph, and includes the IP reflected light image in which the back surface of the imaging plate 10 appears and the IP outside region image, for example. Support of the front surface of the imaging plate 10 by the support plate 21 is also referred to as front surface support, and support of the back surface of the imaging plate 10 by the support plate 21 is also referred to as back surface support. The front surface support is backward setting of the imaging plate 10, and the back surface support is forward setting of the imaging plate 10. An act of backward setting of the imaging plate 10 is referred to as back insertion, and an act of forward setting of the imaging plate 10 is referred to as front insertion.

Assume that the imaging plate 10 is always set properly by being inserted into the reading apparatus 1 with the front surface thereof facing forward in description made above. That is to say, assume that the imaging plate 10 is always subjected to the back surface support in description made above. Thus, in description made above on processing shown in FIG. 18, the light emission-time whole image including the radiograph is acquired in step s2, and the light emission-time whole image including the radiograph is displayed in step s7. When the imaging plate 10 is inserted into the reading apparatus 1 with the back surface thereof facing forward, however, the acquired whole image acquired in step s2, in other words, the whole image based on the image signal output from the detector 40 in step s2 is the back insertion-time whole image in which the back surface of the imaging plate 10 appears. That is to say, not the light emission-time whole image including the radiograph but the back insertion-time whole image not including the radiograph is acquired in step s2. Thus, when the imaging plate 10 is inserted into the reading apparatus 1 with the back surface thereof facing forward, the back insertion-time whole image is displayed in step s7. In the reading processing in step s2, the radiograph is read from the imaging plate 10 when the imaging plate 10 is set properly, and the back insertion-time whole image in which the back surface of the imaging plate 10 appears is acquired when the imaging plate 10 is set in reverse.

Similarly, when the imaging plate 10 is inserted into the reading apparatus 1 with the back surface thereof facing forward, the back insertion-time whole image is acquired in step s2, and the erasing-time whole image acquired in step s22 is the back insertion-time whole image in the above-mentioned processing in FIG. 27. Thus, when the imaging plate 10 is inserted into the reading apparatus 1 with the back surface thereof facing forward, two back insertion-time whole images are displayed in step s27. The back insertion-time whole image in which the back surface of the imaging plate 10 appears and the erasing-time whole image acquired when the erased imaging plate 10 is held by the holder 20 each include only the reflected light image, and do not include the radiograph.

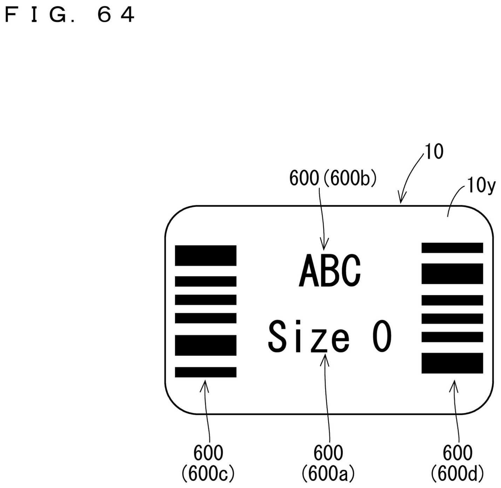

FIG. 64 is a schematic diagram showing one example of a back surface 10y of the imaging plate 10. In this example, information (also referred to as back surface specific information) 600 specific to the back surface 10y of the imaging plate 10 is shown on the back surface 10y to be visible. At least one piece of back surface specific information 600 may be shown on the back surface 10y of the imaging plate 10.

It can be said that the back surface specific information 600 is information not shown on the front surface of the imaging plate 10. It can also be said that the back surface specific information 600 is back surface identification information or a back surface identifier to identify the back surface of the imaging plate 10. It can also be said that the back surface specific information 600 is information indicating that the surface on which the back surface specific information 600 is shown is the back surface 10y of the imaging plate 10.

In the example of FIG. 64, a plurality of pieces of back surface specific information 600 are shown on the back surface of the imaging plate 10. The plurality of pieces of back surface specific information 600 include characters 600a representing the type of the size of the imaging plate 10, characters 600b representing a manufacturer of the imaging plate 10, a barcode 600c, and a barcode 600d. The barcode 600c and the barcode 600d may be the same, or may be different. At least one of the barcode 600c and the barcode 600d may represents a serial number of the imaging plate 10, for example.

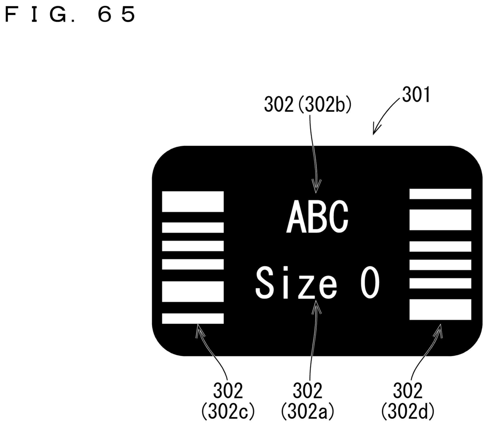

FIG. 65 is a schematic diagram showing one example of an IP reflected light image 301 included in the back insertion-time whole image acquired when the imaging plate 10 shown in FIG. 64 is set in reverse. The back surface 10y of the imaging plate 10 shown in FIG. 64 appears in the IP reflected light image 301.

The IP reflected light image 301 shown in FIG. 65 includes images 302 of the plurality of pieces of back surface specific information 600. It can be said that the images 302 of the pieces of back surface specific information 600 are reflected light images of the pieces of back surface specific information 600. The images 302 include an image 302a of the characters 600a representing the type of the size of the imaging plate 10, an image 302b of the characters 600b representing the manufacturer of the imaging plate 10, an image 302c of the barcode 600c, and an image 302d of the barcode 600d, for example.

A fringe region as an edge portion region of the imaging plate 10 is sometimes excluded from a target of image processing to acquire the radiograph. The back surface specific information 600 such as a barcode having an extremely small width may be shown in the fringe region, for example.

When the display 3 displays the back insertion-time whole image, the user can identify insertion of the imaging plate 10 into the reading apparatus 1 with the back surface thereof facing forward, that is, reverse setting of the imaging plate 10 based on the IP reflected light image 301 included in the displayed back insertion-time whole image. Specifically, the user can identify insertion of the imaging plate 10 into the reading apparatus 1 with the back surface thereof facing forward, that is, reverse setting of the imaging plate 10 by recognizing the images 302 (also referred to as back surface specific images 302) of the pieces of back surface specific information 600 included in the displayed IP reflected light image 301. As described above, the user can recognize that the imaging plate 10 has not properly been inserted into the reading apparatus 1.

Viewed another way, it can be said that the user can identify proper insertion of the imaging plate 10 into the reading apparatus 1 with the front surface thereof facing forward, that is, proper setting of the imaging plate 10 by recognizing the absence of any back surface specific images 302 included in the IP reflected light image displayed by the display 3.

The back surface specific information 600 shown on the back surface of the imaging plate 10 is not limited to that described above. For example, a two-dimensional barcode may be shown on the back surface of the imaging plate 10 as the back surface specific information 600. Characters, a symbol, or a graphic as the back surface specific information 600 may be shown in at least one of four corners on the back surface of the imaging plate 10. Minimal back surface specific information 600 required to identify the back surface of the imaging plate 10 may be shown on the back surface of the imaging plate 10.

The image processing unit 81 may cut out at least portion of the IP reflected light image 301 from the back insertion-time whole image as the cutout image as in a case where the second cutout image including at least portion of the IP reflected light image is cut out from the erasing-time whole image. In this case, the display control unit 82 may cause the display 3 to display the at least portion of the IP reflected light image 301 cut out from the back insertion-time whole image. The user can thus identify insertion of the imaging plate 10 into the reading apparatus 1 with the back surface thereof facing forward by mistake based on the displayed at least portion of the IP reflected light image 301.

While the back surface specific information shown on the back surface of the imaging plate 10 is used for the user to identify insertion of the imaging plate 10 into the reading apparatus 1 with the back surface thereof facing forward in the above-mentioned example, information (also referred to as front surface specific information) specific to the front surface of the imaging plate 10 shown on the front surface may be used. At least one piece of front surface specific information may be shown on the front surface of the imaging plate 10 to be visible. It can be said that the front surface specific information is information not shown on the back surface of the imaging plate 10. It can also be said that the front surface specific information is front surface identification information or a front surface identifier to identify the front surface of the imaging plate 10. It can also be said that the front surface specific information is information indicating that the surface on which the front surface specific information is shown is the front surface of the imaging plate 10.

An image signal as a result of detection of light from the imaging plate 10 when the imaging plate 10 is set by being inserted into the reading apparatus 1 with the front surface thereof facing forward may be referred to as a front insertion-time image signal. A whole image based on the front insertion-time image signal may be referred to as a front insertion-time whole image.

An image signal as a result of detection of light from the imaging plate 10 when the imaging plate 10 is set by being inserted into the reading apparatus 1 may be referred to as an insertion-time image signal, and a whole image based on the insertion-time image signal may be referred to as an insertion-time whole image. The front insertion-time image signal and the back insertion-time image signal are examples of the insertion-time image signal, and the front insertion-time whole image and the back insertion-time whole image are examples of the insertion-time whole image.

Light with which the imaging plate 10 is irradiated to acquire the insertion-time image signal may not necessarily be the excitation light L10. For example, when a surface of the imaging plate 10 is irradiated with light having no excitation ability, it may be determined that the surface is the back surface if the image of the back surface specific information 600 is acquired through detection of the reflected light. In this case, the reading processing through irradiation with the excitation light L10 may be started when it is not determined that the surface is the back surface.

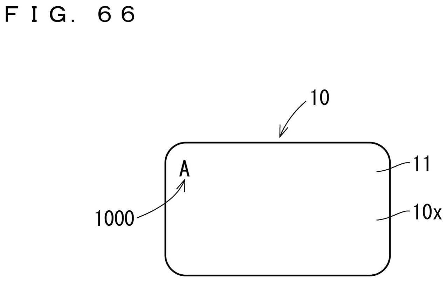

FIG. 66 is a schematic diagram showing one example of the imaging plate 10 having a front surface 10x on which front surface specific information 1000 is shown. In the example of FIG. 66, a single piece of front surface specific information 1000 is shown on the front surface 10x of the imaging plate 10. The front surface specific information 1000 is characters, for example. The front surface specific information 1000 may be characters representing the manufacturer of the imaging plate 10, for example, an initial letter of a name of the manufacturer of the imaging plate 10.

The radiograph formation layer 11 is provided to the front surface 10x of the imaging plate 10 excluding the front surface specific information 1000. In this example, the light emission-time whole image based on detection of the emitted light L2 and the reflected light from the imaging plate 10 includes a reflected light image of the front surface specific information 1000. The user can identify insertion of the imaging plate 10 into the reading apparatus 1 with the back surface thereof facing forward by mistake, that is, reverse setting of the imaging plate 10 by checking the absence of the image (i.e., the reflected light image) of the front surface specific information 1000 included in the acquired whole image or the cutout image displayed by the display 3, for example. The user can also identify proper insertion of the imaging plate 10 into the reading apparatus 1 with the front surface thereof facing forward, that is, proper setting of the imaging plate 10 by checking the presence of the image of the front surface specific information 1000 included in the acquired whole image or the cutout image displayed by the display 3.

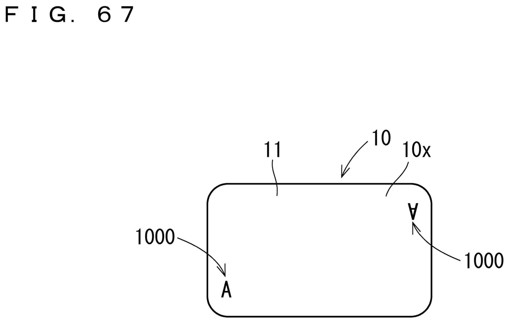

The front surface specific information 1000 is not limited to that in the example of FIG. 66. As shown in FIG. 67, a plurality of pieces of front surface specific information 1000 may be shown on the front surface 10x of the imaging plate 10. While the same two pieces of front surface specific information 1000 are shown on the front surface 10x of the imaging plate 10 in the example of FIG. 67, two different pieces of front surface specific information 1000 may be shown. Minimal front surface specific information 1000 required to identify the front surface 10x of the imaging plate 10 may be shown on the front surface of the imaging plate 10. The back surface specific information may be shown on the back surface of the imaging plate 10 while the front surface specific information 1000 is shown on the front surface of the imaging plate 10.

The fringe region as the edge portion region of the imaging plate 10 is sometimes excluded from the target of the image processing to acquire the radiograph. The front surface specific information 1000 such as a barcode having an extremely small width may be shown in the fringe region, for example.

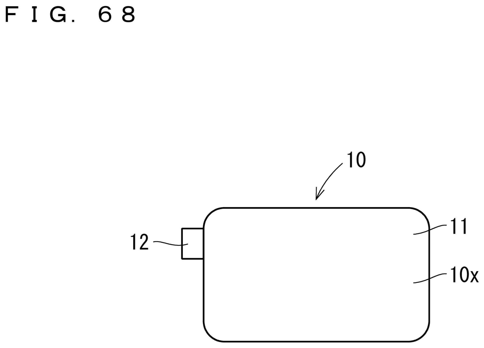

The imaging plate 10 may have a protrusion 12 for the user to identify insertion of the imaging plate 10 into the reading apparatus 1 with the back surface thereof facing forward, that is, reverse setting of the imaging plate 10. FIG. 68 shows one example of the imaging plate 10 having the protrusion 12. FIG. 68 shows a side of the front surface 10x of the imaging plate 10 in a state of the imaging plate 10 being set with the front surface thereof facing forward. In the example of FIG. 68, the imaging plate 10 has the protrusion 12 at a peripheral edge thereof. The imaging plate 10 has the protrusion 12 at one of four sides constituting the peripheral edge of the imaging plate 10 excluding a middle portion thereof. In the example of FIG. 68, the imaging plate 10 has the protrusion 12 at a short side thereof excluding a middle portion thereof. The radiograph formation layer 11 is not provided to a surface of the protrusion 12, and black anodizing is not performed on the surface of the protrusion 12. Not only the imaging plate 10 but also a region at the peripheral edge of the imaging plate 10 is scanned with the excitation light L10. A protrusion region as a region in which the protrusion 12 is present when the imaging plate 10 is inserted into the reading apparatus 1 with the front surface thereof facing forward is scanned with the excitation light L10. When the imaging plate 10 is inserted into the reading apparatus 1 with the back surface thereof facing forward, the protrusion 12 is not present in the protrusion region. The excitation light L10 is reflected from the surface of the protrusion 12 to the same extent as the surface of the imaging plate 10, for example. The acquired whole image based on the image signal output from the detector 40 includes a reflected light image of the protrusion 12 regardless of whether the imaging plate 10 is set in reverse. The imaging plate 10 may have the protrusion 12 at a long side thereof.

As described above, the imaging plate 10 has the protrusion 12 at one side thereof excluding the middle portion thereof, so that the protrusion 12 is always at different positions in a case where the imaging plate 10 is subjected to the back surface support and a case where the imaging plate 10 is subjected to the front surface support when the imaging plate 10 is viewed from a side of the light source 30.

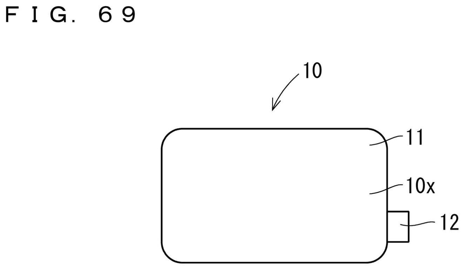

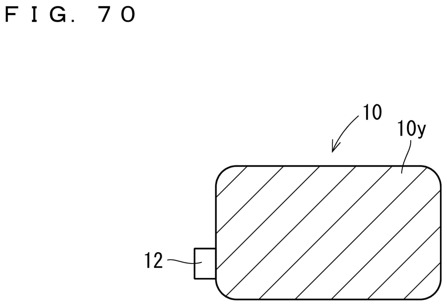

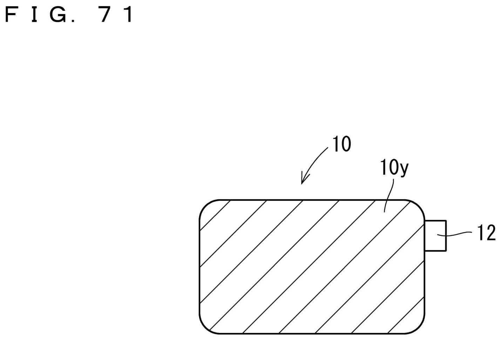

FIG. 69 is a schematic diagram showing one example of the imaging plate 10 viewed from a side of the front surface 10x as in FIG. 68. FIG. 69 shows the imaging plate 10 shown in FIG. 68 having been rotated by 180 degrees parallel to the main surface thereof. FIGs. 70 and 71 are schematic diagrams each showing one example of the imaging plate 10 viewed from a side of the back surface 10y in a state of the imaging plate 10 being set with the back surface thereof facing forward. FIG. 71 shows the imaging plate 10 shown in FIG. 70 having been rotated by 180 degrees parallel to the main surface thereof.

As shown in FIGs. 68 to 71, a position of the protrusion 12 when the imaging plate 10 is viewed from a side of the front surface 10x and a position of the protrusion 12 when the imaging plate 10 is viewed from a side of the back surface 10y are always different. As shown in FIGs. 68 and 69, when the imaging plate 10 is viewed from a side of the front surface 10x, the protrusion 12 is present at a position closer to a side (an upper side in FIG. 68) adjacent clockwise to the side to which the protrusion 12 is provided than to a side (a lower side in FIG. 68) adjacent counterclockwise to the side to which the protrusion 12 is provided. In contrast, as shown in FIGs. 70 and 71, when the imaging plate 10 is viewed from a side of the back surface 10y, the protrusion 12 is present at a position closer to the side (a lower side in FIG. 70) adjacent counterclockwise to the side to which the protrusion 12 is provided than to the side (an upper side in FIG. 70) adjacent clockwise to the side to which the protrusion 12 is provided.

As described above, when the imaging plate 10 has the protrusion 12 at one side thereof excluding the middle portion thereof, the position of the protrusion 12 when the imaging plate 10 is viewed from a side of the front surface 10x and the position of the protrusion 12 when the imaging plate 10 is viewed from a side of the back surface 10y are always different. The protrusion 12 is thus always at different positions in a case where the imaging plate 10 is subjected to the back surface support and a case where the imaging plate 10 is subjected to the front surface support when the imaging plate 10 is viewed from a side of the light source 30. In other words, the protrusion 12 is always at different positions in a case where the imaging plate 10 is set in reverse and a case where the imaging plate 10 is set properly when the imaging plate 10 is viewed from a side of the light source 30. A position of an image (i.e., a reflected light image) of the protrusion 12 in the light emission-time whole image or the erasing-time whole image acquired when the imaging plate 10 is subjected to the back surface support and a position of an image (i.e., a reflected light image) of the protrusion 12 in the back insertion-time whole image acquired when the imaging plate 10 is subjected to the front surface support are thus always different. The user can easily identify the front surface support of the imaging plate 10, that is, insertion of the imaging plate 10 into the reading apparatus 1 with the back surface thereof facing forward by checking the position of the image of the protrusion 12 in the acquired whole image displayed by the display 3. In other words, the user can easily identify reverse setting of the imaging plate 10 by checking the position of the protrusion 12 appearing in the acquired whole image displayed by the display 3. Viewed another way, the user can easily identify proper setting of the imaging plate 10 by checking the position of the protrusion 12 appearing in the acquired whole image displayed by the display 3.

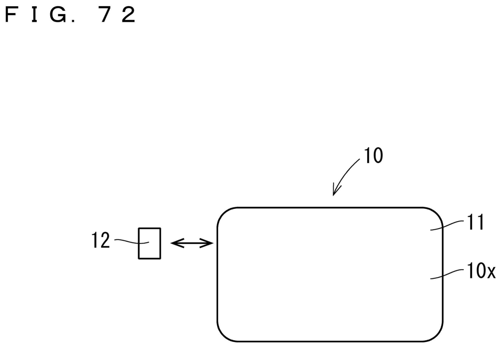

The protrusion 12 may be removable from the imaging plate 10 as shown in FIG. 72.

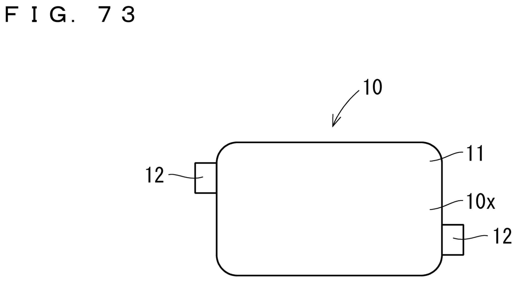

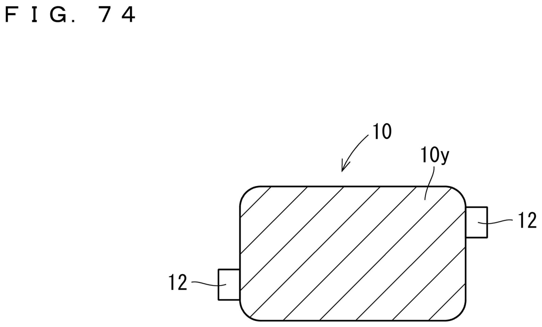

The imaging plate 10 may have a plurality of protrusions 12 at the peripheral edge thereof. FIGs. 73 and 74 are schematic diagrams showing one example of the imaging plate 10 having two protrusions 12 at the peripheral edge thereof. FIG. 73 shows the imaging plate 10 viewed from a side of the front surface 10x, and FIG. 74 shows the imaging plate 10 viewed from a side of the back surface 10y. In the example of FIGs. 73 and 74, the imaging plate 10 has the two protrusions 12 at two opposing sides thereof. The two protrusions 12 are present at positions point symmetric with respect to the center of the front surface 10x (or the back surface 10y) when the imaging plate 10 is viewed from a side of the front surface 10x (or the back surface 10y). Even when the imaging plate 10 has the two protrusions 12 at the peripheral edge thereof as shown in FIGs. 73 and 74, positions of images of the protrusions 12 in the light emission-time whole image or the erasing-time whole image acquired when the imaging plate 10 is subjected to the back surface support and positions of images of the protrusions 12 in the back insertion-time whole image acquired when the imaging plate 10 is subjected to the front surface support are always different. The user can thus easily identify insertion of the imaging plate 10 into the reading apparatus 1 with the back surface thereof facing forward, that is, reverse setting of the imaging plate 10 by checking the positions of the images of the protrusions 12 in the acquired whole image displayed by the display 3. The user can also easily identify insertion of the imaging plate 10 into the reading apparatus 1 with the front surface thereof facing forward, that is, proper setting of the imaging plate 10 by checking the positions of the images of the protrusions 12 in the acquired whole image displayed by the display 3.

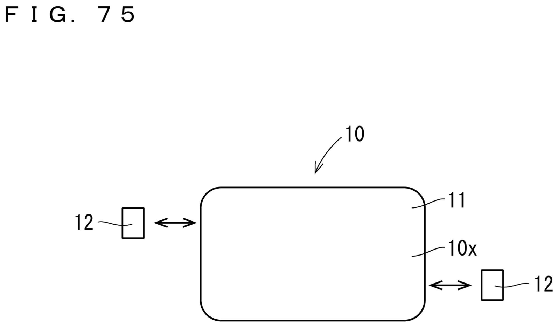

The two protrusions 12 may each be removable from the imaging plate 10 as shown in FIG. 75. Only one of the two protrusions 12 may be removable from the imaging plate 10. When the imaging plate 10 has at least one protrusion 12, the front surface specific information may be shown on the front surface 10x of the imaging plate 10, and the back surface specific information may be shown on the back surface 10y of the imaging plate 10.

The image processing unit 81 may perform determination processing of determining whether the imaging plate 10 is set in reverse based on a result of detection of the detector 40. In this case, the image processing unit 81 functions as a determination unit that determines whether the imaging plate 10 is set in reverse. It can be said that the determination processing is processing of determining whether the imaging plate 10 is inserted into the reading apparatus 1 with the back surface thereof facing forward.

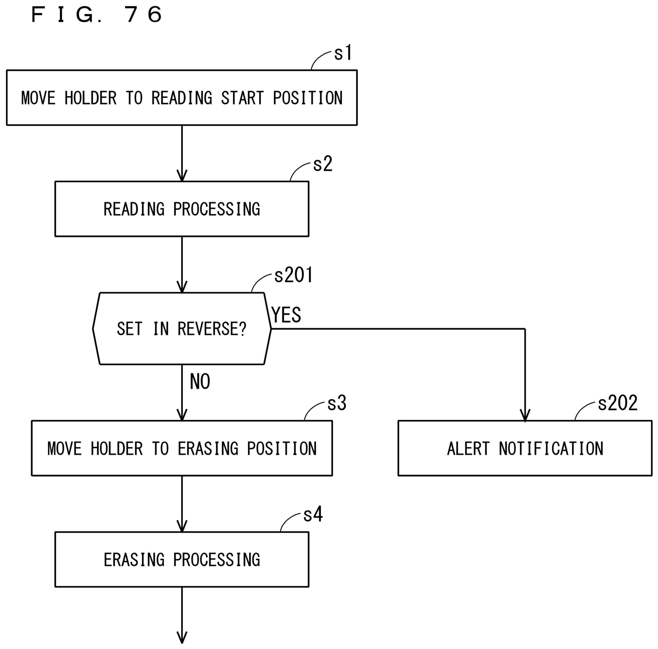

FIG. 76 is a flowchart showing one example of operation of the reading apparatus 1 when the image processing unit 81 performs the determination processing. When the start button included in the operation unit 4 is operated after the imaging plate 10 inserted through the inlet 2a of the housing 2 is held by the holder 20, that is, the imaging plate 10 is set in the reading apparatus 1, the above-mentioned steps s1 and s2 are performed. At the start of step s1, it is not known whether the imaging plate 10 is set properly or is set in reverse in the reading apparatus 1.

When the reading processing in step s2 ends, the image processing unit 81 performs the determination processing in step s201. In the determination processing, the image processing unit 81 determines whether the imaging plate 10 is set in reverse based on the image signal output from the detector 40 in the reading processing in step s2.

Consider herein a case where the back surface specific information 600 is shown on the back surface of the imaging plate 10 as in the above-mentioned example of FIG. 64. In this case, the image processing unit 81 determines whether the acquired whole image based on the image signal acquired in step s2 includes the image 302 (i.e., the back surface specific image 302) of the back surface specific information 600. The image processing unit 81 determines whether the acquired whole image includes the image 302c of the barcode 600c, for example. When determining that the acquired whole image acquired in step s2 includes the back surface specific image 302, the image processing unit 81 determines that the imaging plate 10 is set in reverse. On the other hand, when determining that the acquired whole image acquired in step s2 does not include the back surface specific image 302, the image processing unit 81 determines that the imaging plate 10 is not set in reverse. That is to say, the image processing unit 81 determines that the imaging plate 10 is set properly.

Consider, as another example, a case where the front surface specific information 1000 is shown on the front surface of the imaging plate 10 as shown in FIGs. 66 and 67 described above. In this case, the image processing unit 81 determines whether the acquired whole image acquired in step s2 includes the image of the front surface specific information 1000. When determining that the acquired whole image includes the image of the front surface specific information 1000, the image processing unit 81 determines that the imaging plate 10 is not set in reverse. On the other hand, when determining that the acquired whole image does not include the image of the front surface specific information 1000, the image processing unit 81 determines that the imaging plate 10 is set in reverse.

Consider, as yet another example, a case where the imaging plate 10 has at least one protrusion 12 to determine whether the imaging plate 10 is set in reverse at the peripheral edge thereof as in the above-mentioned examples of FIGs. 68 to 74. In this case, the image processing unit 81 checks the position of the image of the protrusion 12 in the acquired whole image acquired in step s2. The image processing unit 81 determines whether the imaging plate 10 is set in reverse based on the position of the image of the protrusion 12 in the acquired whole image. As described above, the position of the image of the protrusion 12 in the acquired whole image acquired when the imaging plate 10 is subjected to the back surface support and the position of the image of the protrusion 12 in the acquired whole image acquired when the imaging plate 10 is subjected to the front surface support are always different. The image processing unit 81 can thus determine whether the imaging plate 10 is set in reverse based on the position of the image of the protrusion 12 in the acquired whole image. The image processing unit 81 can identify the position of the image of the protrusion 12 in the acquired whole image based on the binarized image acquired by binarizing the acquired whole image, for example.

When it is determined that the imaging plate 10 is not set in reverse in step s201, the above-mentioned steps s3 and s4 are performed. After step s4, steps s5, s6, and s7 may be performed as in the above-mentioned example of FIG. 18, or steps s21, s22, s5, s6, and s27 may be performed as in the above-mentioned example of FIG. 27.

On the other hand, when it is determined that the imaging plate 10 is set in reverse in step s201, step s202 is performed. In step s202, the reading apparatus 1 notifies the user of an alert. In this case, the display control unit 82 may cause the display 3 to display alert information 650 to notify the user of the alert. In this case, the display 3 functions as a notification unit that notifies the user of the alert.

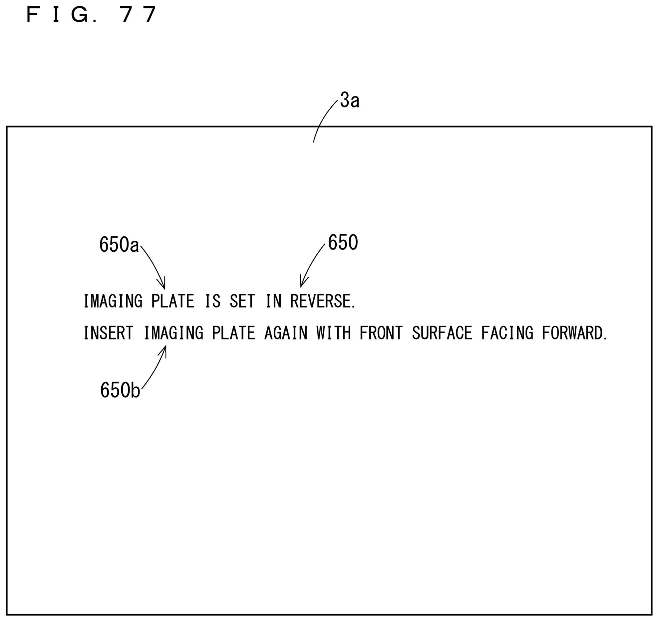

FIG. 77 is a schematic diagram showing an example of display of the alert information 650. In the example of FIG. 77, the alert information 650 includes notification information 650a that notifies the user of reverse setting of the imaging plate 10 and instruction information 650b that instructs the user to insert the imaging plate 10 again with the front surface thereof facing forward. It can be said that the instruction information 650b is instruction information to instruct the user to set the imaging plate 10 properly.

The alert information 650 is not limited to that in the above-mentioned example. The reading apparatus 1 may notify the user of the alert by means other than display of information. For example, when the reading apparatus 1 includes a sound output means, such as a speaker, of outputting a sound to the outside of the housing 2, the reading apparatus 1 may notify the user of the alert by outputting a predetermined alert sound from the sound output means. In this case, the sound output means functions as the notification unit that notifies the user of the alert. When the reading apparatus 1 includes a light emitter, such as an LED, that outputs light to the outside of the housing 2, the reading apparatus 1 may notify the user of the alert by causing the light emitter to emit light. In this case, the light emitter functions as the notification unit that notifies the user of the alert.

When notified of the alert by the reading apparatus 1, the user operates a discharge button included in the operation unit 4, for example. When the operation unit 4 receives an operation on the discharge button, the above-mentioned steps s5 and s6 are performed to discharge the imaging plate 10 from which the radiograph has not been erased to the outlet 2b in the reading apparatus 1. The user then inserts the imaging plate 10 discharged from the reading apparatus 1 into the reading apparatus 1 again. Then, when the start button included in the operation unit 4 is operated, a series of processes in FIG. 76 is performed again.

The reading apparatus 1 may cause the display 3 to display the acquired whole image (i.e., the back insertion-time whole image) acquired in step s2 while notifying the user of the alert in step s202. In this case, the display 3 may simultaneously and separately display the alert information 650 and the back insertion-time whole image, for example.

As described above, the image processing unit 81 can determine whether the imaging plate is set in reverse based on the result of detection of the detector 40. This allows the radiograph to be more surely read from the imaging plate 10 based on a result of determination. Notification of the user of the alert in response to a result of determination as in the above-mentioned example can prompt the user to properly set the imaging plate 10 to the reading apparatus 1, for example. This allows the radiograph to be more surely read from the imaging plate 10.

In the example of FIG. 76, the user is notified of the alert without performing the erasing processing when it is determined that the imaging plate is set in reverse. More specifically, in the example of FIG. 76, while the erasing processing is performed when it is not determined that the imaging plate is set in reverse, the erasing processing is not performed, and, further, the user is notified of the alert when it is determined that the imaging plate is set in reverse. In the example of FIG. 76, the user is notified of the alert without irradiating the imaging plate 10 with the erasing light L3 when it is determined that the imaging plate is set in reverse. The user can thus be notified of the alert immediately upon determination that the imaging plate is set in reverse. Notification of the alert in step s202 may be performed between steps s3 and s4, or may be performed after step s4.

The erasing processing is accompanied by irradiation of the imaging plate 10 with the erasing light L3. Irradiation with the erasing light L3 in a state of the imaging plate 10 being set in reverse can adversely affect the radiograph recorded on the imaging plate 10 as the latent image. For example, when there is a gap between the support plate 21 and the imaging plate 10, the erasing light L3 sometimes enters the gap to partially erase or fade the radiograph recorded on the imaging plate 10. The imaging plate 10 can also generate heat to adversely affect the detected signal due to irradiation with the erasing light L3. It also takes additional time for the erasing processing. The erasing processing is not performed when the imaging plate is set in reverse to prevent these adverse effects and losses.

When at least one piece of back surface specific information 600 is shown on the back surface of the imaging plate 10 as in the example of FIG. 64, it is easy to determine whether the imaging plate 10 is set in reverse.

When at least one piece of front surface specific information 1000 is shown on the front surface of the imaging plate 10 as in the examples of FIGs. 66 and 67, it is easy to determine whether the imaging plate 10 is set in reverse.

When the imaging plate 10 has at least one protrusion 12 to determine whether the imaging plate 10 is set in reverse at the peripheral edge thereof as in the examples of FIGs. 68 to 75, it is easy to determine whether the imaging plate 10 is set in reverse.

While the reading apparatus 1 discharges the imaging plate 10 in response to the instructions from the user when it is determined that the imaging plate 10 is set in reverse in the above-mentioned examples, the imaging plate 10 may automatically be discharged without the instructions from the user. For example, step s3 may be performed after step s202. In this case, after the erasing processing is performed, the above-mentioned steps s5 and s6 are performed to automatically discharge the imaging plate 10.

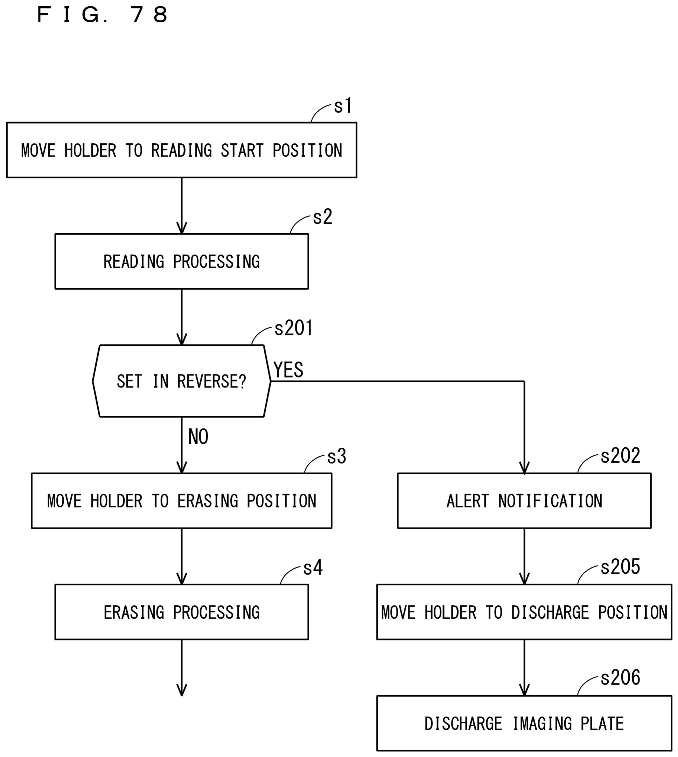

As shown in FIG. 78, steps s205 and s206 may sequentially be performed after step s202. In step s205, the holder 20 is moved to the discharge position as in the above-mentioned step s5. In step s206, the imaging plate 10 is discharged to the outlet 2b as in the above-mentioned step s6. The imaging plate 10 is thereby automatically discharged when it is determined that the imaging plate 10 is set in reverse. That is to say, the imaging plate 10 is discharged in response to determination that the imaging plate 10 is set in reverse.

As described above, discharge of the imaging plate 10 when it is determined that the imaging plate 10 is set in reverse eliminates the need for the user operation to provide instructions to discharge the imaging plate 10 on the reading apparatus 1. Usability of the reading apparatus 1 is thereby improved. Discharge of the imaging plate 10 can prompt the user to set the imaging plate 10 again.

In the example of FIG. 78, the imaging plate 10 is discharged without performing the erasing processing when it is determined that the imaging plate is set in reverse. The imaging plate 10 can thereby be discharged immediately upon determination that the imaging plate is set in reverse.

After affirmative determination in step s201, steps s205 and s206 may be performed without performing step s202. That is to say, the imaging plate 10 may be discharged without notifying the user of the alert when it is determined that the imaging plate 10 is set in reverse. Step s202 may be performed after steps s205 and s206, or may be performed between steps s205 and s206.

When the plurality of pieces of back surface specific information 600 are shown on the back surface of the imaging plate 10 as in the example of FIG. 64, reverse setting of the imaging plate 10 can be identified immediately even if the orientation of the imaging plate when the imaging plate 10 is set is not constant. Description will be made in this respect below.

In the reading processing, the driver 50 moves the holder 20 holding the imaging plate 10 in the subscannig direction DRs while the light source 30 performs scanning processing of repeatedly performing main scanning direction scanning of scanning the imaging plate 10 with the excitation light L10 in the main scanning direction DRm as described above. The imaging plate 10 is thus raster scanned with the excitation light L10. It can be said that the start of the reading processing is the start of the scanning processing of the light source 30.

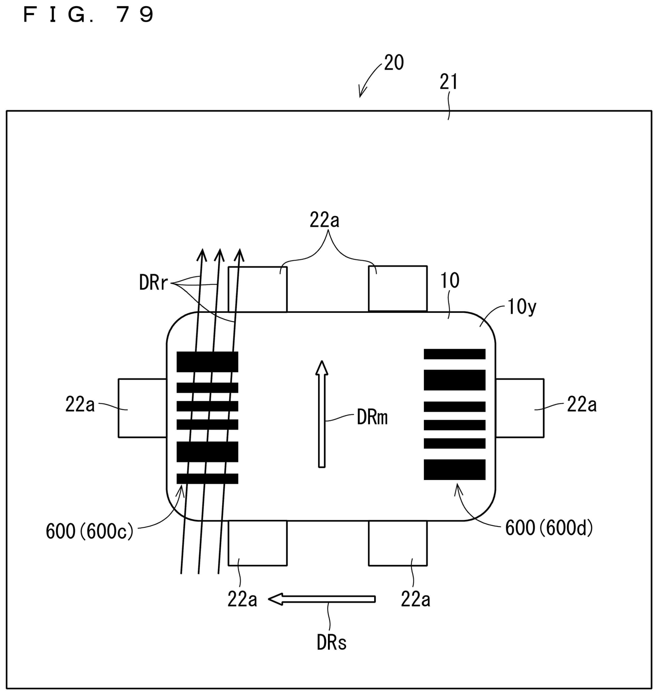

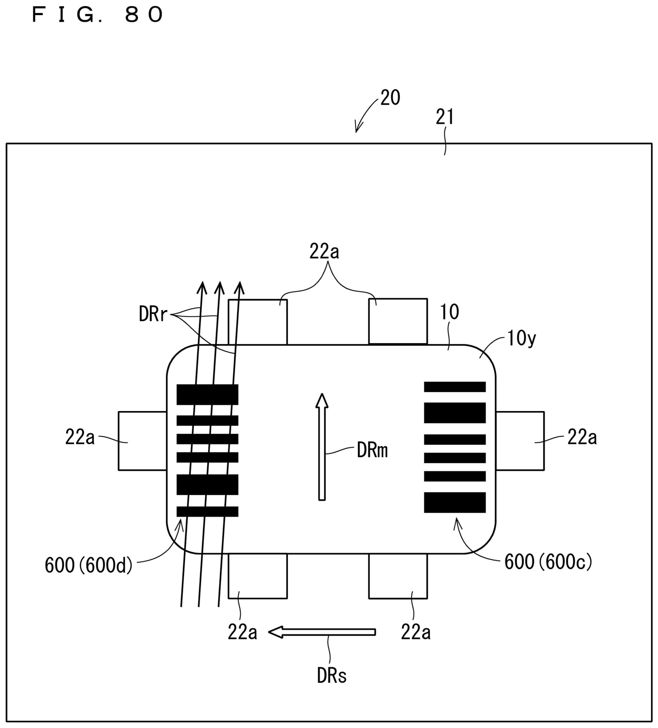

FIGs. 79 and 80 are schematic diagrams each illustrating one example of reverse setting of the imaging plate 10 having the back surface 10y on which the plurality of pieces of back surface specific information 600 are shown. In the examples of FIGs. 79 and 80, only the barcodes 600c and 600d are shown on the back surface 10y of the imaging plate 10. The barcodes 600c and 600d are provided to respective end portions in a longitudinal direction of the back surface 10y of the imaging plate 10. A scanning direction DRr in which the imaging plate 10 is scanned with the excitation light L10 in the scanning processing (i.e., raster scanning) is shown in each of FIGs. 79 and 80. In the example of FIG. 80, the imaging plate 10 is set in a state of being rotated by 180 degrees parallel to the main surface thereof compared with that in the example of FIG. 79.

One of opposite sides in the longitudinal direction of the imaging plate 10 on which raster scanning starts is referred to as a scanning forward side, and the other one of the opposite sides in the longitudinal direction of the imaging plate 10 on which raster scanning ends is referred to as a scanning backward side. The scanning forward side of the imaging plate 10 is a left side in each of FIGs. 79 and 80, and the scanning backward side of the imaging plate 10 is a right side in each of FIGs. 79 and 80. In the examples of FIGs. 79 and 80, the back surface specific information 600 is shown in each of the end portion on the scanning forward side and the end portion on the scanning backward side on the back surface 10y of the imaging plate 10. In this example, the imaging plate 10 is sometimes set so that one of opposite end portions in the longitudinal direction thereof is located on the scanning forward side, and is sometimes set so that the other one of the opposite end portions in the longitudinal direction thereof is located on the scanning forward side as illustrated in FIGs. 79 and 80.

As described above, in the reading processing according to this example, the main scanning direction scanning is repeatedly performed during movement of the imaging plate 10 in the subscannig direction DRs. Scanning (also referred to as unit scanning) of the imaging plate 10 with the excitation light L10 in a direction crossing the longitudinal direction of the imaging plate 10 is thus repeatedly performed from the scanning forward side to the scanning backward side as shown by the scanning direction DRr in each of FIGs. 79 and 80. In each of FIGs. 79 and 80, each of a plurality of arrows showing the scanning direction DRr represents the unit scanning.

When the imaging plate 10 is set so that the barcode 600c is located on the scanning forward side as illustrated in FIG. 79, the image of the barcode 600c is acquired in the first half of the reading processing in step s2. When the imaging plate 10 is set so that the barcode 600d is located on the scanning forward side as illustrated in FIG. 80, the image of the barcode 600d is acquired in the first half of the reading processing. The image processing unit 81 can thus determine whether the acquired whole image includes the image of the back surface specific information 600 by checking not the acquired whole image acquired in the reading processing as a whole but only the image acquired in the first half of the reading processing in the acquired whole image in the above-mentioned step s201. When the image acquired in the first half of the reading processing in the acquired whole image includes the image of the back surface specific information 600, the image processing unit 81 determines that the imaging plate 10 is set in reverse. On the other hand, when the image acquired in the first half of the reading processing in the acquired whole image does not include the image of the back surface specific information 600, the image processing unit 81 determines that the imaging plate 10 is not set in reverse.

In contrast, consider a case where the barcode 600c is not shown on the back surface 10y of the imaging plate 10, for example. In this case, the image of the barcode 600c is acquired in the first half of the reading processing when the imaging plate 10 is set in the orientation in FIG. 79. The image of the barcode 600c, however, is acquired in the second half of the reading processing when the imaging plate 10 is set in the orientation in FIG. 80. The image processing unit 81 is thus required to check the acquired whole image as a whole, and determine whether the acquired whole image includes the image of the back surface specific information 600 to identify reverse setting of the imaging plate 10 when the imaging plate 10 is set in the orientation in FIG. 79 and when the imaging plate 10 is set in the orientation in FIG. 80.

As described above, the image processing unit 81 can determine whether the acquired whole image includes the image of the back surface specific information 600 by checking only a portion of the acquired whole image even when the plurality of pieces of back surface specific information 600 are shown on the back surface of the imaging plate 10, and the orientation of the imaging plate when the imaging plate 10 is set is not constant. The image processing unit 81 can thus determine whether the acquired whole image includes the image of the back surface specific information 600 immediately. The image processing unit 81 can thus identify reverse setting of the imaging plate 10 immediately. As a result, the reading apparatus 1 can notify the user of the alert immediately, and can discharge the imaging plate 10 immediately, for example.

In a case where the back surface specific information 600 is shown in each of the end portion on the scanning forward side and the end portion on the scanning backward side on the back surface of the imaging plate 10 as in the examples of FIGs. 79 and 80, the image processing unit 81 can determine whether the acquired whole image includes the image of the back surface specific information 600 by checking only an image acquired early in the reading processing in the acquired whole image when the imaging plate 10 is set in the orientation in FIG. 79 and when the imaging plate 10 is set in the orientation in FIG. 80. The image processing unit 81 can thus identify reverse setting of the imaging plate 10 immediately. The number of pieces of back surface specific information 600 shown on the back surface of the imaging plate 10 may be one. A position on the back surface of the imaging plate 10 where the back surface specific information 600 is shown is not limited to that in the above-mentioned example.

When the plurality of pieces of front surface specific information 1000 are shown on the front surface of the imaging plate 10 as in the example of FIG. 67, reverse setting of the imaging plate 10 can similarly be identified immediately even if the orientation of the imaging plate 10 when the imaging plate 10 is set is not constant.

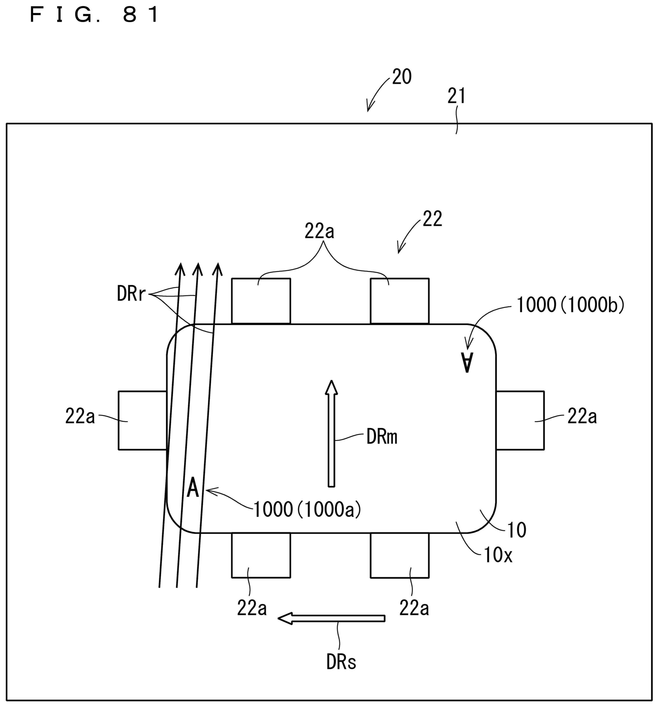

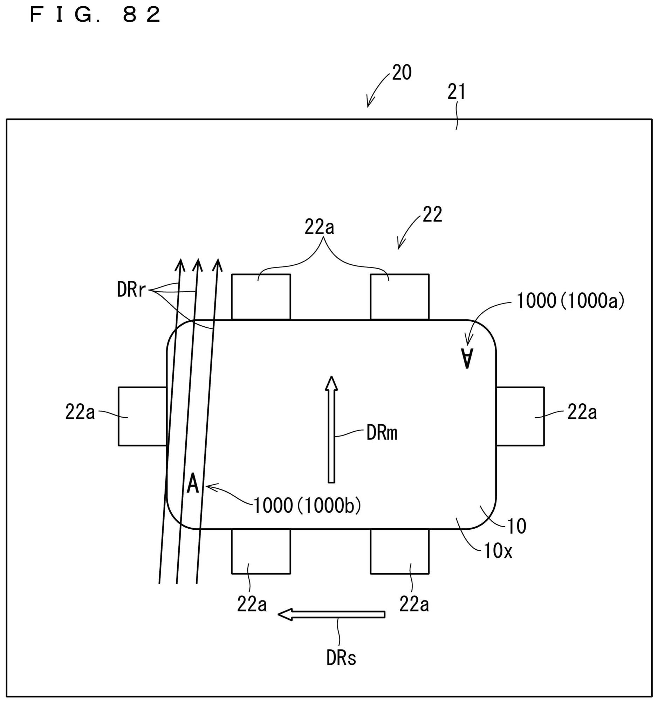

FIGs. 81 and 82 are schematic diagrams each illustrating one example of normal setting of the imaging plate 10 having the front surface 10x on which the plurality of pieces of front surface specific information 1000 are shown. In the examples of FIGs. 81 and 82, letters 1000a and 1000b as the pieces of front surface specific information 1000 are shown on the front surface 10x of the imaging plate 10. The letters 1000a and 1000b are shown in the end portion on the scanning forward side and the end portion on the scanning backward side on the front surface 10x of the imaging plate 10. In the example of FIG. 82, the imaging plate 10 is set in a state of being rotated by 180 degrees parallel to the main surface thereof compared with that in the example of FIG. 81.

When the imaging plate 10 is set so that the letter 1000a is located on the scanning forward side as illustrated in FIG. 81, an image of the letter 1000a is acquired in the first half of the reading processing. When the imaging plate 10 is set so that the letter 1000b is located on the scanning forward side as illustrated in FIG. 82, an image of the letter 1000b is acquired in the first half of the reading processing. The image processing unit 81 can thus determine whether the acquired whole image includes the image of the front surface specific information 1000 by checking only the image acquired in the first half of the reading processing in the acquired whole image acquired in the reading processing in step s201. When the image acquired in the first half of the reading processing in the acquired whole image does not include the image of the front surface specific information 1000, the image processing unit 81 can determine that the imaging plate 10 is set in reverse.

As described above, the image processing unit 81 can determine whether the acquired whole image includes the image of the front surface specific information 1000 by checking only a portion of the acquired whole image even when the plurality of pieces of front surface specific information 1000 are shown on the front surface of the imaging plate 10, and the orientation of the imaging plate 10 when the imaging plate 10 is set is not constant. The image processing unit 81 can thus identify reverse setting of the imaging plate 10 immediately.

In a case where the front surface specific information 1000 is shown in each of the end portion on the scanning forward side and the end portion on the scanning backward side on the front surface of the imaging plate 10 as in the examples of FIGs. 81 and 82, the image processing unit 81 can determine whether the acquired whole image includes the image of the front surface specific information 1000 by checking only the image acquired early in the reading processing in the acquired whole image when the imaging plate 10 is set in the orientation in FIG. 81 and when the imaging plate 10 is set in the orientation in FIG. 82. The image processing unit 81 can thus identify reverse setting of the imaging plate 10 immediately.

In the examples of FIGs. 81 and 82, the front surface specific information 1000 is shown, in the end portion on the scanning forward side on the front surface 10x of the set imaging plate 10, on a side closer to a position where unit scanning of raster scanning starts than a middle portion is. The image processing unit 81 can thus determine whether the acquired whole image includes the image of the front surface specific information 1000 by checking only a smaller portion of the acquired whole image. The image processing unit 81 can thus identify reverse setting of the imaging plate 10 immediately.

A position on the front surface of the imaging plate 10 where the front surface specific information 1000 is shown is not limited to that in the above-mentioned example. The back surface specific information 600 may be shown, in the end portion on the scanning forward side on the back surface of the imaging plate 10, on a side closer to a position where unit scanning of raster scanning starts than a middle portion is. The image processing unit 81 can thus determine whether the acquired whole image includes the image of the back surface specific information 600 by checking only a smaller portion of the acquired whole image. The image processing unit 81 can thus identify reverse setting of the imaging plate 10 immediately.

When the imaging plate 10 has the plurality of protrusions 12 at the peripheral edge thereof as in the examples of FIGs. 73 to 75, the image processing unit 81 can similarly identify reverse setting of the imaging plate 10 immediately even if the orientation of the imaging plate 10 when the imaging plate 10 is set is not constant.

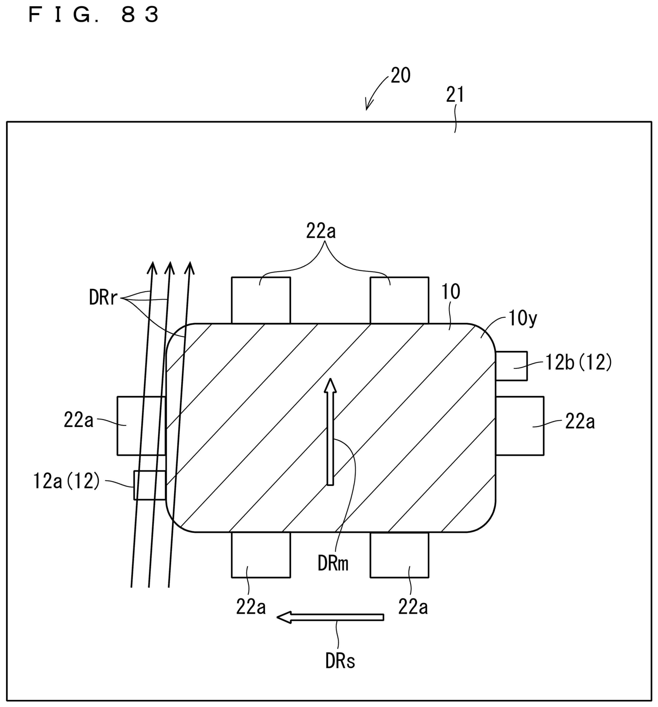

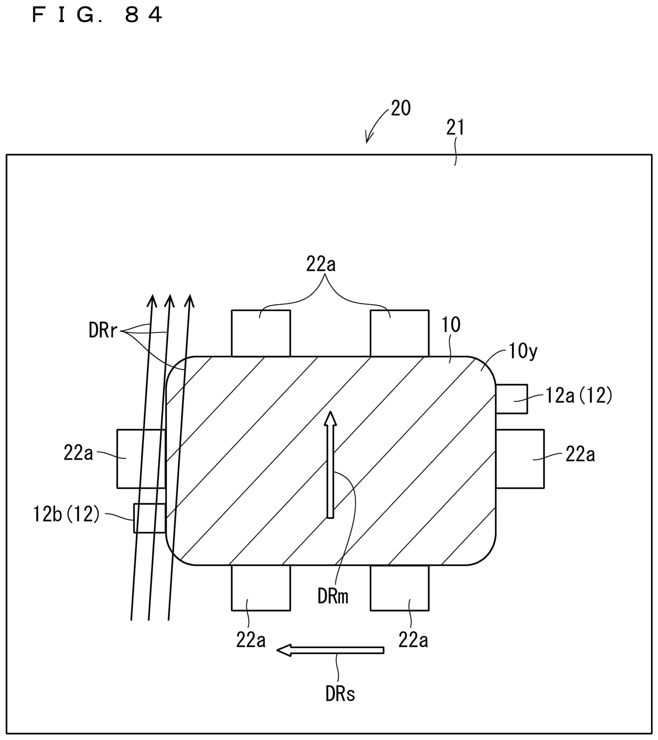

FIGs. 83 and 84 are schematic diagrams each illustrating one example of reverse setting of the imaging plate 10 having the plurality of protrusions 12. The imaging plate 10 has protrusions 12a and 12b at the peripheral edge thereof in the examples of FIGs. 83 and 84. The imaging plate 10 has the protrusions 12a and 12b at a short side on the scanning forward side and a short side on the scanning backward side of the peripheral edge thereof. In the example of FIG. 84, the imaging plate 10 is set in a state of being rotated by 180 degrees parallel to the main surface thereof compared with that in the example of FIG. 83.

When the imaging plate 10 is set so that the protrusion 12a is located on the scanning forward side as illustrated in FIG. 83, an image of the protrusion 12a is acquired in the first half of the reading processing. When the imaging plate 10 is set so that the protrusion 12b is located on the scanning forward side as illustrated in FIG. 84, an image of the protrusion 12b is acquired in the first half of the reading processing. The image processing unit 81 can thus determine whether the acquired whole image includes the image of the protrusion 12 by checking only the image acquired in the first half of the reading processing in the acquired whole image acquired in the reading processing in step s201. When the image acquired in the first half of the reading processing in the acquired whole image includes the image of the protrusion 12, the image processing unit 81 identifies the position of the protrusion 12 relative to the imaging plate 10 based on the position of the image of the protrusion 12 in the image acquired in the first half of the reading processing. When the identified position of the protrusion 12 matches the position (see FIGs. 73 and 74, for example) of the protrusion 12 when the imaging plate 10 is subjected to the front surface support, the image processing unit 81 determines that the imaging plate 10 is set in reverse. On the other hand, when the identified position of the protrusion 12 does not match the position of the protrusion 12 when the imaging plate 10 is subjected to the front surface support, the image processing unit 81 determines that the imaging plate 10 is not set in reverse.

As described above, even when the imaging plate 10 has the plurality of protrusions 12 at the peripheral edge thereof, and the orientation of the imaging plate 10 when the imaging plate 10 is set is not constant, the image processing unit 81 can determine whether the acquired whole image includes the image of the protrusion 12 by checking only a portion of the acquired whole image. The image processing unit 81 can thus identify reverse setting of the imaging plate 10 immediately.

In a case where the imaging plate 10 has the protrusions 12 at a side on the scanning forward side and a side on the scanning backward side of the peripheral edge thereof as in the examples of FIGs. 83 and 84, the image processing unit 81 can determine whether the acquired whole image includes the image of the protrusion 12 by checking only an image acquired early in the reading processing in the acquired whole image when the imaging plate 10 is set in the orientation in FIG. 83 and when the imaging plate 10 is set in the orientation in FIG. 84. The image processing unit 81 can thus identify reverse setting of the imaging plate 10 immediately.

In the examples of FIGs. 83 and 84, the imaging plate 10 has the protrusion 12 at a side of the scanning forward side of the peripheral edge thereof on a side closer to a position where unit scanning of raster scanning starts than a middle portion is. The image processing unit 81 can thus determine whether the acquired whole image includes the image of the protrusion 12 by checking only a smaller portion of the acquired whole image. The image processing unit 81 can thus identify reverse setting of the imaging plate 10 immediately. A position at the peripheral edge of the imaging plate 10 where the imaging plate 10 has the protrusion 12 is not limited to that in the above-mentioned example.

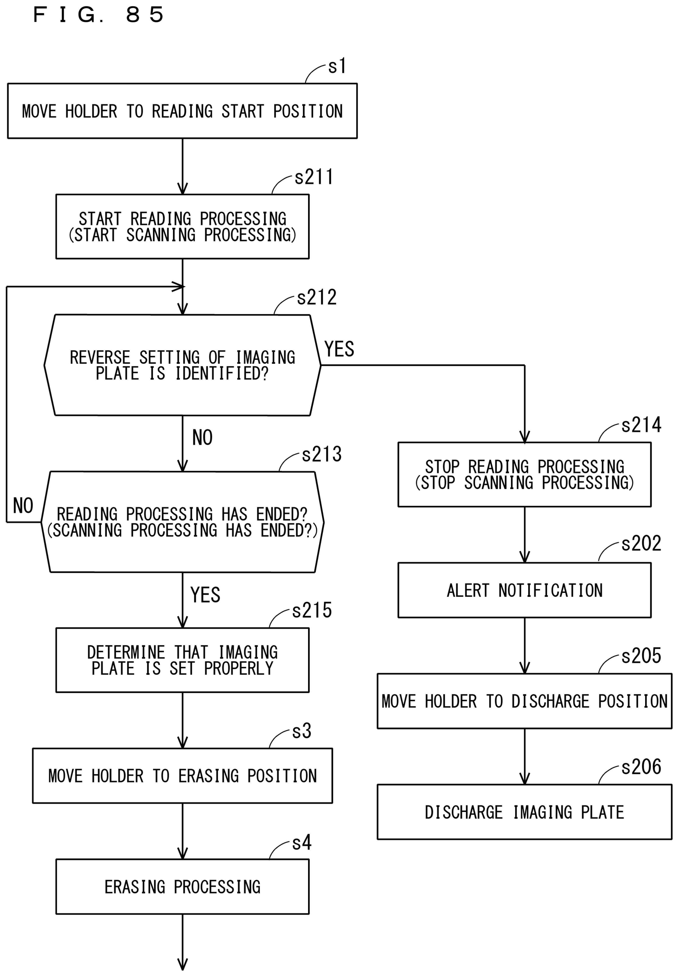

While the determination processing is performed after the reading processing in step s2 ends in the above-mentioned example, the reading processing and the determination processing may be performed in parallel. FIG. 85 is a flowchart showing one example of operation of the reading apparatus 1 in this case. When the start button included in the operation unit 4 is operated to perform step s1, the reading processing starts in step s211.

When the reading processing starts, in other words, the scanning processing starts, the image processing unit 81 determines whether reverse setting of the imaging plate 10 can be identified based on luminance values sequentially output from the detector 40 in response to raster scanning in step s212. Specifically, the image processing unit 81 determines whether reverse setting of the imaging plate 10 can be identified based on a plurality of luminance values (i.e., a plurality of pixel signals or a plurality of pixel values) sequentially output from the detector 40 from the start of the reading processing to a current time. Step s212 may be performed each time a single luminance value is output from the detector 40, or each time a plurality of luminance values are output from the detector 40, for example. Step s212 may be performed each time a row of raster scanning with the excitation light L10 is performed, or each time a plurality of rows of raster scanning with the excitation light L10 are performed, for example.

Consider herein a case where the back surface specific information 600 is shown on the back surface of the imaging plate 10. In this case, the image processing unit 81 determines whether an image (also referred to as a determination target image) represented by the plurality of luminance values output from the detector 40 from the start of the reading processing to the current time includes the image 302 (i.e., the back surface specific image 302) of the back surface specific information 600 in step s212. Luminance values of a plurality of pixels constituting the determination target image include the luminance values output from the detector 40 from the start of the reading processing to the current time. The determination target image is at least portion of the acquired whole image acquired by completion of the reading processing. When determining that the determination target image includes the back surface specific image 302, the image processing unit 81 determines that reverse setting of the imaging plate 10 can be identified. That is to say, the image processing unit 81 determines that the imaging plate 10 is set in reverse. On the other hand, when determining that the determination target image does not include the back surface specific image 302, the image processing unit 81 determines that reverse setting of the imaging plate 10 cannot be identified.

Consider, as another example, a case where two pieces of front surface specific information 1000 are shown on the front surface of the imaging plate 10 as illustrated in FIGs. 81 and 82 described above, for example. In this case, the image processing unit 81 determines whether the determination target image includes the images of the pieces of front surface specific information 1000 in step s212. When determining that the determination target image includes the images of the pieces of front surface specific information 1000, the image processing unit 81 determines that reverse setting of the imaging plate 10 cannot be identified. On the other hand, when determining that the determination target image does not include the pieces of front surface specific information 1000, the image processing unit 81 determines whether the determination target image corresponds to the half of the acquired whole image, for example. When determining that the determination target image corresponds to the half of the acquired whole image, the image processing unit 81 determines that reverse setting of the imaging plate 10 can be identified. On the other hand, when determining that the determination target image does not correspond to the half of the acquired whole image, in other words, when determining that the determination target image is less than half of the acquired whole image, the image processing unit 81 determines that reverse setting of the imaging plate 10 cannot be identified.

The determination target image not including the image of the front surface specific information 1000 and corresponding to the half of the acquired whole image herein means that the image acquired in the first half of the reading processing in the acquired whole image does not include the image of the front surface specific information 1000. It can thus be said that, when the image acquired in the first half of the reading processing in the acquired whole image does not include the image of the front surface specific information 1000, the image processing unit 81 determines that reverse setting of the imaging plate 10 can be identified.

Consider, as another example, a case where the imaging plate 10 has the protrusion 12 at the peripheral edge thereof. In this case, the image processing unit 81 determines whether the determination target image includes the image of the protrusion 12 in step s212. When determining that the determination target image includes the image of the protrusion 12, the image processing unit 81 identifies the position of the protrusion 12 relative to the imaging plate 10 based on the position of the image of the protrusion 12 in the determination target image. When the identified position of the protrusion 12 matches the position (see FIGs. 83 and 84, for example) of the protrusion 12 when the imaging plate 10 is subjected to the front surface support, the image processing unit 81 determines that reverse setting of the imaging plate 10 can be identified. On the other hand, when the identified position of the protrusion 12 does not match the position of the protrusion 12 when the imaging plate 10 is subjected to the front surface support, the image processing unit 81 determines that reverse setting of the imaging plate 10 cannot be identified. In other words, when the identified position of the protrusion 12 matches the position (see FIG. 73, for example) of the protrusion 12 when the imaging plate 10 is subjected to the back surface support, the image processing unit 81 determines that reverse setting of the imaging plate 10 cannot be identified.

When the image processing unit 81 determines that reverse setting of the imaging plate 10 can be identified, in other words, when the image processing unit 81 determines that the imaging plate 10 is set in reverse in step s212, step s214 is performed. The reading processing is stopped in step s214. That is to say, the light emission control unit 86 causes the light source 30 to stop the scanning processing, and the drive control unit 83 causes the driver 50 to stop moving the holder 20. Useless continuation of the scanning processing can thereby be avoided. After step s214, the above-mentioned steps s202, s205, and s206 are sequentially performed to notify the user of the alert, and discharge the imaging plate 10.

On the other hand, when the image processing unit 81 determines that reverse setting of the imaging plate 10 cannot be identified in step s212, step s213 is performed. In step s213, the image processing unit 81 determines whether the reading processing has ended (i.e., the reading processing has been completed). The image processing unit 81 can determine whether the reading processing has ended by being notified from the light emission control unit 86 that the scanning processing has ended, for example.

When the image processing unit 81 determines that the reading processing has not ended in step s213, step s212 is performed again. The reading apparatus 1 then similarly operates. On the other hand, when determining that the reading processing has ended, the image processing unit 81 determines that the imaging plate 10 is set properly in step s215. That is to say, when the reading processing ends without identification of reverse setting of the imaging plate 10 by the image processing unit 81, the image processing unit 81 determines that the imaging plate 10 is set properly. The above-mentioned steps s3 and s4 are then performed to erase the radiograph from the imaging plate 10. After step s4, steps s5, s6, and s7 may be performed as in the above-mentioned example of FIG. 18, or steps s21, s22, s5, s6, and s27 may be performed as in the above-mentioned example of FIG. 27.

In a case where the front surface specific information 1000 is shown on the front surface of the imaging plate 10, the imaging plate 10 is set properly when it is determined that the determination target image includes the image of the front surface specific information 1000 in step s212. Thus, step s212 may not be performed until the reading processing ends after it is determined that the determination target image includes the image of the front surface specific information 1000 in step s212.

In a case where the imaging plate 10 has the protrusion 12 at the peripheral edge thereof, the imaging plate 10 is set properly when negative determination is made in step s212. Thus, in a case where the imaging plate 10 has the protrusion 12 at the peripheral edge thereof, step s212 may not be performed until the reading processing ends after negative determination is once made in step s212.

Steps s205 and s206 may not be performed after step s202. When affirmative determination is made in step s212, steps s205 and s206 may be performed without performing step s202. Step s202 may be performed between steps s205 and s206, or may be performed after steps s205 and s206.

In the example of FIG. 85, when it is determined that the imaging plate 10 is set in reverse during the reading processing, the reading processing is stopped, and the user is notified of the alert. In other words, when it is determined that the imaging plate 10 is set in reverse during the scanning processing of scanning the imaging plate 10 with the excitation light L10, the scanning processing is stopped, and the user is notified of the alert. The user can thereby be notified of the alert immediately. The user can thus know reverse setting of the imaging plate 10 immediately. As a result, the user can set the imaging plate 10 again immediately.

In the example of FIG. 85, when it is determined that the imaging plate 10 is set in reverse during the reading processing, the reading processing is stopped, and the imaging plate 10 is discharged. The imaging plate 10 can thereby be discharged immediately to prompt the user to set the imaging plate 10 again.

When the plurality of pieces of back surface specific information 600 are shown on the back surface of the imaging plate 10 as in the example of FIG. 64, the determination target image including the image of the back surface specific information 600 can be acquired immediately from the result of detection of the detector 40 even if the orientation of the imaging plate 10 when the imaging plate 10 is set is not constant. For example, when the imaging plate 10 is set so that the barcode 600c is located on the scanning forward side as illustrated in FIG. 79 described above, the determination target image including the image of the barcode 600c is acquired early in the reading processing. When the imaging plate 10 is set so that the barcode 600d is located on the scanning forward side as illustrated in FIG. 80 described above, the determination target image including the image of the barcode 600d can be acquired early in the reading processing. The image processing unit 81 can thus acquire the image of the back surface specific information 600 from the result of detection of the detector 40 immediately. The image processing unit 81 can thus identify reverse setting of the imaging plate 10 immediately from the start of the reading processing. As a result, the reading processing can be stopped immediately when the imaging plate 10 is set in reverse.

When the plurality of pieces of front surface specific information 1000 are shown on the front surface of the imaging plate 10 as in the example of FIG. 67, reverse setting of the imaging plate 10 can similarly be identified immediately even if the orientation of the imaging plate 10 when the imaging plate 10 is set is not constant. In the above-mentioned step s212, the image processing unit 81 determines that the imaging plate 10 is set in reverse when the image acquired in the first half of the reading processing in the acquired whole image does not include the image of the front surface specific information 1000. In a case where the plurality of pieces of front surface specific information 1000 are arranged as in FIGs. 81 and 82, the image processing unit 81 may determine that the imaging plate 10 is set in reverse when an image acquired in the first third of the reading processing in the acquired whole image does not include the image of the front surface specific information 1000. Reverse setting of the imaging plate 10 can thereby be identified immediately from the start of the reading processing.

When the imaging plate 10 has the plurality of protrusions 12 at the peripheral edge thereof as in the examples of FIGs. 73 to 75, reverse setting of the imaging plate 10 can similarly be identified immediately even if the orientation of the imaging plate 10 when the imaging plate 10 is set is not constant.

In the example of FIG. 85, when affirmative determination is made in step s212, the reading processing may not be stopped, and step s202 may be performed after the reading processing ends.

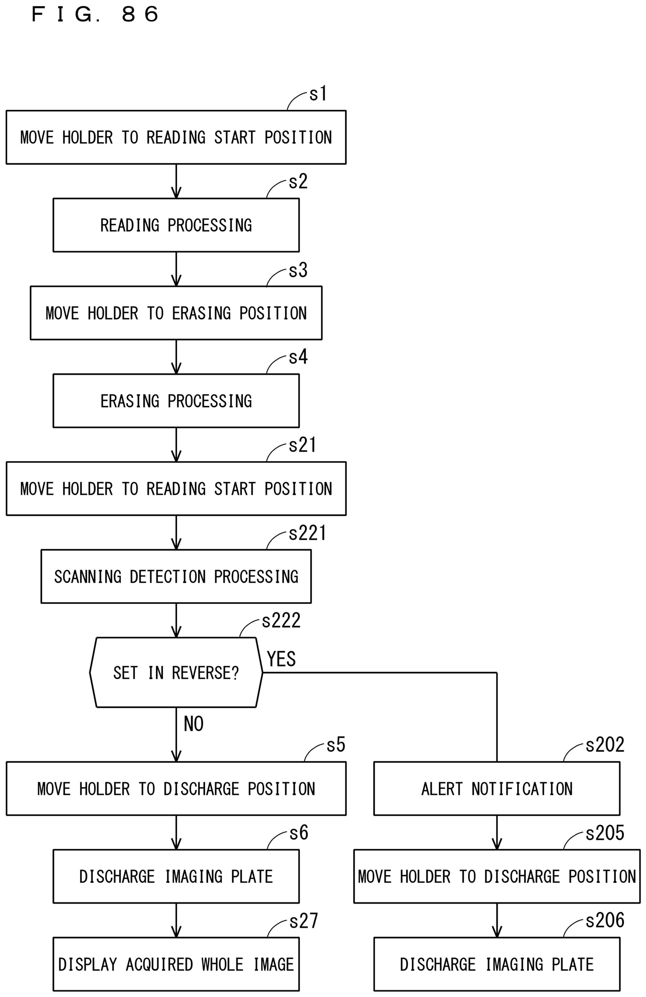

While whether the imaging plate 10 is set in reverse is determined based on the result of detection of the detector 40 in the reading processing in the above-mentioned example, whether the imaging plate 10 is set in reverse may be determined based on the result of detection of the detector 40 after the erasing processing is performed. FIG. 86 is a flowchart showing one example of operation of the reading apparatus 1 in this case. When the start button included in the operation unit 4 is operated, steps s1, s2, s3, s4, and s21 are sequentially performed as in FIG. 27 described above. Once again, step s4 is processing of irradiating the imaging plate 10 with the erasing light L3 to erase the radiograph.

Next, in step s221, the reading apparatus 1 performs scanning detection processing of performing the scanning processing and detection of light by the detector 40. The scanning detection processing is processing of scanning the imaging plate 10 having undergone the erasing processing. In the scanning detection processing, the reading apparatus 1 moves the holder 20 holding the imaging plate 10 in the subscannig direction DRs during the scanning processing. The imaging plate 10 is thus raster scanned with the excitation light L10. In the scanning detection processing, the detector 40 detects the reflected light of the excitation light L10 from the imaging plate 10 and the IP outside region during raster scanning, and outputs the erasing-time image signal as a result of detection. Step s221 is processing similar to that in step s22 in FIG. 27.

When the scanning detection processing ends, the image processing unit 81 performs, in step s222, the determination processing of determining whether the imaging plate 10 is set in reverse based on the erasing-time image signal output from the detector 40 in step s221. In other words, the image processing unit 81 performs the determination processing based on the erasing-time whole image acquired in the scanning detection processing. The determination processing in step s222 is similar to the determination processing in the above-mentioned step s201.

The acquired whole image acquired in the reading processing in step s2 herein sometimes includes a radiograph not necessary for the determination processing. Thus, when the acquired whole image acquired in step s2 is used in the determination processing, the image processing unit 81 can have difficulty determining whether the acquired whole image includes the image of the back surface specific information, the image of the front surface specific information, or the image of the protrusion 12. Thus, when the acquired whole image acquired in the reading processing is used in the determination processing, the image processing unit 81 can thus have difficulty determining whether the imaging plate 10 is set in reverse. In contrast, the erasing-time whole image acquired in step s221 does not include the radiograph not necessary for the determination processing. The image processing unit 81 can easily determine whether the imaging plate 10 is set in reverse when the erasing-time whole image is used in the determination processing. A large effect is obtained particularly when the front surface specific information is used because a radiograph that can be slipped in is erased.

When it is determined that the imaging plate 10 is set in reverse in step s222, steps s202, s205, and s206 performed in FIG. 78 described above are sequentially performed. On the other hand, when it is determined that the imaging plate 10 is not set in reverse in step s222, steps s5, s6, and s27 performed in FIG. 27 described above are sequentially performed.

Steps s205 and s206 may not be performed after step s202. When affirmative determination is made in step s222, steps s205 and 206 may be performed without performing step s202. Step s202 may be performed between steps s205 and s206, or may be performed after steps s205 and s206.

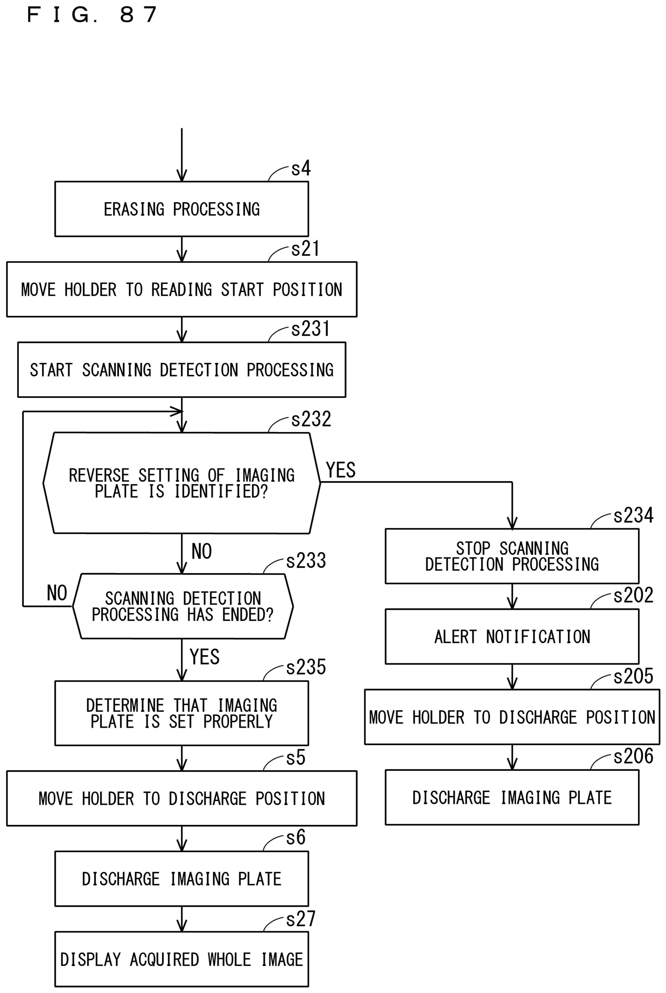

While the determination processing is performed after the scanning detection processing ends in the example of FIG. 86, the scanning detection processing and the determination processing may be performed in parallel. FIG. 87 is a flowchart showing one example of operation of the reading apparatus 1 in this case. After the start button included in the operation unit 4 is operated, steps s1, s2, and s3 are performed, and then steps s4 and s21 are performed.

After step s21, the scanning detection processing starts in step s231. When the scanning detection processing starts, the image processing unit 81 determines whether reverse setting of the imaging plate 10 can be identified based on luminance values sequentially output from the detector 40 in response to raster scanning in step s232. Processing in step s232 is similar to that in the above-mentioned step s212.

When the image processing unit 81 determines that reverse setting of the imaging plate 10 can be identified, in other words, when the image processing unit 81 determines that the imaging plate 10 is set in reverse in step s232, step s234 is performed. The scanning detection processing is stopped in step s234. The scanning processing and movement of the holder 20 are thereby stopped. Steps s202, s205, and s206 are then sequentially performed.

On the other hand, when the image processing unit 81 determines that reverse setting of the imaging plate 10 cannot be identified in step s232, step s233 is performed. In step s233, the image processing unit 81 determines whether the scanning detection processing has ended. The image processing unit 81 can determine whether the scanning detection processing has ended by being notified from the light emission control unit 86 that the scanning processing has ended, for example.

When the image processing unit 81 determines that the scanning detection processing has not ended in step s233, step s232 is performed again. The reading apparatus 1 then similarly operates. On the other hand, when determining that the scanning detection processing has ended, the image processing unit 81 determines that the imaging plate 10 is set properly in step s235. Steps s5, s6, and s27 are then sequentially performed.

Steps s205 and s206 may not be performed after step s202. When affirmative determination is made in step s232, steps s205 and s206 may be performed without performing step s202. Step s202 may be performed between steps s205 and s206, or may be performed after steps s205 and s206.

In a case where the front surface specific information 1000 is shown on the front surface of the imaging plate 10, and the erasing-time whole image is not required to be acquired, the scanning detection processing may be stopped, and step s235 may be performed when it is determined that the determination target image includes the image of the front surface specific information 1000 in step s232. In a case where the imaging plate 10 has the protrusion 12 at the peripheral edge thereof, and the erasing-time whole image is not required to be acquired, the scanning detection processing may be stopped, and step s235 may be performed when negative determination is once made in step s232.

In the example of FIG. 87, when it is determined that the imaging plate 10 is set in reverse during the scanning processing, the scanning processing is stopped, and the user is notified of the alert as in the example of FIG. 85. The user can thereby be notified of the alert immediately. In the example of FIG. 87, when it is determined that the imaging plate is set in reverse during the scanning processing, the scanning processing is stopped, and the imaging plate 10 is discharged. The imaging plate 10 can thereby be discharged immediately.

In the example of FIG. 87, when affirmative determination is made in step s232, the scanning detection processing may not be stopped, and step s202 may be performed after the scanning detection processing ends.

### <Evaluation of Image Quality of Radiograph>

While the object irradiated with the excitation light L10 is the imaging plate 10 in the above-mentioned example, the object irradiated with the excitation light L10 may be an object other than the imaging plate 10. The object irradiated with the excitation light L10 may be an evaluation member having, on the surface thereof, an evaluation pattern for evaluation of an image quality of the radiograph read from the imaging plate 10 by the reading apparatus 1, for example.

The evaluation member has a similar size and a similar shape to those of the imaging plate 10, and is inserted into the reading apparatus 1 through the inlet 2a of the reading apparatus 1, for example. The evaluation member inserted into the reading apparatus 1 is held by the holder 20 as with the imaging plate 10. The radiograph formation layer 11 may be formed, or may not be formed in the evaluation member. When the radiograph formation layer 11 is formed in the evaluation member, energy of radiation is not stored in the radiograph formation layer 11.

When the holder 20 holds the evaluation member, the above-mentioned irradiation object 1200 includes the holder 20 and the evaluation member held by the holder 20. A region of the support side main surface 1200a of the irradiation object 1200 in which an image formed by the acted light L2 therefrom is an image of the evaluation member is hereinafter also referred to as an evaluation member image region, or is hereinafter simply referred to as a member image region. It can be said that the evaluation member image region is an evaluation member presence region of the support side main surface 1200a in which the evaluation member is present. A region excluding the member image region in the detection range R110 is also referred to as an evaluation member image region outside region, or is simply referred to as a member image region outside region. The member image region corresponds to the IP image region R100, and the evaluation member image region outside region (i.e., the member image region outside region) corresponds to the IP image region outside region R130.

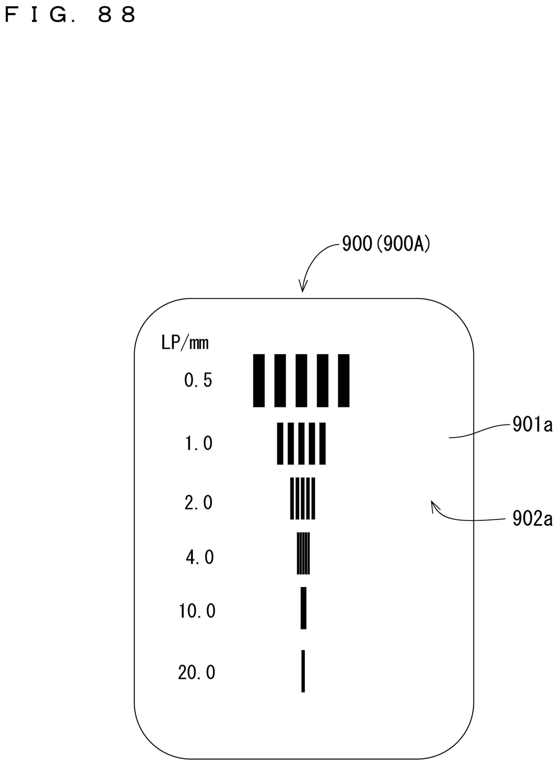

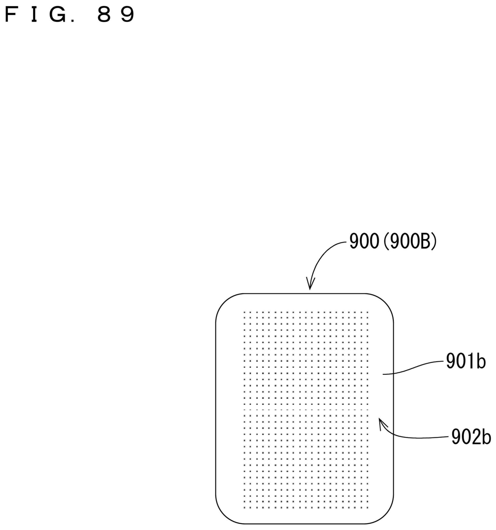

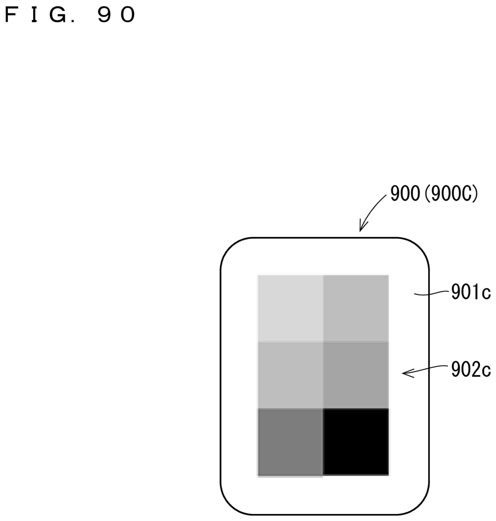

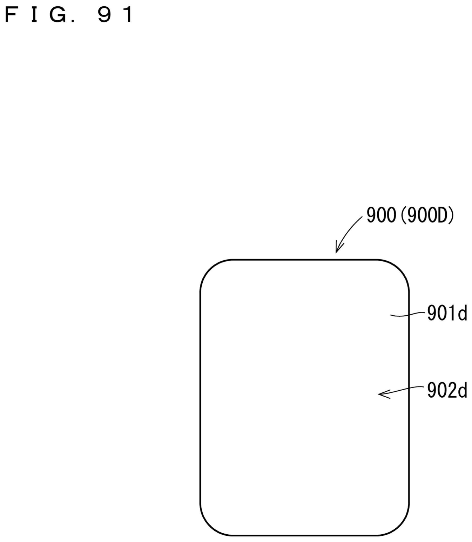

FIGs. 88 to 91 are schematic diagrams each showing one example of an evaluation member 900. FIG. 88 shows the evaluation member 900 (also referred to as a resolution evaluation member 900A) for evaluating resolution of the detected radiograph. FIG. 89 shows the evaluation member 900 (also referred to as a geometric accuracy evaluation member 900B) for evaluating geometric accuracy of the detected radiograph. FIG. 90 shows the evaluation member 900 (also referred to as a contrast evaluation member 900C) for evaluating contrast of the detected radiograph. FIG. 91 shows the evaluation member 900 (also referred to as an artifact evaluation member 900D) for evaluating an artifact of the detected radiograph.

As shown in FIG. 88, a resolution evaluation pattern 902a for evaluating the resolution of the detected radiograph is shown on a front surface 901a of the resolution evaluation member 900A. A line pair chart is used as the resolution evaluation pattern 902a, for example. In the resolution evaluation pattern 902a, a plurality of sets of lines are arranged so that widths of and spacing between lines differ among the plurality of sets of lines.

As shown in FIG. 89, a geometric accuracy evaluation pattern 902b for evaluating the geometric accuracy of the detected radiograph is shown on a front surface 901b of the geometric accuracy evaluation member 900B. A pattern of a plurality of small points in a grid is used as the geometric accuracy evaluation pattern 902b, for example. The plurality of small points may be arranged to form a square with right angle corners as a whole. A plurality of rows forming the grid may be arranged in parallel, and a plurality of columns forming the grid may be arranged in parallel.

As shown in FIG. 90, a contrast evaluation pattern 902c for evaluating the contrast of the detected radiograph is shown on a front surface 901c of the contrast evaluation member 900C. A pattern of a plurality of squares differing in brightness (i.e., density) expressed in grayscale is used as the contrast evaluation pattern 902c, for example.

As shown in FIG. 91, an artifact evaluation pattern 902d for evaluating the artifact of the detected radiograph is shown on a front surface 901d of the artifact evaluation member 900D. A monochrome uniform pattern is used as the artifact evaluation pattern 902d, for example. For example, a plain white pattern is used as the artifact evaluation pattern 902d.

In the reading apparatus 1, the evaluation member 900 is held by the holder 20 so that the evaluation pattern thereof is directed toward the light source 30. In other words, the evaluation member 900 is held by the holder 20 so that the front surface thereof is directed toward the light source 30. The evaluation pattern of the evaluation member 900 is thereby irradiated with the excitation light L10. The detector 40 detects the reflected light of the excitation light L10 from the front surface of the evaluation member 900 (i.e., the member image region) and the member image region outside region, and outputs an image signal as a result of detection.

An image signal as a result of detection of reflected light of light when the evaluation member 900 is held by the holder 20 is hereinafter referred to as an evaluation image signal. A whole image based on the evaluation image signal is referred to as an evaluation whole image. The evaluation pattern appears in the evaluation whole image, and the evaluation whole image includes a reflected light image (hereinafter referred to as an evaluation pattern image) of the evaluation pattern. The evaluation whole image does not include the radiograph. The evaluation whole image includes only the reflected light image.

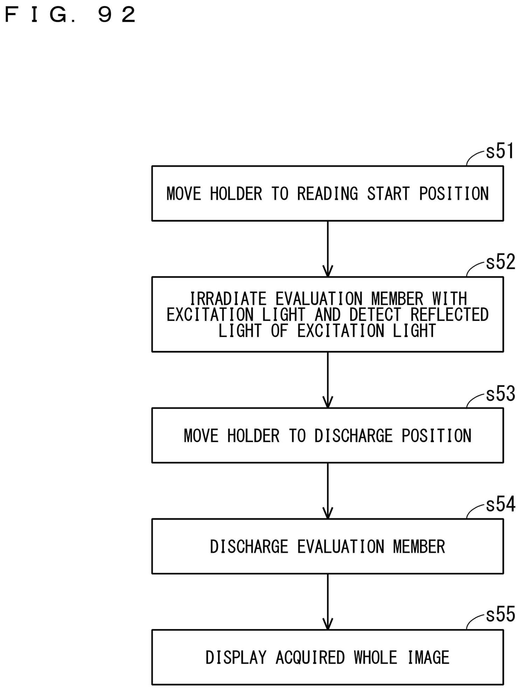

FIG. 92 is a flowchart showing one example of operation of the reading apparatus 1 when the reading apparatus 1 acquires the evaluation whole image in which the evaluation pattern appears. When the evaluation member 900 inserted through the inlet 2a of the housing 2 is held by the holder 20, and the operation unit 4 receives a predetermined operation from the user, step s51 is performed as shown in FIG. 92. In step s51, the driver 50 moves the holder 20 to the reading start position through control performed by the drive control unit 83. Step s52 is performed after step s51.

In step s52, the light source 30 irradiates the front surface of the evaluation member 900 and the outside region with the excitation light L10. The detector 40 detects the reflected light of the excitation light L10 from the front surface of the evaluation member 900 and the outside region, and outputs the evaluation image signal as a result of detection. The evaluation image signal is a gray-scale image signal.

After step s52, the driver 50 moves the holder 20 to the discharge position through control performed by the drive control unit 83 in step s53. Next, in step s54, the evaluation member 900 is discharged to the outlet 2b of the housing 2. In step s55, the display control unit 82 causes the display 3 to display the evaluation whole image in grayscale based on the evaluation image signal, for example. When the resolution evaluation member 900A is inserted into the reading apparatus 1, the evaluation whole image including an image of the resolution evaluation pattern 902a is displayed in step s55. When the geometric accuracy evaluation member 900B is inserted into the reading apparatus 1, the evaluation whole image including an image of the geometric accuracy evaluation pattern 902b is displayed in step s55. When the contrast evaluation member 900C is inserted into the reading apparatus 1, the evaluation whole image including an image of the contrast evaluation pattern 902c is displayed in step s55. When the artifact evaluation member 900D is inserted into the reading apparatus 1, the evaluation whole image including an image of the artifact evaluation pattern 902d is displayed in step s55. Step s55 may be performed at any time after step s52.

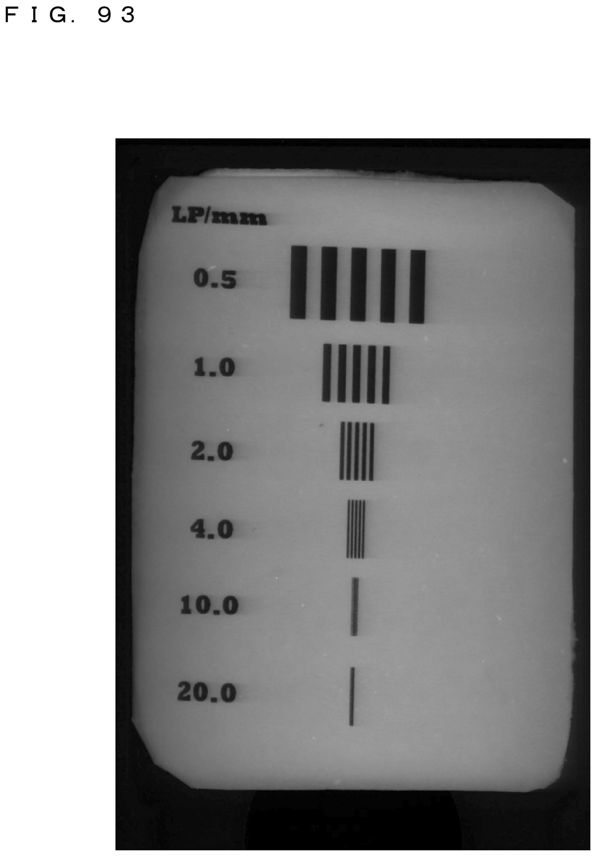

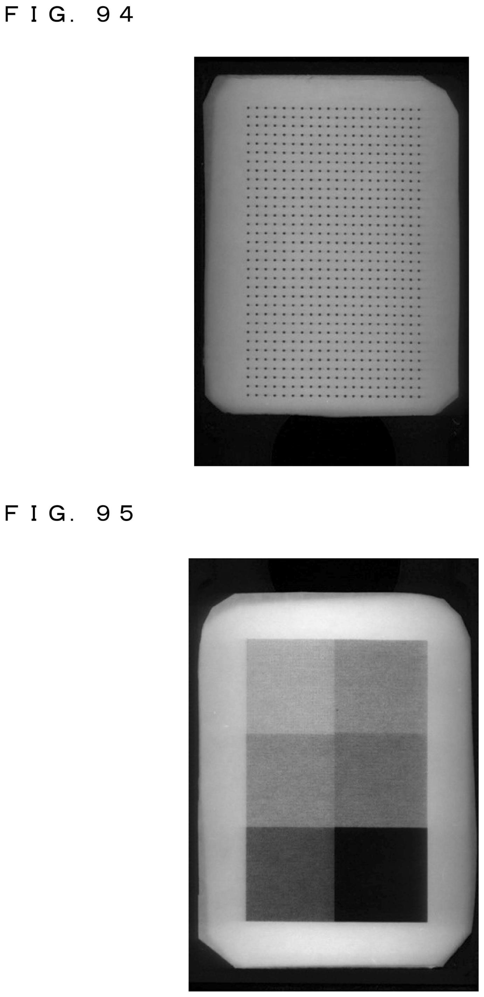

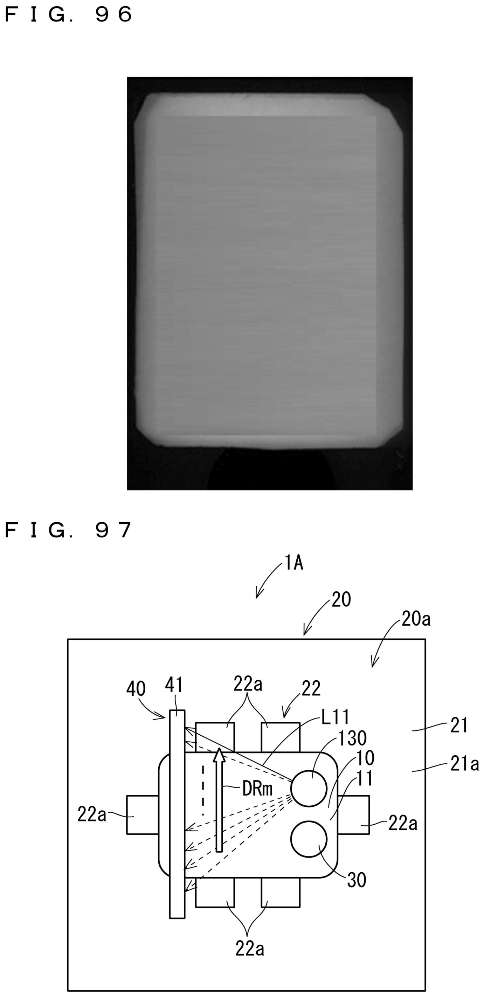

FIG. 93 is a schematic diagram showing one example of the evaluation whole image including the image of the resolution evaluation pattern 902a. FIG. 94 is a schematic diagram showing one example of the evaluation whole image including the image of the geometric accuracy evaluation pattern 902b. FIG. 95 is a schematic diagram showing one example of the evaluation whole image including the image of the contrast evaluation pattern 902c. FIG. 96 is a schematic diagram showing one example of the evaluation whole image including the image of the artifact evaluation pattern 902d. The display 3 displays the evaluation whole image in grayscale in step s55 as shown in FIGs. 93 to 96, for example.

The user can evaluate the image quality of the detected radiograph based on the evaluation pattern image included in the evaluation whole image displayed by the display 3. That is to say, the user can evaluate the image quality of the detected radiograph based on the evaluation pattern image displayed by the display 3. For example, the user evaluates the resolution of the detected radiograph based on the image of the resolution evaluation pattern 902a displayed by the display 3. The resolution may be evaluated by evaluating whether lines can be detected even when the lines have smaller widths or whether lines can be detected independently of one another even when the spacing between lines is reduced, for example. The user evaluates the geometric accuracy of the detected radiograph based on the image of the geometric accuracy evaluation pattern 902b displayed by the display 3. The geometric accuracy may be evaluated based on whether arrangement of the small points is detected to be faithfully reproducible, for example. The user evaluates the contrast of the detected radiograph based on the image of the contrast evaluation pattern 902c displayed by the display 3. The user evaluates the artifact of the detected radiograph based on the image of the artifact evaluation pattern 902d displayed by the display 3.

As described above, in this example, the light source 30 and the detector 40 to acquire the detected radiograph are used to acquire the evaluation image signal as a result of detection of the reflected light of the excitation light L10 from the front surface of the evaluation member 900. The image quality of the detected radiograph can thereby properly be evaluated based on the evaluation pattern image included in the reflected light image based on the evaluation image signal. The image quality of the detected radiograph can thereby properly be evaluated without using an expensive evaluation phantom to record the image of the evaluation pattern on the radiograph formation layer 11 of the imaging plate 10.

The evaluation member 900 may be formed of paper, resin, or metal, for example. The evaluation pattern may be formed by being printed on the front surface of the evaluation member 900. At least one of the resolution evaluation pattern 902a and the geometric accuracy evaluation pattern 902b may be irregularities in the front surface of the evaluation member 900. When the evaluation pattern is printed on the front surface of the evaluation member 900, the evaluation pattern having high accuracy can be acquired by printing technology. The image quality of the detected radiograph can thereby properly be evaluated. The evaluation member 900 may be formed of printed paper on which the evaluation pattern is printed. In this case, the image quality of the detected radiograph can be evaluated using an inexpensive evaluation member 900. The evaluation member 900 may be formed of cardboard.

According to the present embodiment, the light source 30 irradiates the imaging plate 10 with the excitation light L10. The detector 40 functions as a first detector that detects the emitted light L2 by the excitation light L10 from the imaging plate 10, and outputs a first image signal as a result of detection of the emitted light L2. The light source 30 irradiates the evaluation member 900 having, on the surface thereof, the evaluation pattern to evaluate the image quality of the detected radiograph based on the first image signal with the excitation light L10. The detector 40 functions as a second detector that detects the reflected light of the excitation light L10 from the surface of the evaluation member 900, and outputs a second image signal as a result of detection of the reflected light.

While the detector 40 combines the first detector and the second detector in the present embodiment, the first detector and the second detector may be provided separately to detect the emitted light using the first detector and detect the reflected light using the second detector as will be described below.

### <Another Example of Configuration of Reading Apparatus>

While the holder 20 is moved in the above-mentioned example, the holder 20 may not be moved. In this case, the light source 30, the detector 40, and the erasing light source 70 are moved with the holder 20 being stopped to achieve processing similar to the above-mentioned processing in the reading apparatus 1. The light source 30, the detector 40, the erasing light source 70, and the holder 20 may be moved.

The reading apparatus 1 may include a plurality of light sources. FIG. 97 is a schematic diagram illustrating an example of a configuration of the reading apparatus 1 (also referred to as a reading apparatus 1A) including two light sources.

As illustrated in FIG. 97, the reading apparatus 1A includes the above-mentioned light source 30 and a light source 130 different from the light source 30. The light source 130 can irradiate the imaging plate 10 or the evaluation member 900 held by the holder 20 with irradiation light L11. The light source 130 has a similar configuration to the light source 30, for example, and can perform scanning with the irradiation light L11 in the main scanning direction DRm. The irradiation light L11 is visible laser light, for example. The irradiation light L11 may have the same wavelength as the excitation light L10, or may have a different wavelength from the excitation light L10. The light source 130 is controlled by the light emission control unit 86 as with the light source 30. It can be said that the irradiation light L11 is the acting light L1.

The light source 130 may be used in step s22 in FIG. 27 described above, for example. In this case, not the light source 30 but the light source 130 irradiates the front surface (i.e., the IP image region) of the erased imaging plate 10 and the IP image region outside region with the irradiation light L11 in step s22. In step s22, the light source 130 repeatedly performs processing of scanning the imaging plate 10 and the IP image region outside region with the irradiation light L11 in the main scanning direction DRm through control performed by the light emission control unit 86 as with the light source 30. On the other hand, in step s22, the driver 50 moves the holder 20 holding the imaging plate 10 in the subscannig direction DRs as in the above-mentioned reading processing. Processing of performing scanning with the irradiation light L11 in the main scanning direction DRm is repeatedly performed while the holder 20 is moved in the subscannig direction DRs to raster scan the imaging plate 10 and the IP image region outside region with the irradiation light L11 as with the excitation light L10. In step s22, while the imaging plate 10 is raster scanned with the irradiation light L 11, the detector 40 detects the reflected light of the irradiation light L11 from the imaging plate 10, and outputs the erasing-time image signal as a result of detection. The erasing-time whole image based on the erasing-time image signal includes the IP whole reflected light image and the IP image region outside region image, and does not include the radiograph as with the above-mentioned erasing-time whole image based on detection of the reflected light of the excitation light L10. The erasing-time whole image based on detection of the reflected light of the irradiation light L11 is displayed in step s27 in FIG. 27. The reading apparatus 1A can use the erasing-time whole image based on detection of the reflected light of the irradiation light L11 as with the erasing-time whole image based on detection of the reflected light of the excitation light L10. The reading apparatus 1A may identify the IP tilt angle, or may identify the IP size based on the erasing-time whole image based on detection of the reflected light of the irradiation light L11, for example. The reflected light of the irradiation light L11 is included in the acted light L2.

In a case where the reading apparatus 1A performs processing in FIG. 27 when the imaging plate 10 is inserted into the reading apparatus 1A with the back surface thereof facing forward, the back insertion-time whole image based on detection of the reflected light of the irradiation light L11 is acquired in step s22 as with the above-mentioned back insertion-time whole image based on detection of the reflected light of the excitation light L10. The user can identify insertion of the imaging plate 10 into the reading apparatus 1A with the back surface thereof facing forward based on the back insertion-time whole image displayed by the display 3 of the reading apparatus 1A.

The light source 130 may be used in step s52 in FIG. 92 described above, for example. In this case, in the reading apparatus 1A, not the light source 30 but the light source 130 irradiates the front surface of the evaluation member 900 and the member image region outside region with the irradiation light L11 in step s52. Operation of the reading apparatus 1A in step s52 is similar to operation of the reading apparatus 1A in the above-mentioned step s22. In step s52, while the evaluation member 900 and the member image region outside region are raster scanned with the irradiation light L11, the detector 40 detects the reflected light of the irradiation light L11 from the evaluation member 900 and the outside region, and outputs the evaluation image signal as a result of detection. The evaluation whole image based on the evaluation image signal includes the evaluation pattern image, and does not include the radiograph as with the above-mentioned evaluation whole image based on detection of the reflected light of the excitation light L10. In step s55 in FIG. 92, the evaluation whole image based on detection of the reflected light of the irradiation light L11 is displayed by the display 3 based on the evaluation image signal acquired in step s52. The user can evaluate the image quality of the detected radiograph based on the evaluation pattern image included in the evaluation whole image displayed by the display 3 of the reading apparatus 1A.

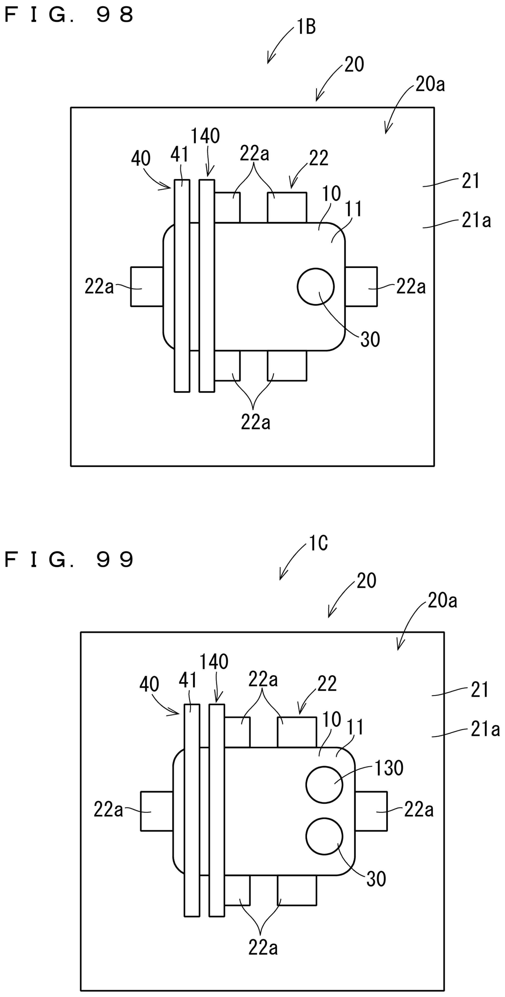

The reading apparatus 1 may include a plurality of detectors. FIG. 98 is a schematic diagram illustrating an example of a configuration of the reading apparatus 1 (also referred to as a reading apparatus 1B) including two detectors and one light source.

As illustrated in FIG. 98, the reading apparatus 1B includes the above-mentioned detector 40 and a detector 140 different from the detector 40. The reading apparatus 1B includes the light source 30, and does not include the light source 130. The detector 140 has a similar configuration to the detector 40, for example. The detector 140 can detect the emitted light L2 from the imaging plate 10 as with the detector 40. The detector 140 can also detect the reflected light of the excitation light L10 from the imaging plate 10 or the evaluation member 900 and the reflected light of the excitation light L10 from the IP image region outside region or the member image region outside region as with the detector 40.

The detector 140 may be used in step s22 in FIG. 27 described above, for example. In this case, in the reading apparatus 1B, while the light source 30 raster scans the front surface of the erased imaging plate 10 and the IP image region outside region, the detector 140 detects the reflected light of the excitation light L10 from the imaging plate 10 and the IP image region outside region, and outputs the erasing-time image signal as a result of detection in step s22. The erasing-time whole image based on the erasing-time image signal includes the IP whole reflected light image and the IP image region outside region image, and does not include the radiograph as with the erasing-time whole image described so far. The reading apparatus 1B can perform various types of processing, such as identification of the IP tilt angle, based on the erasing-time whole image acquired in step s22.

In a case where the reading apparatus 1B performs processing in FIG. 27 when the imaging plate 10 is inserted into the reading apparatus 1B with the back surface thereof facing forward, the back insertion-time whole image in which the back surface of the imaging plate 10 appears is acquired in step s22 as with the back insertion-time whole image described so far.

The detector 140 may be used in step s52 in FIG. 92 described above, for example. In this case, in the reading apparatus 1B, while the light source 30 raster scans the front surface of the evaluation member 900 and the member image region outside region, the detector 140 detects the reflected light of the excitation light L10 from the evaluation member 900 and the member image region outside region, and outputs the evaluation image signal as a result of detection in step s52. The evaluation whole image based on the evaluation image signal includes the evaluation pattern image, and does not include the radiograph as with the evaluation whole image described so far.

The reading apparatus 1 may include a plurality of detectors and a plurality of light sources. FIG. 99 is a schematic diagram illustrating an example of a configuration of the reading apparatus 1 (also referred to as a reading apparatus 1C) including the detectors 40 and 140 and the light sources 30 and 130.

The reading apparatus 1C may use the detector 140 and the light source 130 in step s22 in FIG. 27 described above, for example. In this case, in the reading apparatus 1C, while the light source 130 raster scans the front surface of the erased imaging plate 10 and the IP image region outside region, the detector 140 detects the reflected light of the excitation light L10 from the imaging plate 10 and the IP image region outside region, and outputs the erasing-time image signal as a result of detection in step s22. The erasing-time whole image based on the erasing-time image signal includes the IP whole reflected light image and the IP image region outside region image, and does not include the radiograph as with the erasing-time whole image described so far. In a case where the reading apparatus 1C performs processing in FIG. 27 when the imaging plate 10 is inserted into the reading apparatus 1C with the back surface thereof facing forward, the acquired whole image in which the back surface of the imaging plate 10 appears is acquired in step s22.

The reading apparatus 1C may use the detector 140 and the light source 130 in step s52 in FIG. 92 described above, for example. In this case, in the reading apparatus 1C, while the light source 130 raster scans the front surface of the evaluation member 900 and the member image region outside region, the detector 140 detects the reflected light of the excitation light L10 from the evaluation member 900 and the member image region outside region, and outputs the evaluation image signal as a result of detection in step s52. The evaluation whole image based on the evaluation image signal includes the evaluation pattern image, and does not include the radiograph as with the evaluation whole image described so far.

In each of the reading apparatuses 1A and 1C, the light source 130 may output light other than visible light as the irradiation light L11. For example, the irradiation light L11 may be infrared rays, or may be ultraviolet rays. In this case, a detector that can detect infrared rays or ultraviolet rays is used as each of the detector 40 and the detector 140. The irradiation light L11 output from the light source 130 may be light that cannot excite the radiograph formation layer 11 of the imaging plate 10. That is to say, the emitted light L2 may not be output from the radiograph formation layer 11 even when the radiograph formation layer 11 on which the radiograph is recorded is irradiated with the irradiation light L11.

In each of the reading apparatuses 1A and 1C, when the irradiation light L11 does not excite the radiograph formation layer 11, the determination processing of determining whether the imaging plate 10 is set in reverse may be performed before the reading processing. In this case, the image processing unit 81 can determine whether the imaging plate 10 is set in reverse based on the image signal output from the detector 40 as a result of detection of the reflected light of the irradiation light L11 from the imaging plate 10.

In each of the reading apparatuses 1B and 1C, the detector 140 that detects the reflected light of the irradiation light L11 may include a CCD sensor or a CMOS sensor used in a camera, for example. CCD is an abbreviation for "charge coupled device", and CMOS is an abbreviation for "complementary metal oxide semiconductor".

In the reading apparatus 1C, the light source 130 may irradiate the whole range of the imaging plate 10 or the evaluation member 900 with the irradiation light L11 in a single irradiation as with the erasing light source 70 without performing scanning with the irradiation light L11. In this case, the detector 140 may include the CCD sensor or the CMOS sensor used in the camera, for example.

In each of the reading apparatuses 1B and 1C, the detector 40 may not be able to detect the reflected light of the excitation light L10. In this case, the optical filter 42 of the detector 40 may not transmit the excitation light L10. In the reading apparatus 1C, the detector 40 may not be able to detect the reflected light of the irradiation light L11. In this case, the optical filter 42 of the detector 40 may not transmit the irradiation light L11.

In the light emission-time whole image acquired when the detector 40 does not detect the reflected light of the excitation light L10, the luminance value for the unexposed region image and the luminance value for the IP image region outside region image have similar values when the imaging plate 10 includes the unexposed portion. When the imaging plate 10 includes the unexposed portion, the image processing unit 81 has difficulty identifying the IP tilt angle and the IP size based on the light emission-time whole image by a method using the binarized image as described above. Even when the imaging plate 10 includes the unexposed portion, however, the image processing unit 81 can properly identify the IP tilt angle and the IP size as described above based on the erasing-time whole image based on the erasing-time image signal output from the detector 140.

In each of the reading apparatuses 1B and 1C, the detector 140 may not be able to detect the photostimulated light L5. In this case, each of the reading apparatuses 1B and 1C can acquire the IP whole reflected light image without erasing the radiograph from the imaging plate 10. For example, the reading apparatus 1B may simultaneously operate the detectors 40 and 140 in the reading processing in the above-mentioned step s2. In this case, detection of the excited region light L20 by the detector 40 and detection of the reflected light of the excitation light L10 from the imaging plate 10 by the detector 140 are performed in parallel. The whole image based on the image signal output from the detector 140 is similar to the erasing-time whole image, and includes the IP whole reflected light image and the IP image region outside region image, and does not include the radiograph. The reading apparatus 1C may simultaneously operate the detectors 40 and 140 while simultaneously operating the light sources 30 and 130 in the reading processing in the above-mentioned step s2. In this case, detection of the excited region light L20 by the detector 40 and detection of the reflected light of the irradiation light L11 from the imaging plate 10 by the detector 140 are performed in parallel. Also in this case, the whole image based on the image signal output from the detector 140 is similar to the erasing-time whole image, and includes the IP whole reflected light image and the IP image region outside region image, and does not include the radiograph. As described above, acquisition of not only the radiograph based on detection of the excited region light L20 but also the reflected light image based on detection of the reflected light of the excitation light L10 or the irradiation light L11 eliminates the need for processing in steps s21 and s22 in FIG. 27. That is to say, the radiograph based on detection of the excited region light L20 and the reflected light image based on detection of the reflected light of the excitation light L10 or the irradiation light L11 can be acquired during the above-mentioned series of processes in FIG. 18. Operation of the reading apparatus 1 can thereby be simplified.

In the example of FIGs. 2 to 6 or the example of FIG. 98 described above, the light source to read the radiograph from the imaging plate 10 and the light source to acquire the reflected light image of the imaging plate 10 are the same. In other words, the light source to read the radiograph from the imaging plate 10 functions as the light source to acquire the reflected light image of the imaging plate 10. A configuration of the reading apparatus 1 can thereby be simplified.

It can be said that, in the example of FIGs. 2 to 6 or the example of FIG. 98 described above, light to acquire the reflected light image of the imaging plate 10 functions as excitation light to read the radiograph from the imaging plate 10. This eliminates the need for a light source to irradiate the imaging plate 10 with the excitation light separately from a light source to irradiate the imaging plate 10 with light to acquire the reflected light image of the imaging plate 10. The configuration of the reading apparatus 1 can thereby be simplified.

In the example of FIGs. 2 to 6 or the example of FIG. 97 described above, the detector to detect the emitted light L2 and the detector to detect the reflected light of light are the same. In other words, the detector to detect the emitted light L2 functions as the detector to detect the reflected light of light. The configuration of the reading apparatus 1 can thereby be simplified.

It can be said that, in the example of FIGs. 2 to 6 or the example of FIG. 97 described above, the detector to detect the reflected light of light also detects the emitted light L2. This eliminates the need for the detector to detect the emitted light L2 separately from the detector to detect the reflected light of light. The configuration of the reading apparatus 1 can thereby be simplified.

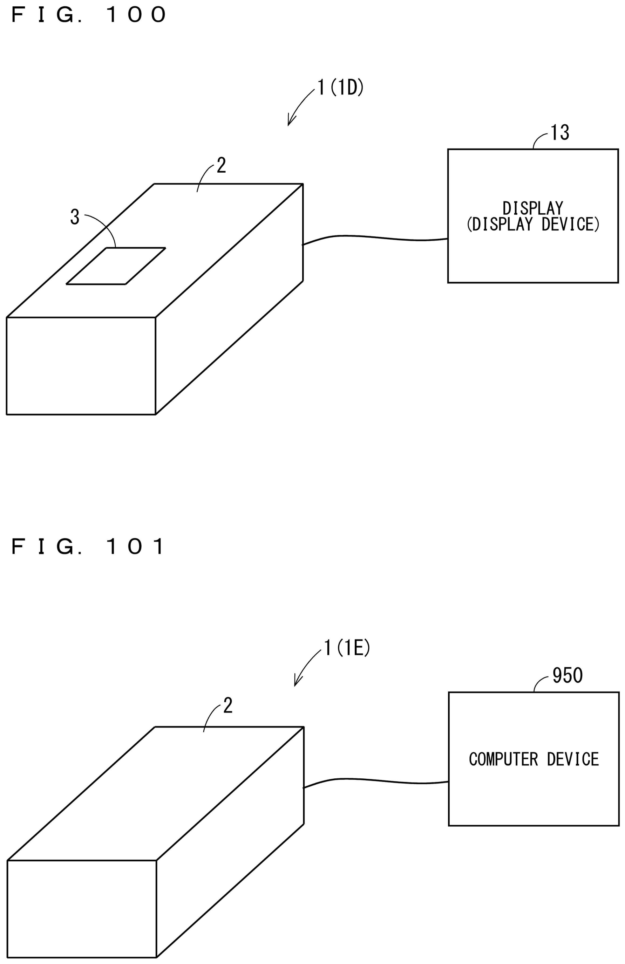

While a plurality of components other than the AC adapter 5 are integrated in the housing 2 in the reading apparatus 1 in the above-mentioned example, they may not be integrated. For example, the reading apparatus 1 may include a display 13 located outside the housing 2 separately from or in place of the display 3 provided to the housing 2. FIG. 100 is a schematic diagram illustrating one example of a configuration of the reading apparatus 1 (also referred to as a reading apparatus 1D) including the display 13 outside the display surface of the housing 2. It can be said that the display 13 is a display device 13.

The display 13 is a liquid crystal display or an organic EL display, and can display various pieces of information, such as characters, symbols, graphics, and images, for example. The display 13 is controlled by the display control unit 82 of the controller 80 within the housing 2. The display control unit 82 can control the display 13 via the interface 95 within the housing 2, for example. Communication between the interface 95 and the display 13 may conform to USB, DisplayPort, or HDMI (High-Definition Multimedia Interface)^{®}. The interface 95 may be connected to the display 13 by wire or wirelessly. The display 13 may display the acquired whole image, or may display the cutout image. While the display 3 is provided to the housing 2 of the reading apparatus 1D in the example of FIG. 100, the display 3 may not be provided. The reading apparatus 1D may include a plurality of light sources, or may include a plurality of detectors as shown in FIGs. 97 to 99.

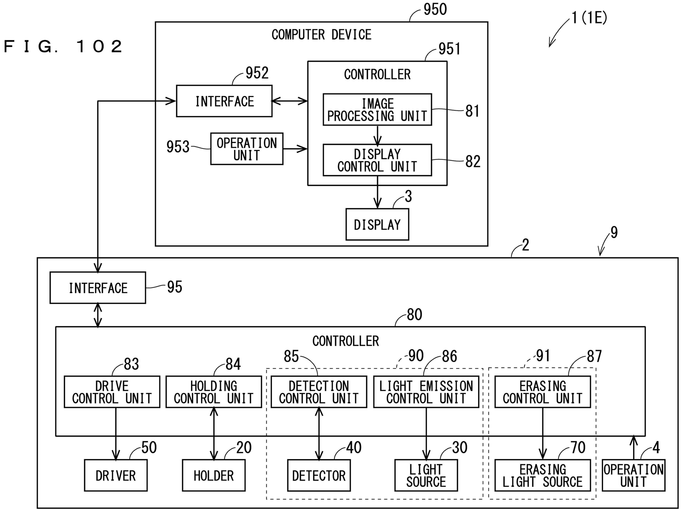

FIGs. 101 and 102 are schematic diagrams each showing another example of the configuration of the reading apparatus 1. In the reading apparatus 1 (also referred to as a reading apparatus 1E) shown in each of FIGs. 101 and 102, a computer device 950 having one or more functions of the reading apparatus 1E is provided outside the housing 2. As shown in FIG. 102, the computer device 950 includes the display 3, the image processing unit 81, and the display control unit 82 described above, for example. The computer device 950 may be a personal computer (also referred to as a general-purpose computer), for example. In this case, the computer device 950 may be a notebook computer device, or may be a desktop computer device. In the reading apparatus 1E, the housing 2, the plurality of components integrated by the housing 2, and the AC adapter 5 are hereinafter also collectively referred to as a reading apparatus main body 9.

The computer device 950 can communicate with the reading apparatus main body 9. The computer device 950 includes a controller 951 including the image processing unit 81 and the display control unit 82 and an interface 952 that communicates with the reading apparatus main body 9, for example. The computer device 950 also includes an operation unit 953 that receives an operation from the user.

The controller 951 can manage operation of the computer device 950 in an integrated manner, and can be said to be a control circuit. The controller 951 can control the display 3 and the interface 952, for example. The controller 951 can perform processing responsive to a user operation received by the operation unit 953.

The controller 951 includes at least one processor and a storage, and can be said to be a computer device, for example. The at least one processor of the controller 951 may include a CPU, or may include a processor other than the CPU. The at least one processor of the controller 951 executes a program in the storage (also referred to as a storage circuit) to perform various functions. The at least one processor of the controller 951 executes the program in the storage to form the image processing unit 81 and the display control unit 82 described above as the functional blocks.

The operation unit 953 includes a keyboard and a mouse, for example. The operation unit 953 may include a touch sensor that detects a touch operation of the user. When the operation unit 953 includes the touch sensor, the touch sensor and the display 3 may constitute the touch panel display having the display function and the touch detection function.

The interface 952 can communicate with the interface 95 of the reading apparatus main body 9. Communication between the interface 952 and the interface 95 of the reading apparatus main body 9 may conform to Ethernet, USB, WiFi, or other standards. The interface 952 may communicate with the interface 95 by wire or wirelessly. It can be said that the interface 952 is an interface circuit, a communication unit, or a communication circuit. The controller 951 of the computer device 950 and the controller 80 of the reading apparatus main body 9 can exchange information via the interface 952 and the interface 95.

In the reading apparatus 1E, the controller 951 of the computer device 950 and the controller 80 of the reading apparatus main body 9 cooperate with each other to perform the above-mentioned various types of processing performed by the controller 80. In the reading apparatus 1E, the detection control unit 85 outputs the image signal output from the detector 40 to the interface 95. The interface 95 outputs the input image signal to the interface 952. The interface 952 inputs the input image signal into the controller 951. The image processing unit 81 performs the above-mentioned image processing on the image signal input into the controller 951. The image processing unit 81 performs the tilt angle identification processing, the size identification processing, and the cutting-out processing described above based on the image signal after the image processing. The display control unit 82 causes the display 3 to display the acquired whole image based on the image signal after the image processing, for example.

The operation unit 953 of the computer device 950 may receive at least one or more of a plurality of user operations received by the operation unit 4 of the reading apparatus main body 9. For example, the operation unit 953 may receive a user operation to provide instructions to start the series of processes in FIG. 18, may receive a user operation to provide instructions to start the series of processes in FIG. 26, may receive a user operation to provide instructions to start the series of processes in FIG. 27, or may receive a user operation to provide instructions to start the series of processes in FIG. 74. In this case, the controller 951 notifies the reading apparatus main body 9 of the user operation received by the operation unit 953 via the interface 952. In the reading apparatus main body 9, the controller 80 receives the notification from the controller 951 via the interface 95, and the operation unit 953 performs processing responsive to the received user operation.

The operation unit 953 may receive a user operation not received by the operation unit 4, or the operation unit 4 may receive a user operation not received by the operation unit 953. When the user operation received by the operation unit 953 and the user operation received by the operation unit 4 compete against each other, processing responsive to the user operation received by the reading apparatus main body 9 may preferentially be performed in the reading apparatus 1E, for example.

The reading apparatus 1E may include a display provided to the housing 2 as illustrated in FIG. 1 and the like described above. The reading apparatus 1E may include a plurality of light sources, or may include a plurality of detectors as shown in FIGs. 97 to 99. The reading apparatus 1E may not include one of the operation units 4 and 953. In the reading apparatus 1E, the controller 80 of the reading apparatus main body 9 may instead perform one or more of a plurality of processes performed by the image processing unit 81 of the computer device 950. For example, the controller 80 may perform the image processing on the image signal from the detector 40, and the image signal after the image processing may be input into the image processing unit 81.

While the reading apparatus 1 has been described in detail as described above, the foregoing description is in all aspects illustrative and does not limit the present disclosure. Various modifications described above can be combined with each other for application unless any contradiction occurs. It is understood that numerous unillustrated modifications can be devised without departing from the scope of the present disclosure.

The present description and drawings disclose the following aspects:
A reading apparatus according to a first aspect is a reading apparatus that reads a radiograph from an imaging plate, and includes: a first light source that irradiates the imaging plate with excitation light; a first detector that detects photostimulated light from the imaging plate emitted by the excitation light; a second light source that irradiates an object with light; and a second detector that detects reflected light of the light from the object.

According to the first aspect, the radiograph based on detection of the photostimulated light from the imaging plate emitted by the excitation light and a reflected light image based on detection of the reflected light of the light from the object can be acquired to improve usability of the reading apparatus.

A second aspect is the reading apparatus according to the first aspect, wherein the first light source functions as the second light source, and irradiates the object with the excitation light as the light.

According to the second aspect, the first light source functions as the second light source to simplify the configuration of the reading apparatus.

A third aspect is the reading apparatus according to the first or the second aspect, wherein the object is the imaging plate, and the first detector detects the photostimulated light from the imaging plate from IP acted light, the second detector detects the reflected light from the imaging plate from the IP acted light, and the first detector and the second detector output an IP acted light image signal being an image signal as a result of detection of the IP acted light, the IP acted light being light emitted by the imaging plate being acted on by at least one of the excitation light and the light.

According to the third aspect, the second detector detects the reflected light from the imaging plate from the IP acted light to acquire a reflected light image of the imaging plate.

A fourth aspect is the reading apparatus according to the third aspect, wherein the first detector functions as the second detector, and the first detector outputs the IP acted light image signal.

According to the fourth aspect, the first detector functions as the second detector to simplify the configuration of the reading apparatus.

A fifth aspect is the reading apparatus according to the third or the fourth aspect further including a size identification unit that identifies a size of the imaging plate based on the IP acted light image signal.

According to the fifth aspect, the size of the imaging plate can properly be identified based on the IP acted light image signal as a result of detection of the IP acted light from the imaging plate.

A sixth aspect is the reading apparatus according to the fifth aspect, wherein the size identification unit identifies, based on the identified size, a type of the size.

According to the sixth aspect, the type of the size of the imaging plate can properly be identified.

A seventh aspect is the reading apparatus according to the fifth or the sixth aspect, wherein the first detector detects the reflected light from the imaging plate from which the radiograph has been erased, and outputs an erasing-time IP image signal being an image signal as a result of detection of the reflected light, and the size identification unit identifies the size of the imaging plate based on the erasing-time IP image signal.

According to the seventh aspect, the size identification unit identifies the size of the imaging plate based on the erasing-time IP image signal, so that the size of the imaging plate can properly be identified based on the reflected light not affected by the radiograph from the imaging plate.

An eighth aspect is the reading apparatus according to any one of the third to the seventh aspects further including a tilt angle identification unit that identifies a tilt angle of the imaging plate relative to a reference orientation based on the IP acted light image signal.

According to the eighth aspect, the tilt angle of the imaging plate relative to the reference orientation can properly be identified based on the IP acted light image signal as a result of detection of the IP acted light from the imaging plate.

A ninth aspect is the reading apparatus according to the eighth aspect further including a correction processing unit that corrects a tilt of an image of the imaging plate based on the tilt angle.

According to the ninth aspect, the tilt of the image of the imaging plate is corrected based on the tilt angle identified based on the IP acted light image signal as a result of detection of the IP acted light from the imaging plate, so that the image of the imaging plate whose tilt has been properly corrected can be acquired.

A tenth aspect is the reading apparatus according to any one of the fifth to the ninth aspects further including an image processing unit that performs image processing on a detected image signal from the first detector and the second detector, and the image processing unit sets a cutout range of an IP biological radiographically captured image from a biological radiographically captured image, the biological radiographically captured image being an image acquired by scanning the imaging plate as a light receiver in biological radiography with the excitation light, the IP biological radiographically captured image being an image based on detection of the IP acted light.

According to the tenth aspect, the cutout range of an image based on detection of the IP acted light from the imaging plate from the biological radiographically captured image is set, so that an image in a portion corresponding to the imaging plate of the biological radiographically captured image can properly be cut out.

An eleventh aspect is the reading apparatus according to any one of the third to the tenth aspects further including: a display; and a display control unit that controls the display, wherein the display control unit simultaneously and separately displays an acquired image acquired in biological radiography and an imaging plate shape extraction image representing a shape of the imaging plate extracted by performing processing on the IP acted light image signal.

According to the eleventh aspect, the image acquired in biological radiography and the image representing the shape of the imaging plate can easily be compared.

A twelfth aspect is the reading apparatus according to the eleventh aspect further including an unexposed region image identification unit that identifies an unexposed region image of a portion not exposed to radiation in the acquired image acquired in biological radiography.

According to the twelfth aspect, identification of the unexposed region image of the unexposed portion facilitates identification of a biological image region in the acquired image acquired by biological radiography.

The present description and drawings also disclose the following aspects:
A reading apparatus according to a first aspect is a reading apparatus that reads a radiograph from an imaging plate, and includes: a first light source that irradiates the imaging plate with excitation light; a first detector that detects emitted light from the imaging plate emitted by the excitation light, and outputs a first image signal as a result of detection of the emitted light; a second light source that irradiates the imaging plate with light; a second detector that detects reflected light of the light from the imaging plate, and outputs a second image signal as a result of detection of the reflected light; and an identification unit that identifies any abnormality of a surface of the imaging plate based on the second image signal.

According to the first aspect, the abnormality of the surface of the imaging plate can properly be identified based on the second image signal as a result of detection of the reflected light of the light from the imaging plate.

A second aspect is the reading apparatus according to the first aspect, wherein the first light source functions as the second light source, and irradiates the imaging plate with the excitation light as the light.

According to the second aspect, the first light source functions as the second light source to simplify the configuration of the reading apparatus.

A third aspect is the reading apparatus according to the first or the second aspect, wherein the first detector functions as the second detector.

According to the third aspect, the first detector functions as the second detector to simplify the configuration of the reading apparatus.

A fourth aspect is the reading apparatus according to the second aspect, wherein the first detector functions as the second detector, the reading apparatus further includes an erasing unit that erases the radiograph from the imaging plate after the first detector outputs the first image signal, the first light source irradiates the imaging plate from which the radiograph has been erased with the excitation light as the light, and the first detector detects the reflected light from the imaging plate from which the radiograph has been erased.

According to the fourth aspect, the erasing unit that erases the radiograph from the imaging plate after the first detector outputs the first image signal as a result of detection of the emitted light is provided, so that both the radiograph recorded on the imaging plate and a reflected light image of the imaging plate can easily be acquired.

A fifth aspect is the reading apparatus according to any one of the first to the fourth aspects, wherein the identification unit identifies a position and a shape of an abnormal region image in which the abnormality appears in a reflected light image based on the second image signal, and a display displays an abnormal region display which is a display of a position and a shape of an abnormal region against a radiograph generated by processing of the first image signal.

According to the fifth aspect, the user can easily visualize a region corresponding to the abnormality of the surface of the imaging plate in the radiograph.

A sixth aspect is the reading apparatus according to any one of the first to the fifth aspects, wherein the identification unit identifies a position and a shape of an abnormal region image in which the abnormality appears in a reflected light image based on the second image signal, and, when the radiograph based on the first image signal is displayed, superimposes the shape on the radiograph at a position of the radiograph corresponding to the position.

According to the sixth aspect, the user can easily identify a region corresponding to the abnormality of the surface of the imaging plate in the radiograph.

A seventh aspect is the reading apparatus according to the fifth or the sixth aspect, wherein switching between display and hiding of the shape is made in response to instructions from the user.

According to the seventh aspect, switching between display and hiding of the shape of the abnormal region image is made in response to the instructions from the user to improve usability of the reading apparatus.

An eighth aspect is the reading apparatus according to any one of the fifth to the seventh aspects, wherein abnormality corresponding region luminance adjustment processing of adjusting luminance information for an abnormality corresponding region corresponding to the abnormality in the read radiograph based on luminance information for the abnormal region image is performed.

According to the eighth aspect, a proper radiograph can be acquired by the abnormality corresponding region luminance adjustment processing.

A program according to a ninth aspect is a program that causes a computer device to function as the identification unit of the reading apparatus according to the first or the fifth aspect.

The present description and drawings also disclose the following aspects:
A reading apparatus according to a first aspect is a reading apparatus that reads a radiograph from an imaging plate, and includes: a light source that irradiates the imaging plate with light; a detector that detects reflected light of the light from the imaging plate; and a determination unit that determines whether the imaging plate is set in reverse based on a result of detection performed by the detector.

According to the first aspect, whether the imaging plate is set in reverse can be determined based on a result of detection performed by the detector. This allows the radiograph to be more surely read from the imaging plate based on a result of determination.

A second aspect is the reading apparatus according to the first aspect, wherein the light functions as excitation light to excite the imaging plate to read the radiograph from the imaging plate.

According to the second aspect, the light emitted from the light source functions as the excitation light to excite the imaging plate to read the radiograph from the imaging plate. This eliminates the need for another light source to irradiate the imaging plate with the excitation light. The configuration of the reading apparatus can thereby be simplified.

A third aspect is the reading apparatus according to the first or the second aspect, wherein the detector detects emitted light from the imaging plate emitted by the excitation light.

According to the third aspect, the detector detects the emitted light from the imaging plate. This eliminates the need for another detector to detect the emitted light from the imaging plate. The configuration of the reading apparatus can thereby be simplified.

A fourth aspect is the reading apparatus according to any one of the first to the third aspects, wherein at least one piece of back surface specific information is shown on a back surface of the imaging plate.

According to the fourth aspect, the at least one piece of back surface specific information is shown on the back surface of the imaging plate, so that whether the imaging plate is set in reverse can easily be determined.

A fifth aspect is the reading apparatus according to the fourth aspect, wherein the light source scans the imaging plate with the light, and a plurality of pieces of back surface specific information are shown on the back surface of the imaging plate.

According to the fifth aspect, when the light source scans the imaging plate with the light, the plurality of pieces of back surface specific information are shown on the back surface of the imaging plate. Reverse setting of the imaging plate can thus be identified immediately even if an orientation of the imaging plate when the imaging plate is set is not constant.

A sixth aspect is the reading apparatus according to any one of the first to the fifth aspects, wherein at least one piece of front surface specific information is shown on a front surface of the imaging plate.

According to the sixth aspect, the at least one piece of front surface specific information is shown on the front surface of the imaging plate, so that whether the imaging plate is set in reverse can easily be determined.

A seventh aspect is the reading apparatus according to the sixth aspect, wherein the light source scans the imaging plate with the light, and a plurality of pieces of front surface specific information are shown on the front surface of the imaging plate.

According to the seventh aspect, when the light source scans the imaging plate with the light, the plurality of pieces of front surface specific information are shown on the front surface of the imaging plate. Reverse setting of the imaging plate can thus be identified immediately even if the orientation of the imaging plate when the imaging plate is set is not constant.

An eighth aspect is the reading apparatus according to any one of the first to the seventh aspects, wherein the imaging plate has, at a peripheral edge thereof, at least one protrusion to determine whether the imaging plate is set in reverse.

According to the eighth aspect, the imaging plate has, at the peripheral edge thereof, the at least one protrusion to determine whether the imaging plate is set in reverse, so that whether the imaging plate is set in reverse can easily be determined.

A ninth aspect is the reading apparatus according to the eighth aspect, wherein the imaging plate has, at the peripheral edge thereof, a plurality of protrusions to determine whether the imaging plate is set in reverse, and the light source scans the imaging plate with the light, and scans a region at the peripheral edge with the light.

According to the ninth aspect, when the light source scans the imaging plate with the light, the imaging plate has, at the peripheral edge thereof, the plurality of protrusions to determine whether the imaging plate is set in reverse. Reverse setting of the imaging plate can thus be identified immediately even if the orientation of the imaging plate when the imaging plate is set is not constant.

A tenth aspect is the reading apparatus according to any one of the first to the ninth aspects, wherein a user is notified of an alert when it is determined that the imaging plate is set in reverse.

According to the tenth aspect, the user is notified of the alert when it is determined that the imaging plate is set in reverse. This can prompt the user to properly set the imaging plate.

An eleventh aspect is the reading apparatus according to any one of the first to the tenth aspects, wherein the imaging plate from which the radiograph has been read is irradiated with erasing light to erase the radiograph when it is determined that the imaging plate is not set in reverse, and the imaging plate is not irradiated with the erasing light when it is determined that the imaging plate is set in reverse.

According to the eleventh aspect, the imaging plate is not irradiated with the erasing light when it is determined that the imaging plate is set in reverse. The radiograph recorded on the imaging plate is thus less likely to be affected by the erasing light.

A twelfth aspect is the reading apparatus according to any one of the first to the eleventh aspects, wherein the light source performs scanning processing of scanning the imaging plate with the light, and stops the scanning processing when it is determined that the imaging plate is set in reverse during the scanning processing.

According to the twelfth aspect, the scanning processing is stopped when it is determined that the imaging plate is set in reverse during the scanning processing. Useless continuation of the scanning processing can thereby be avoided.

A thirteenth aspect is the reading apparatus according to any one of the first to the tenth aspects, wherein the imaging plate is discharged when it is determined that the imaging plate is set in reverse.

According to the thirteenth aspect, the imaging plate is discharged when it is determined that the imaging plate is set in reverse. This eliminates the need for a user operation to provide instructions to discharge the imaging plate on the reading apparatus. Furthermore, discharge of the imaging plate can prompt the user to set the imaging plate again.

A fourteenth aspect is the reading apparatus according to the thirteenth aspect, wherein the imaging plate from which the radiograph has been read is irradiated with erasing light to erase the radiograph when it is determined that the imaging plate is not set in reverse, and the imaging plate is discharged without being irradiated with the erasing light when it is determined that the imaging plate is set in reverse.

According to the fourteenth aspect, the imaging plate is discharged without being irradiated with the erasing light when it is determined that the imaging plate is set in reverse. The imaging plate can thereby be discharged immediately when it is determined that the imaging plate is set in reverse.

A fifteenth aspect is the reading apparatus according to the thirteenth or the fourteenth aspect, wherein the light source performs scanning processing of scanning the imaging plate with the light, and stops the scanning processing and discharges the imaging plate when it is determined that the imaging plate is set in reverse during the scanning processing.

According to the fifteenth aspect, the scanning processing is stopped, and the imaging plate is discharged when it is determined that the imaging plate is set in reverse during the scanning processing. The imaging plate can thereby be discharged immediately when it is determined that the imaging plate is set in reverse.

A sixteenth aspect is a program to control a reading apparatus that reads a radiograph from an imaging plate, and includes: a light source that irradiates the imaging plate with light; and a detector that detects reflected light of the light from the imaging plate, and the program causes the reading apparatus to determine whether the imaging plate is set in reverse based on a result of detection performed by the detector.

A seventeenth aspect is the program according to the sixteenth aspect, wherein the program causes the reading apparatus to notify a user of an alert when it is determined that the imaging plate is set in reverse.

An eighteenth aspect is the program according to the sixteenth or the seventeenth aspect, wherein the program causes the reading apparatus to discharge the imaging plate when it is determined that the imaging plate is set in reverse.

The present description and drawings also disclose the following aspects:
A reading apparatus according to a first aspect is a reading apparatus that reads a radiograph from an imaging plate, and includes: a light source that irradiates the imaging plate with excitation light; and a first detector that detects emitted light from the imaging plate emitted by the excitation light, and outputs a first image signal as a result of detection of the emitted light, wherein the light source irradiates an evaluation member having, on a surface thereof, an evaluation pattern to evaluate an image quality of a detected radiograph based on the first image signal with the excitation light, and the reading apparatus further includes a second detector that detects reflected light of the excitation light from the surface of the evaluation member, and outputs a second image signal as a result of detection of the reflected light.

According to the first aspect, the image quality of the detected radiograph, that is, the radiograph read from the imaging plate can properly be evaluated based on an image of the evaluation pattern included in a reflected light image based on the second image signal.

A reading apparatus according to a second aspect is the reading apparatus according to the first aspect, wherein the first detector functions as the second detector, and the first detector detects the emitted light and the reflected light.

According to the second aspect, the first detector functions as the second detector to simplify the configuration of the reading apparatus.

A third aspect is the reading apparatus according to the first or the second aspect, wherein the evaluation pattern is printed on the surface of the evaluation member.

According to the third aspect, the evaluation pattern is formed by printing, so that the evaluation pattern having high accuracy can be acquired. The image quality of the detected radiograph can thereby properly be evaluated.

A fourth aspect is the reading apparatus according to the third aspect, wherein the evaluation member is printed paper.

According to the fourth aspect, the evaluation member is the printed paper, so that the image quality of the detected radiograph can be evaluated using an inexpensive evaluation member.

A fifth aspect is the reading apparatus according to any one of the first to the fourth aspects, wherein a reflected light image based on the second image signal is displayed.

According to the fifth aspect, a user can evaluate the image quality of the detected radiograph by checking an image of the evaluation pattern included in the reflected light image based on the second image signal.

Support for the claims and further information is defined in the following itemized list:
1. A reading apparatus (1, 1A, 1B, 1C, 1D, 1E) that reads a radiograph (101a, 101b, 151a) from an imaging plate (10), the reading apparatus (1, 1A, 1B, 1C, 1D, 1E) comprising:
   a first light source (30) that irradiates the imaging plate (10) with excitation light (L10);
   a first detector (40) that detects photostimulated light (L5) from the imaging plate (10) emitted by the excitation light (L10);
   a second light source (30, 130) that irradiates an object (10, 900, 900A, 900B, 900C, 900D) with light (L10, L11); and
   a second detector (40, 140) that detects reflected light (L4, L40) of the light (L10, L11) from the object (10, 900, 900A, 900B, 900C, 900D).
2. The reading apparatus (1, 1B, 1D, 1E) according to item 1, wherein
   the first light source (30) functions as the second light source (30), and irradiates the object (10, 900, 900A, 900B, 900C, 900D) with the excitation light (L11) as the light (L11).
3. The reading apparatus (1, 1A, 1B, 1C, 1D, 1E) according to item 1 or 2, wherein
   the object (10) is the imaging plate (10), and
   the first detector (40) detects the photostimulated light (L5) from the imaging plate (10) from IP acted light (L2), the second detector (40, 140) detects the reflected light (L4, L40) from the imaging plate (10) from the IP acted light (L2), and the first detector (40) and the second detector (40, 140) output an IP acted light image signal being an image signal as a result of detection of the IP acted light (L2), the IP acted light (L2) being light emitted by the imaging plate (10) being acted on by at least one of the excitation light (L10) and the light (L11).
4. The reading apparatus (1, 1A, 1D, 1E) according to item 3, wherein
   the first detector (40) functions as the second detector (40), and
   the first detector (40) outputs the IP acted light image signal.
5. The reading apparatus (1, 1A, 1B, 1C, 1D, 1E) according to item 3 or 4, further comprising
   a size identification unit (81) that identifies a size of the imaging plate (10) based on the IP acted light image signal.
6. The reading apparatus (1, 1A, 1B, 1C, 1D, 1E) according to item 5, wherein
   the size identification unit (81) identifies, based on the identified size, a type of the size.
7. The reading apparatus (1, 1A, 1B, 1C, 1D, 1E) according to item 5 or 6, wherein
   the first detector (40) detects the reflected light (L40) from the imaging plate (10) from which the radiograph (101a, 101b, 151a) has been erased, and outputs an erasing-time IP image signal being an image signal as a result of detection of the reflected light (L40), and
   the size identification unit (81) identifies the size of the imaging plate (10) based on the erasing-time IP image signal.
8. The reading apparatus (1, 1A, 1B, 1C, 1D, 1E) according to any one of items 3 to 7, further comprising
   a tilt angle identification unit (81) that identifies a tilt angle of the imaging plate (10) relative to a reference orientation based on the IP acted light image signal.
9. The reading apparatus (1, 1A, 1B, 1C, 1D, 1E) according to item 8, further comprising
   a correction processing unit (81) that corrects a tilt of an image of the imaging plate (10) based on the tilt angle.
10. The reading apparatus (1, 1A, 1B, 1C, 1D, 1E) according to any one of items 5 to 9, further comprising
   an image processing unit (81) that performs image processing on a detected image signal from the first detector (40) and the second detector (40, 140), and
   the image processing unit (81) sets a cutout range (150) of an IP biological radiographically captured image (101a, 101b, 151a, 151b, 201) from a biological radiographically captured image (100a, 100b, 200), the biological radiographically captured image (100a, 100b, 200) being an image acquired by scanning the imaging plate (10) as a light receiver in biological radiography with the excitation light (L10), the IP biological radiographically captured image (101a, 101b, 151a, 151b, 201) being an image based on detection of the IP acted light (L2).
11. The reading apparatus (1, 1A, 1B, 1C, 1D, 1E) according to any one of items 3 to 10, further comprising
   a display (3, 13); and
   a display control unit (82) that controls the display (3, 13), wherein
   the display control unit (82) simultaneously and separately displays an acquire image (100a, 100b, 151) acquired in biological radiography and an imaging plate shape extraction image (200, 500, 501) representing a shape of the imaging plate (10) extracted by performing processing on the IP acted light image signal.
12. The reading apparatus (1, 1A, 1B, 1C, 1D, 1E) according to item 11, further comprising
   an unexposed region image identification unit (81) that identifies an unexposed region image (103b, 151b) of a portion not exposed to radiation in the acquired image (100a, 100b, 101a, 101b, 151) acquired in biological radiography.

While the invention has been shown and described in detail, the foregoing description is in all aspects illustrative and not restrictive. It is therefore understood that numerous modifications and variations can be devised without departing from the scope of the invention.

## Claims

1. A reading apparatus (1, 1A, 1B, 1C, 1D, 1E) that is configured to read a radiograph (101a, 101b, 151a) from an imaging plate (10), the reading apparatus (1, 1A, 1B, 1C, 1D, 1E) comprising:
a first light source (30) that is configured to irradiate the imaging plate (10) with excitation light (L10);
a first detector (40) that is configured to detect photostimulated light (L5) from the imaging plate (10) emitted due to irradiation by the excitation light (L10), and to output a first image signal as a result of detection of the photostimulated light (L5);
a second light source (30, 130) that is configured to irradiate the imaging plate (10) with light (L10, L11);
a second detector (40, 140) that is configured to detect reflected light (L4, L40) of the light (L10, L11) from the imaging plate (10), and to output a second image signal as a result of detection of the reflected light (L4, L40); and
an identification unit (81) that is configured to identify abnormality of a surface of the imaging plate (10) based on the second image signal.

2. The reading apparatus (1, 1B, 1D, 1E) according to claim 1, wherein
the first light source (30) and the second light source (30) are the same, and the excitation light (L10) and the light (L10) are the same.

3. The reading apparatus (1, 1A, 1D, 1E) according to claim 1 or 2, wherein
the first detector (40) and the second detector (40) are the same.

4. The reading apparatus (1, 1A, 1D, 1E) according to claim 2, wherein
the first detector (40) and the second detector (40) are the same,
the reading apparatus (1, 1A, 1D, 1E) further comprises an erasing unit (91) that is configured to erase the radiograph from the imaging plate (10) after the first detector (40) outputs the first image signal, and
the first light source (30) is configured to irradiate the imaging plate (10) from which the radiograph has been erased with the excitation light (L10) as the light(L10), and the first detector (40) is configured to detect the reflected light (L40) from the imaging plate (10) from which the radiograph has been erased.

5. The reading apparatus (1, 1A, 1B, 1C, 1D, 1E) according to any one of claims 1 to 4, wherein
the identification unit (81) is configured to identify a position and a shape of an abnormal region image (2010) in which the abnormality appears in a reflected light image (201) based on the second image signal, and
when the radiograph (101a, 101b, 151a) based on the first image signal is displayed, the shape of the abnormal region image (2010) is superimposed on the radiograph (101a, 101b, 151a) at a position of the radiograph (101a, 101b, 151a) corresponding to the position of the abnormal region image (2010).

6. The reading apparatus (1, 1A, 1B, 1C, 1D, 1E) according to claim 5, wherein
when the reflected light image (201) is displayed, the shape of the abnormal region image (2010) is superimposed on the radiograph (101a, 101b, 151a).

7. The reading apparatus (1, 1A, 1B, 1C, 1D, 1E) according to claim 5 or 6, wherein
the reading apparatus (1, 1A, 1B, 1C, 1D, 1E) is configured to switch between display and hiding of the shape of the abnormal region image in response to the instructions from the user.

8. The reading apparatus (1, 1A, 1B, 1C, 1D, 1E) according to any one of claims 5 to 7, wherein
the reading apparatus (1, 1A, 1B, 1C, 1D, 1E) is configured to perform abnormality corresponding region luminance adjustment processing of adjusting luminance information for an abnormality corresponding region (2011) corresponding to the abnormality in the read radiograph (101a, 101b, 151a) based on luminance information for the abnormal region image (2010).

9. A program that is configured to cause a computer device to function as the identification unit (81) of the reading apparatus (1, 1A, 1B, 1C, 1D, 1E) according to claim 1 or 5.
